# EUROPEAN PATENT APPLICATION

(11) **EP 3 800 182 A1**
(43) Date of publication of application: **07.04.2021**
(21) Application number: 20194890.8
(22) Date of filing: 14.10.2016
(51) Int. Cl.: C07D 213/50, C07D 213/68, C07D 237/14, C07D 401/04, A61K 31/4412, A61P 35/00, A61P 31/18, A61P 29/00, C07D 213/76, C07D 405/12

(54) **PYRIDIN-2(1H)-ONE DERIVATIVES AS BROMODOMAIN INHIBITORS**

(62) Divisional of application: 16918992.5
(71) Applicant: AbbVie Inc., North Chicago, IL 60064 (US)
(72) Inventor: BOGDAN, Andrew, Evanston, IL 60201 (US); FIDANZE, Steven, Grayslake, IL 60030 (US); MCDANIEL, Keith, Wauconda, IL 60084 (US); PRATT, John, Kenosha, WI 53142 (US); WANG, Le, Vernon Hills, IL 60061 (US)
(74) Representative: Schlich, George

(57) **Abstract**

The present invention provides for compounds of formula (I) 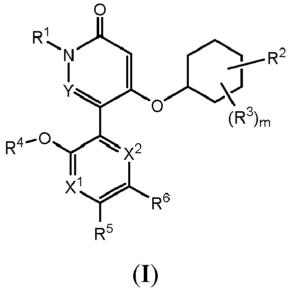 wherein R¹, Y, X¹, X², R², R³, R⁴, R⁵, R⁶, and m have any of the values defined in the specification, and pharmaceutically acceptable salts thereof, that are useful as agents in the treatment of diseases and conditions, including inflammatory diseases, cancer, and AIDS. Also provided are pharmaceutical compositions comprising compounds of formula (I).

## Description

### BACKGROUND

Bromodomains refer to conserved protein structural folds which bind to *N*-acetylated lysine residues that are found in some proteins. The BET family of bromodomain containing proteins comprises four members (BRD2, BRD3, BRD4 and BRDt). Each member of the BET family employs two bromodomains to recognize *N-*acetylated lysine residues typically, but not exclusively those found on transcription factors (Shi, J., et al. Cancer Cell 25(2): 210-225 (2014)) or on the amino-terminal tails of histone proteins. Numbering from the N-terminal end of each BET protein the tandem bromodomains are typically labelled Binding Domain I (BDI) and Binding Domain II (BDII). These interactions modulate gene expression by recruiting transcription factors to specific genome locations within chromatin. For example, histone-bound BRD4 recruits the transcription factor P-TEFb to promoters, resulting in the expression of a subset of genes involved in cell cycle progression (Yang et al., Mol. Cell. Biol. 28: 967-976 (2008)). BRD2 and BRD3 also function as transcriptional regulators of growth promoting genes (LeRoy et al., Mol. Cell 30: 51-60 (2008)). BET family members were recently established as being important for the maintenance of several cancer types (Zuber et al., Nature 478: 524-528 (2011); Mertz et al; Proc. Nat'1. Acad. Sci. 108: 16669-16674 (2011); Delmore et al., Cell 146: 1-14, (2011); Dawson et al., Nature 478: 529-533 (2011)). BET family members have also been implicated in mediating acute inflammatory responses through the canonical NF-KB pathway (Huang et al., Mol. Cell. Biol. 29: 1375-1387 (2009)) resulting in the upregulation of genes associated with the production of cytokines (Nicodeme et al., Nature 468: 1119-1123, (2010)). Suppression of cytokine induction by BET bromodomain inhibitors has been shown to be an effective approach to treat inflammation-mediated kidney disease in an animal model (Zhang, et al., J. Biol. Chem. 287: 28840-28851 (2012)). BRD2 function has been linked to pre-disposition for dyslipidemia or improper regulation of adipogenesis, elevated inflammatory profiles and increased susceptibility to autoimmune diseases (Denis, Discovery Medicine 10: 489-499 (2010)). The human immunodeficiency virus utilizes BRD4 to initiate transcription of viral RNA from stably integrated viral DNA (Jang et al., Mol. Cell, 19: 523-534 (2005)). BET bromodomain inhibitors have also been shown to reactivate HIV transcription in models of latent T cell infection and latent monocyte infection (Banerjee, et al, J. Leukocyte Biol. doi:10.1189/jlb.0312165). BRDt has an important role in spermatogenesis that is blocked by BET bromodomain inhibitors (Matzuk, et al., Cell 150: 673-684 (2012)). Thus, compounds that inhibit the binding of BET family bromodomains to their cognate acetylated lysine proteins are being pursued for the treatment of cancer, inflammatory diseases, kidney diseases, diseases involving metabolism or fat accumulation, and some viral infections, as well as for providing a method for male contraception. Accordingly, there is an ongoing medical need to develop new drugs to treat these indications.

### SUMMARY

In one aspect the present invention provides for compounds of formula (I) or a pharmaceutically acceptable salt thereof, 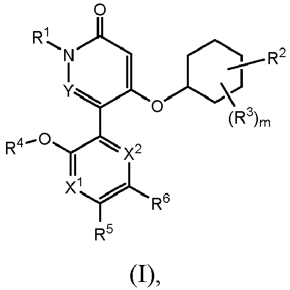 wherein
R¹ is C₁-C₃ alkyl;
Y is N or C(R^{Y}) wherein R^{Y} is hydrogen or C₁-C₃ alkyl;
m is 0, 1, 2, 3, or 4;
R² is G^{1A}, -N(R^{a})S(O)₂R^{b}, -N(R^{a})C(O)R^{b}, or -N(R^{a})C(O)OR^{b};
R^{a}, at each occurrence, is independently hydrogen, C₁-C₆ alkyl, C₁-C₆ haloalkyl, or -(C₂-C₆ alkylenyl)-OR^{x} wherein R^{x} is hydrogen, C₁-C₆ alkyl, or C₁-C₆ haloalkyl;
R^{b}, at each occurrence, is independently C₁-C₆ alkyl, C₂-C₆ alkenyl, C₁-C₆ haloalkyl, G^{1B}, or -(C₁-C₆ alkylenyl)-OR^{j};
G^{1A} is formula (i) 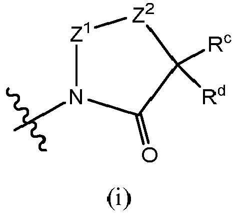 wherein
   Z¹ is C(O) or C(R^{e})(R^{f});
   Z² is a bond, O, -(C(R^{g})(R^{h}))ₚ-C(R^{m})(Rⁿ)- wherein the left end of the moiety is attached to Z¹, or N(Rⁱ) wherein Rⁱ is hydrogen, C₁-C₃ alkyl, or cyclopropyl; wherein the cyclopropyl is optionally substituted with 1, 2, 3, or 4 independently selected R^{s} groups;
   p is 0 or 1;
   R^{c}, R^{d}, R^{e}, R^{f}, R^{g}, R^{h}, R^{m}, and Rⁿ, are each independently hydrogen, C₁-C₆ alkyl, C₁-C₆ haloalkyl, or cyclopropyl; wherein the cyclopropyl, at each occurrence, is optionally substituted with 1, 2, 3, or 4 independently selected R^{s} groups; or
   R^{c} and R^{m} together with the carbon atoms to which they are attached, form a C₃-C₆ monocyclic cycloalkyl or a 4-6 membered monocyclic heterocycle; wherein the C₃-C₆ monocyclic cycloalkyl and the 4-6 membered monocyclic heterocycle are each optionally substituted with 1, 2, 3, or 4 independently selected R^{s} groups; or
   R^{c} and Rⁱ together with the atoms to which they are attached, form a 4-6 membered monocyclic heterocycle; wherein the 4-6 membered monocyclic heterocycle is optionally substituted with 1, 2, 3, or 4 independently selected R^{s} groups; or
   R^{c} and R^{d} together with the carbon atoms to which they are attached, form a C₃-C₆ monocyclic cycloalkyl or a 4-6 membered monocyclic heterocycle; wherein the C₃-C₆ monocyclic cycloalkyl and the 4-6 membered monocyclic heterocycle are each optionally substituted with 1, 2, 3, or 4 independently selected R^{s} groups;
G^{1B} is oxetanyl, cyclopropyl, cyclobutyl, or cyclopentyl; wherein each G^{1B} is optionally substituted with 1, 2, 3, or 4 R^{t} groups; wherein each R^{t} is independently halogen, C₁-C₆ alkyl, or C₁-C₆ haloalkyl;
each R³ is independently C₁-C₃ alkyl, C₁-C₃ haloalkyl, halogen, -CN, -OR^{3a}, -N(R^{3a})₂, -C(O)R^{3a}, cyclopropyl, or cyclobutyl; wherein each R^{3a} is independently hydrogen, C₁-C₃ alkyl, or C₁-C₃ haloalkyl;
R⁴ is phenyl or monocyclic heteroaryl; wherein each R⁴ is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH;
R⁵ is hydrogen, halogen, -CN, C₁-C₆ haloalkyl, or C₁-C₆ alkyl;
R⁶ is -(C₁-C₃ alkylenyl)ₙ-S(O)₂-R^{6a} or -N(R^{6b})-SO₂-R^{6c};
n is 0 or 1;
R^{6a} and R^{6c} are each independently C₁-C₆ alkyl, C₁-C₆ haloalkyl, or cyclopropyl; wherein the cyclopropyl is optionally substituted with 1, 2, 3, or 4 substituents independently selected from the group consisting of halogen, C₁-C₆ alkyl, and C₁-C₆ haloalkyl;
R^{6b} is hydrogen, C₁-C₆ alkyl, or C₁-C₆ haloalkyl;
X¹ and X² are C(R⁷) or
one of X¹ and X² is N and the other is C(R⁷);
R⁷, at each occurrence, is independently hydrogen or halogen;
R^{s}, at each occurrence, is independently C₁-C₆ alkyl, halogen, or C₁-C₆ haloalkyl;
R^{j}, at each occurrence, is independently hydrogen, C₁-C₆ alkyl, or C₁-C₆ haloalkyl; and
R^{k}, at each occurrence, is independently C₁-C₆ alkyl or C₁-C₆ haloalkyl.

In another aspect, the present invention provides for methods for treating or preventing disorders that are ameliorated by inhibition of BET. Such methods comprise of administering to the subject a therapeutically effective amount of a compound of formula (I), (I-a), or (I-b), alone, or in combination with a pharmaceutically acceptable carrier.

Some of the methods are directed to treating or preventing an inflammatory disease or cancer or AIDS.

In another aspect, the present invention relates to methods of treating cancer in a subject comprising administering a therapeutically effective amount of a compound of formula (I), (I-a), or (I-b), or a pharmaceutically acceptable salt thereof, to a subject in need thereof. In certain embodiments, the cancer is selected from the group consisting of: acoustic neuroma, acute leukemia, acute lymphocytic leukemia, acute myelocytic leukemia (monocytic, myeloblastic, adenocarcinoma, angiosarcoma, astrocytoma, myelomonocytic and promyelocytic), acute t-cell leukemia, basal cell carcinoma, bile duct carcinoma, bladder cancer, brain cancer, breast cancer, bronchogenic carcinoma, cervical cancer, chondrosarcoma, chordoma, choriocarcinoma, chronic leukemia, chronic lymphocytic leukemia, chronic myelocytic (granulocytic) leukemia, chronic myelogenous leukemia, colon cancer, colorectal cancer, craniopharyngioma, cystadenocarcinoma, diffuse large B-cell lymphoma, dysproliferative changes (dysplasias and metaplasias), embryonal carcinoma, endometrial cancer, endotheliosarcoma, ependymoma, epithelial carcinoma, erythroleukemia, esophageal cancer, estrogen-receptor positive breast cancer, essential thrombocythemia, Ewing's tumor, fibrosarcoma, follicular lymphoma, germ cell testicular cancer, glioma, glioblastoma, gliosarcoma, heavy chain disease, hemangioblastoma, hepatoma, hepatocellular cancer, hormone insensitive prostate cancer, leiomyosarcoma, leukemia, liposarcoma, lung cancer, lymphagioendotheliosarcoma, lymphangiosarcoma, lymphoblastic leukemia, lymphoma (Hodgkin's and non-Hodgkin's), malignancies and hyperproliferative disorders of the bladder, breast, colon, lung, ovaries, pancreas, prostate, skin and uterus, lymphoid malignancies of T-cell or B-cell origin, leukemia, lymphoma, medullary carcinoma, medulloblastoma, melanoma, meningioma, mesothelioma, multiple myeloma, myelogenous leukemia, myeloma, myxosarcoma, neuroblastoma, NUT midline carcinoma (NMC), non-small cell lung cancer, oligodendroglioma, oral cancer, osteogenic sarcoma, ovarian cancer, pancreatic cancer, papillary adenocarcinomas, papillary carcinoma, pinealoma, polycythemia vera, prostate cancer, rectal cancer, renal cell carcinoma, retinoblastoma, rhabdomyosarcoma, sarcoma, sebaceous gland carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenstrom's macroglobulinemia, testicular tumors, uterine cancer, and Wilms' tumor. In certain embodiments, the methods further comprise administering a therapeutically effective amount of at least one additional therapeutic agent. In certain embodiments, the additional therapeutic agent is selected from the group consisting of cytarabine, bortezomib, and 5-azacitidine.

In another aspect, the present invention relates to methods of treating a disease or condition in a subject comprising administering a therapeutically effective amount of a compound of formula (I), (I-a), or (I-b), or a pharmaceutically acceptable salt thereof, to a subject in need thereof, wherein said disease or condition is selected from the group consisting of: Addison's disease, acute gout, ankylosing spondylitis, asthma, atherosclerosis, Behcet's disease, bullous skin diseases, chronic obstructive pulmonary disease (COPD), Crohn's disease, dermatitis, eczema, giant cell arteritis, glomerulonephritis, hepatitis, hypophysitis, inflammatory bowel disease, Kawasaki disease, lupus nephritis, multiple sclerosis, myocarditis, myositis, nephritis, organ transplant rejection, osteoarthritis, pancreatitis, pericarditis, polyarteritis nodosa, pneumonitis, primary biliary cirrhosis, psoriasis, psoriatic arthritis, rheumatoid arthritis, scleritis, sclerosing cholangitis, sepsis, systemic lupus erythematosus, Takayasu's Arteritis, toxic shock, thyroiditis, type I diabetes, ulcerative colitis, uveitis, vitiligo, vasculitis, and Wegener's granulomatosis. In certain embodiments, the methods further comprise administering a therapeutically effective amount of at least one additional therapeutic agent.

In another aspect, the present invention relates to methods of treating a chronic kidney disease or condition in a subject comprising administering a therapeutically effective amount of a compound of formula (I), (I-a), or (I-b), or a pharmaceutically acceptable salt thereof, to a subject in need thereof, wherein said disease or condition is selected from the group consisting of: diabetic nephropathy, hypertensive nephropathy, HIV-associated nephropathy, glomerulonephritis, lupus nephritis, IgA nephropathy, focal segmental glomerulosclerosis, membranous glomerulonephritis, minimal change disease, polycystic kidney disease, and tubular interstitial nephritis. In certain embodiments, the methods further comprise administering a therapeutically effective amount of at least one additional therapeutic agent.

In another aspect, the present invention relates to methods of treating an acute kidney injury or disease or condition in a subject comprising administering a therapeutically effective amount of a compound of formula (I), (I-a), or (I-b), or a pharmaceutically acceptable salt thereof, to a subject in need thereof, wherein said acute kidney injury or disease or condition is selected from the group consisting of: ischemia-reperfusion induced kidney disease, cardiac and major surgery induced kidney disease, percutaneous coronary intervention induced kidney disease, radio-contrast agent induced kidney disease, sepsis induced kidney disease, pneumonia induced kidney disease, and drug toxicity induced kidney disease. In certain embodiments, the methods further comprise administering a therapeutically effective amount of at least one additional therapeutic agent.

In another aspect, the present invention relates to methods of treating AIDS in a subject comprising administering a therapeutically effective amount of a compound of formula (I), (I-a), or (I-b), or a pharmaceutically acceptable salt thereof, to a subject in need thereof. In certain embodiments, the methods further comprise administering a therapeutically effective amount of at least one additional therapeutic agent.

In another aspect, the present invention relates to methods of treating obesity, dyslipidemia, hypercholesterolemia, Alzheimer's disease, metabolic syndrome, hepatic steatosis, type II diabetes, insulin resistance, diabetic retinopathy, or diabetic neuropathy in a subject comprising administering a therapeutically effective amount of a compound of formula (I), (I-a), or (I-b), or a pharmaceutically acceptable salt thereof, to a subject in need thereof. In certain embodiments, the methods further comprise administering a therapeutically effective amount of at least one additional therapeutic agent.

In another aspect, the present invention relates to methods of preventing conception by inhibiting spermatogenesis in a subject comprising administering a therapeutically effective amount of a compound of formula (I), (I-a), or (I-b), or a pharmaceutically acceptable salt thereof, to a subject in need thereof. In certain embodiments, the methods further comprise administering a therapeutically effective amount of at least one additional therapeutic agent.

A further aspect of the invention provides the use of a compound of formula (I), (I-a), or (I-b), alone or in combination with at least one additional therapeutic agent, in the manufacture of a medicament for treating or preventing conditions and disorders disclosed herein, with or without a pharmaceutically acceptable carrier.

Pharmaceutical compositions comprising a compound of formula (I), (I-a), or (I-b), or a pharmaceutically acceptable salt, alone or in combination with at lease one additional therapeutic agent, are also provided.

### DETAILED DESCRIPTION

Disclosed herein are compounds of formula (I) 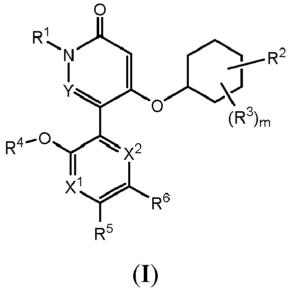 wherein R¹, Y, X¹, X², R², R³, R⁴, R⁵, R⁶, and m are defined above in the Summary of the Invention and below in the Detailed Description. Further, compositions comprising such compounds and methods for treating conditions and disorders using such compounds and compositions are also disclosed.

Compounds disclosed herein may contain one or more variable(s) that occur more than one time in any substituent or in the formulae herein. Definition of a variable on each occurrence is independent of its definition at another occurrence. Further, combinations of substituents are permissible only if such combinations result in stable compounds. Stable compounds are compounds, which can be isolated from a reaction mixture.

### a. Definitions

It is noted that, as used in this specification and the intended claims, the singular form "a," "an," and "the" include plural referents unless the context clearly dictates otherwise. Thus, for example, reference to "a compound" includes a single compound as well as one or more of the same or different compounds, reference to "a pharmaceutically acceptable carrier" means a single pharmaceutically acceptable carrier as well as one or more pharmaceutically acceptable carriers, and the like.

As used in the specification and the appended claims, unless specified to the contrary, the following terms have the meaning indicated:

The term "alkenyl" as used herein, means a straight or branched hydrocarbon chain containing from 2 to 10 carbons and containing at least one carbon-carbon double bond. The term "C₂-C₆ alkenyl" means an alkenyl group containing 2-6 carbon atoms. Non-limiting examples of C₂-C₆ alkenyl include buta-1,3-dienyl, ethenyl, 2-propenyl, 2-methyl-2-propenyl, 3-butenyl, 4-pentenyl, and 5-hexenyl.

The term "alkyl" as used herein, means a saturated, straight or branched hydrocarbon chain radical. In some instances, the number of carbon atoms in an alkyl moiety is indicated by the prefix "Cₓ-C_{y}", wherein x is the minimum and y is the maximum number of carbon atoms in the substituent. Thus, for example, "C₁-C₆ alkyl" means an alkyl substituent containing from 1 to 6 carbon atoms and "C₁-C₃ alkyl" means an alkyl substituent containing from 1 to 3 carbon atoms. Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 1-methylbutyl, 2-methylbutyl, 3-methylbutyl, 3,3-dimethylbutyl, 1,1-dimethylpropyl, 1,2-dimethylpropyl, 2,2-dimethylpropyl, 1-methylpropyl, 2-methylpropyl, 1-ethylpropyl, and 1,2,2-trimethylpropyl. The terms "alkyl," "C₁-C₆ alkyl," "C₁-C₄ alkyl," and "C₁-C₃ alkyl" used herein are unsubstituted, unless otherwise indicated.

The term "alkylene" or "alkylenyl" means a divalent radical derived from a straight or branched, saturated hydrocarbon chain, for example, of 1 to 10 carbon atoms or of 1 to 6 carbon atoms (C₁-C₆ alkylenyl) or of 1 to 4 carbon atoms (C₁-C₄ alkylenyl)or of 1 to 3 carbon atoms (C₁-C₃ alkylenyl) or of 2 to 6 carbon atoms (C₂-C₆ alkylenyl). Examples of C₁-C₆ alkylenyl include, but are not limited to, -CH₂-, -CH₂CH₂-, -C((CH₃)₂)-CH₂CH₂CH₂-, -C((CH₃)₂)-CH₂CH₂, -CH₂CH₂CH₂CH₂-, and -CH₂CH(CH₃)CH₂-.

The term "C₂-C₆ alkynyl" as used herein, means a straight or branched chain hydrocarbon radical containing from 2 to 6 carbon atoms and containing at least one carbon-carbon triple bond. Representative examples of C₂-C₆ alkynyl include, but are not limited, to acetylenyl, 1-propynyl, 2-propynyl, 3-butynyl, 2-pentynyl, and 1-butynyl.

The term "C₃-C₆ monocyclic cycloalkyl" means cyclopropyl, cyclobutyl, cyclopentyl, or cyclohexyl.

The term "halo" or "halogen" as used herein, means Cl, Br, I, and F.

The term "haloalkyl" as used herein, means an alkyl group, as defined herein, in which one, two, three, four, five, or six hydrogen atoms are replaced by halogen. The term "C₁-C₆ haloalkyl" means a C₁-C₆ alkyl group, as defined herein, in which one, two, three, four, five, or six hydrogen atoms are replaced by halogen. The term "C₁-C₃ haloalkyl" means a C₁-C₃ alkyl group, as defined herein, in which one, two, three, four, or five hydrogen atoms are replaced by halogen. Representative examples of haloalkyl include, but are not limited to, chloromethyl, 2-fluoroethyl, 2,2-difluoroethyl, fluoromethyl, 2,2,2-trifluoroethyl, trifluoromethyl, difluoromethyl, pentafluoroethyl, 2-chloro-3-fluoropentyl, trifluorobutyl, and trifluoropropyl.

The term "monocyclic heteroaryl" as used herein, means a five- or six-membered hydrocarbon ring wherein at least one carbon ring atom is replaced by heteroatom independently selected from the group consisting of O, N, and S. The five-membered ring contains two double bonds. The five membered ring may have one heteroatom selected from O or S; or one, two, three, or four nitrogen atoms and optionally one oxygen or one sulfur atom. The six-membered ring contains three double bonds and one, two, three, or four nitrogen atoms. Examples of monocyclic heteroaryl include, but are not limited to, furanyl, imidazolyl, isoxazolyl, isothiazolyl, oxadiazolyl, 1,3-oxazolyl, pyridinyl, pyridazinyl, pyrimidinyl, pyrazinyl, pyrazolyl, pyrrolyl, tetrazolyl, thiadiazolyl, 1,3-thiazolyl, thienyl, triazolyl, and triazinyl. The nitrogen atom in the heteroaryl rings may optionally be oxidized and may optionally be quaternized.

The term "4-6 membered monocyclic heterocycle" as used herein, means a monocyclic hydrocarbon ring radical of 4-6 carbon ring atoms wherein at least one carbon ring atom is replaced by heteroatom independently selected from the group consisting of O, N, and S. A four-membered monocyclic heterocycle contains zero or one double bond, and one carbon ring atom replaced by a heteroatom selected from the group consisting of O, N, and S. A five-membered monocyclic heterocycle contains zero or one double bond and one, two, or three carbon ring atoms replaced by heteroatoms selected from the group consisting of O, N, and S. Examples of five-membered monocyclic heterocycles include those containing in the ring: 1 O; 1 S; 1 N; 2 N; 3 N; 1 S and 1 N; 1 S, and 2 N; 1 O and 1 N; or 1 O and 2 N. Non limiting examples of 5-membered monocyclic heterocyclic groups include 1,3-dioxolanyl, tetrahydrofuranyl, dihydrofuranyl, tetrahydrothienyl, dihydrothienyl, imidazolidinyl, oxazolidinyl, imidazolinyl, isoxazolidinyl, isothiazolidinyl, pyrazolidinyl, pyrazolinyl, pyrrolidinyl, 2-pyrrolinyl, 3-pyrrolinyl, thiazolinyl, and thiazolidinyl. A six-membered monocyclic heterocycle contains zero, one, or two double bonds and one, two, or three carbon ring atoms replaced by heteroatoms selected from the group consisting of O, N, and S. Examples of six-membered monocyclic heterocycles include those containing in the ring: 1 O; 2 O; 1 S; 2 S; 1 N; 2 N; 3 N; 1 S, 1 O, and 1 N; 1 S and 1 N; 1 S and 2 N; 1 S and 1 O; 1 S and 2 O; 1 O and 1 N; and 1 O and 2 N. Examples of six-membered monocyclic heterocycles include 1,3-oxazinanyl, tetrahydropyranyl, dihydropyranyl, 1,6-dihydropyridazinyl, 1,2-dihydropyrimidinyl, 1,6-dihydropyrimidinyl, dioxanyl, 1,4-dithianyl, hexahydropyrimidinyl, morpholinyl, piperazinyl, piperidinyl, 1,2,3,6-tetrahydropyridinyl, tetrahydrothiopyranyl, thiomorpholinyl, thioxanyl, and trithianyl. Examples of monocyclic heterocycles include, but are not limited to, azetidinyl, aziridinyl, diazepanyl, 1,3-dioxanyl, 1,3-dioxolanyl, 1,3-dithiolanyl, 1,3-dithianyl, 1,6-dihydropyridazinyl, 1,2-dihydropyrimidinyl, 1,6-dihydropyrimidinyl, hexahydropyrimidinyl, imidazolinyl, imidazolidinyl, isoindolinyl, isothiazolinyl, isothiazolidinyl, isoxazolinyl, isoxazolidinyl, morpholinyl, oxadiazolinyl, oxadiazolidinyl, 1,3-oxazinanyl, oxazolinyl, 1,3-oxazolidinyl, oxetanyl, piperazinyl, piperidinyl, pyranyl, pyrazolinyl, pyrazolidinyl, pyrrolinyl, pyrrolidinyl, 1,2-dihydropyridinyl, tetrahydrofuranyl, tetrahydropyridinyl, tetrahydropyrimidinyl, tetrahydropyranyl, tetrahydrothienyl, thiadiazolinyl, thiadiazolidinyl, thiazolinyl, thiazolidinyl, thiomorpholinyl, thiopyranyl, and trithianyl. The nitrogen and sulfur heteroatoms in the heterocycle rings may optionally be oxidized (e.g. 1,1-dioxidotetrahydrothienyl, 1,1-dioxido-1,2-thiazolidinyl, 1,1-dioxidothiomorpholinyl)) and the nitrogen atoms may optionally be quaternized.

The phenyl, the C₃-C₆ monocyclic cycloalkyl, the monocyclic heteroaryls, and the monocyclic heterocycles, including the exemplary rings, are optionally substituted unless otherwise indicated; and are attached to the parent molecular moiety through any substitutable atom contained within the ring system.

The term "heteroatom" as used herein, means a nitrogen, oxygen, and sulfur.

The term "oxo" as used herein, means a =O group.

The term "radiolabel" means a compound of the invention in which at least one of the atoms is a radioactive atom or a radioactive isotope, wherein the radioactive atom or isotope spontaneously emits gamma rays or energetic particles, for example alpha particles or beta particles, or positrons. Examples of such radioactive atoms include, but are not limited to, ³H (tritium), ¹⁴C, ¹¹C, ¹⁵O, ¹⁸F, ³⁵S, ¹²³I, and ¹²⁵I.

A moiety is described as "substituted" when a non-hydrogen radical is in the place of hydrogen radical of any substitutable atom of the moiety. Thus, for example, a substituted heterocycle moiety is a heterocycle moiety in which at least one non-hydrogen radical is in the place of a hydrogen radical on the heterocycle. It should be recognized that if there are more than one substitution on a moiety, each non-hydrogen radical may be identical or different (unless otherwise stated).

If a moiety is described as being "optionally substituted," the moiety may be either (1) not substituted or (2) substituted. If a moiety is described as being optionally substituted with up to a particular number of non-hydrogen radicals, that moiety may be either (1) not substituted; or (2) substituted by up to that particular number of non-hydrogen radicals or by up to the maximum number of substitutable positions on the moiety, whichever is less. Thus, for example, if a moiety is described as a heteroaryl optionally substituted with up to 3 non-hydrogen radicals, then any heteroaryl with less than 3 substitutable positions would be optionally substituted by up to only as many non-hydrogen radicals as the heteroaryl has substitutable positions. To illustrate, tetrazolyl (which has only one substitutable position) would be optionally substituted with up to one non-hydrogen radical. To illustrate further, if an amino nitrogen is described as being optionally substituted with up to 2 non-hydrogen radicals, then a primary amino nitrogen will be optionally substituted with up to 2 non-hydrogen radicals, whereas a secondary amino nitrogen will be optionally substituted with up to only 1 non-hydrogen radical.

The terms "treat," "treating," and "treatment" refer to a method of alleviating or abrogating a disease and/or its attendant symptoms. In certain embodiments, "treat," "treating," and "treatment" refer to ameliorating at least one physical parameter, which may not be discernible by the subject. In yet another embodiment, "treat", "treating", and "treatment" refer to modulating the disease or disorder, either physically (for example, stabilization of a discernible symptom), physiologically (for example, , stabilization of a physical parameter), or both. In a further embodiment, "treat", "treating", and "treatment" refer to slowing the progression of the disease or disorder.

The terms "prevent", "preventing", and "prevention" refer to a method of preventing the onset of a disease and/or its attendant symptoms or barring a subject from acquiring a disease. As used herein, "prevent", "preventing" and "prevention" also include delaying the onset of a disease and/or its attendant symptoms and reducing a subject's risk of acquiring or developing a disease or disorder.

The phrase "therapeutically effective amount" means an amount of a compound, or a pharmaceutically acceptable salt thereof, sufficient to prevent the development of or to alleviate to some extent one or more of the symptoms of the condition or disorder being treated when administered alone or in conjunction with another therapeutic agent for treatment in a particular subject or subject population. The "therapeutically effective amount" may vary depending on the compound, the disease and its severity, and the age, weight, health, etc., of the subject to be treated. For example in a human or other mammal, a therapeutically effective amount may be determined experimentally in a laboratory or clinical setting, or may be the amount required by the guidelines of the United States Food and Drug Administration, or equivalent foreign agency, for the particular disease and subject being treated.

The term "subject" is defined herein to refer to animals such as mammals, including, but not limited to, primates (e.g., humans), cows, sheep, goats, pigs, horses, dogs, cats, rabbits, rats, mice and the like. In one embodiment, the subject is a human. The terms "human," "patient," and "subject" are used interchangeably herein.

The term 'at least one additional therapeutic agent' means one to four therapeutic agents other than the compounds of the invention. In one embodiment it means one to three additional therapeutic agents. In further embodiments it means one or two additional therapeutic agents. In a yet further embodiment it means one additional therapeutic agent. In a yet further embodiment it means two additional therapeutic agents. In a yet further embodiment it means three additional therapeutic agents.

### b. Compounds

Compounds of the invention have the general formula (I) as described above.

Particular values of variable groups in compounds of formula (I) are as follows. Such values may be used where appropriate with any of the other values, definitions, claims or embodiments defined hereinbefore or hereinafter.

In certain embodiments, the invention is directed to compounds of formula (I-a) 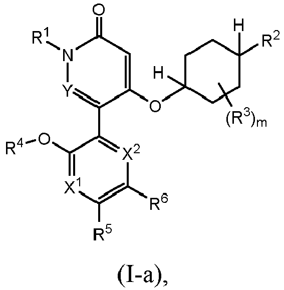 wherein R¹, Y, X¹, X², R², R³, R⁴, R⁵, R⁶, and m are defined above in the Summary of the Invention and embodiments herein below.

In certain embodiments, the invention is directed to compounds of formula (I-b) 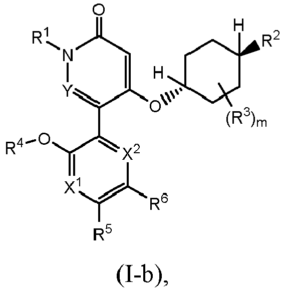 wherein R¹, Y, X¹, X², R², R³, R⁴, R⁵, R⁶, and m are defined above in the Summary of the Invention and embodiments herein below.

In certain embodiments, R¹ is CH₃.

In certain embodiments, Y is N.

In certain embodiments, Y is C(R^{Y}) wherein R^{Y} is hydrogen or C₁-C₃ alkyl. In some such embodiments, R^{Y} is hydrogen. In some such embodiments, R^{Y} is C₁-C₃ alkyl. In some such embodiments, R^{Y} is ethyl.

In certain embodiments, m is 0, 1, or 2. In some such embodiments, m is 0. In some such embodiments, m is 1.

In certain embodiments, R² is G^{1A}.

In certain embodiments, R² is G^{1A} wherein Z¹ is C(O).

In certain embodiments, R² is G^{1A} wherein Z¹ is C(R^{e})(R^{f}).

In certain embodiments, R² is G^{1A} wherein Z¹ is C(R^{e})(R^{f}) and Z² is a bond.

In certain embodiments, R² is G^{1A} wherein Z² is -(C(R^{g})(R^{h}))ₚ-C(R^{m})(Rⁿ)-; and p is 0.

In certain embodiments, R² is G^{1A} wherein Z² is -(C(R^{g})(R^{h}))ₚ-C(R^{m})(Rⁿ)-; and p is 1.

In certain embodiments, R² is G^{1A} wherein Z² is N(Rⁱ).

In certain embodiments, R² is G^{1A} wherein Z² is O.

In certain embodiments, R² is -N(R^{a})S(O)₂R^{b}.

In certain embodiments, R² is -N(R^{a})S(O)₂R^{b} wherein R^{a} is hydrogen or C₁-C₃ alkyl, and R^{b} is C₁-C₆ alkyl.

In certain embodiments, R² is -N(R^{a})C(O)R^{b}.

In certain embodiments, R² is -N(R^{a})C(O)R^{b} wherein R^{a} is hydrogen or C₁-C₃ alkyl, and R^{b} is C₁-C₆ alkyl, C₂-C₆ alkenyl, C₁-C₆ haloalkyl, G^{1B}, or -(C₁-C₆ alkylenyl)-OR^{j}.

In certain embodiments, R² is -N(R^{a})C(O)OR^{b}.

In certain embodiments, R² is -N(R^{a})C(O)OR^{b} wherein R^{a} is hydrogen or C₁-C₃ alkyl, and R^{b} is C₁-C₆ alkyl.

In certain embodiments, R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.

In certain embodiments, R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen and -(C₁-C₆ alkylenyl)-OH.

In certain embodiments, R⁴ is monocyclic heteroaryl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.

In certain embodiments, R⁴ is pyridinyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.

In certain embodiments, R⁴ is pyridinyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen and -(C₁-C₆ alkylenyl)-OH.

In certain embodiments, R⁵ is hydrogen.

In certain embodiments, X¹ is N or C(R⁷); and X² is C(R⁷).

In certain embodiments, X¹ and X² are C(R⁷). In some such embodiments, R⁷ is hydrogen.

In certain embodiments, X¹ is N and X² is C(R⁷). In some such embodiments, R⁷ is hydrogen.

In certain embodiments, n is 0.

In certain embodiments, n is 1.

In certain embodiments, R⁶ is -S(O)₂R^{6a}. In some such embodiments, R^{6a} is C₁-C₆ alkyl.

In certain embodiments, R⁶ is -(CH₂)-S(O)₂R^{6a}. In some such embodiments, R^{6a} is C₁-C₆ alkyl.

In certain embodiments, R⁶ is -N(R^{6b})S(O)₂R^{6c}. In some such embodiments, R^{6b} is hydrogen. In some such embodiments, R^{6c} is C₁-C₆ alkyl.

Various embodiments of substituents R¹, Y, R^{Y}, X¹, X², R², R³, R⁴, R⁵, R⁶, R^{6a}, R^{6b}, R^{6c}, R⁷, G^{1A}, G^{1B}, R^{a}, R^{b}, and m, have been discussed above. These substituents embodiments may be combined to form various embodiments of present compounds. All embodiments of compounds of the invention, formed by combining the substituent embodiments discussed above are within the scope of Applicant's invention, and some illustrative embodiments of the compounds of the invention are provided below.

In one embodiment, the invention is directed to compounds of formula (I), (I-a), and (I-b), wherein Y is C(R^{Y}); X¹ is N or C(R⁷); and X² is C(R⁷).

In one embodiment, the invention is directed to compounds of formula (I), (I-a), and (I-b), wherein Y is C(R^{Y}); and X¹ and X² are C(R⁷).

In one embodiment, the invention is directed to compounds of formula (I), (I-a), and (I-b), wherein Y is C(R^{Y}); X¹ is N; and X² is C(R⁷).

In one embodiment, the invention is directed to compounds of formula (I), (I-a), and (I-b), wherein
Y is C(R^{Y});
X¹ is N or C(R⁷);
X² is C(R⁷); and
R² is G^{1A}, -N(R^{a})C(O)R^{b}, or -N(R^{a})C(O)OR^{b}.

In some such embodiments, X¹ is and X² are C(R⁷). In some such embodiments, X¹ is N, and X² is C(R⁷).

In one embodiment, the invention is directed to compounds of formula (I), (I-a), and (I-b), wherein
Y is C(R^{Y});
X¹ is N or C(R⁷);
X² is C(R⁷); and
R² is G^{1A}.

In some such embodiments, X¹ is and X² are C(R⁷). In some such embodiments, X¹ is N, and X² is C(R⁷).

In one embodiment, the invention is directed to compounds of formula (I), (I-a), and (I-b), wherein
Y is C(R^{Y});
X¹ is N or C(R⁷);
X² is C(R⁷); and
R² is -N(R^{a})C(O)R^{b}.

In some such embodiments, X¹ and X² are C(R⁷). In some such embodiments, X¹ is N, and X² is C(R⁷).

In one embodiment, the invention is directed to compounds of formula (I), (I-a), and (I-b), wherein
Y is C(R^{Y});
X¹ is N or C(R⁷);
X² is C(R⁷); and
R² is -N(R^{a})C(O)OR^{b}.

In some such embodiments, X¹ and X² are C(R⁷). In some such embodiments, X¹ is N, and X² is C(R⁷).

In one embodiment, the invention is directed to compounds of formula (I), (I-a), and (I-b), wherein
Y is C(R^{Y});
X¹ is N or C(R⁷);
X² is C(R⁷); and
R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.

In some such embodiments, X¹ and X² are C(R⁷). In some such embodiments, X¹ is N, and X² is C(R⁷).

In one embodiment, the invention is directed to compounds of formula (I), (I-a), and (I-b), wherein
R² is G^{1A}; and
R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.

In some such embodiments, the optional substituents of R⁴ are independently selected from the group consisting of halogen and -(C₁-C₆ alkylenyl)-OH.

In one embodiment, the invention is directed to compounds of formula (I), (I-a), and (I-b), wherein
Y is C(R^{Y});
X¹ is N or C(R⁷);
X² is C(R⁷);
R² is G^{1A}; and
R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.

In some such embodiments, X¹ and X² are C(R⁷). In some such embodiments, X¹ is N, and X² is C(R⁷).

In some such embodiments, the optional substituents of R⁴ are independently selected from the group consisting of halogen and -(C₁-C₆ alkylenyl)-OH.

In one embodiment, the invention is directed to compounds of formula (I), (I-a), and (I-b), wherein
R² is -N(R^{a})C(O)R^{b}; and
R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.

In some such embodiments, the optional substituents of R⁴ are independently selected from the group consisting of halogen and -(C₁-C₆ alkylenyl)-OH.

In one embodiment, the invention is directed to compounds of formula (I), (I-a), and (I-b), wherein
Y is C(R^{Y});
X¹ is N or C(R⁷);
X² is C(R⁷);
R² is -N(R^{a})C(O)R^{b}; and
R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.

In some such embodiments, X¹ and X² are C(R⁷). In some such embodiments, X¹ is N, and X² is C(R⁷).

In some such embodiments, the optional substituents of R⁴ are independently selected from the group consisting of halogen and -(C₁-C₆ alkylenyl)-OH.

In one embodiment, the invention is directed to compounds of formula (I), (I-a), and (I-b), wherein
R² is -N(R^{a})C(O)OR^{b}; and
R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.

In some such embodiments, the optional substituents of R⁴ are independently selected from the group consisting of halogen and -(C₁-C₆ alkylenyl)-OH.

In one embodiment, the invention is directed to compounds of formula (I), (I-a), and (I-b), wherein
Y is C(R^{Y});
X¹ is N or C(R⁷);
X² is C(R⁷);
R² is -N(R^{a})C(O)OR^{b}; and
R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.

In some such embodiments, X¹ and X² are C(R⁷). In some such embodiments, X¹ is N, and X² is C(R⁷).

In some such embodiments, the optional substituents of R⁴ are independently selected from the group consisting of halogen and -(C₁-C₆ alkylenyl)-OH.

Compound of the invention are named by using Name 2015 naming algorithm by Advanced Chemical Development or Struct=Name naming algorithm as part of CHEMDRAW® ULTRA v. 12.0.2.1076.

Compounds of the invention may exist as stereoisomers wherein asymmetric or chiral centers are present. These stereoisomers are "*R*" or "*S*" depending on the configuration of substituents around the chiral carbon atom. The terms "*R*" and "*S*" used herein are configurations as defined in IUPAC 1974 Recommendations for Section E, Fundamental Stereochemistry, in Pure Appl. Chem., 1976, 45: 13-30. The invention contemplates various stereoisomers and mixtures thereof and these are specifically included within the scope of this invention. Stereoisomers include enantiomers and diastereomers, and mixtures of enantiomers or diastereomers. Individual stereoisomers of compounds of the invention may be prepared synthetically from commercially available starting materials which contain asymmetric or chiral centers or by preparation of racemic mixtures followed by methods of resolution well-known to those of ordinary skill in the art. These methods of resolution are exemplified by (1) attachment of a mixture of enantiomers to a chiral auxiliary, separation of the resulting mixture of diastereomers by recrystallization or chromatography and optional liberation of the optically pure product from the auxiliary as described in Furniss, Hannaford, Smith, and Tatchell, "Vogel's Textbook of Practical Organic Chemistry", 5th edition (1989), Longman Scientific & Technical, Essex CM20 2JE, England, or (2) direct separation of the mixture of optical enantiomers on chiral chromatographic columns or (3) fractional recrystallization methods.

Compounds of the invention may exist as *cis* or *trans* isomers, wherein substituents on a ring may attached in such a manner that they are on the same side of the ring *(cis)* relative to each other, or on opposite sides of the ring relative to each other *(trans).* For example, cyclobutane may be present in the *cis* or *trans* configuration, and may be present as a single isomer or a mixture of the *cis* and *trans* isomers. Individual *cis* or *trans* isomers of compounds of the invention may be prepared synthetically from commercially available starting materials using selective organic transformations, or prepared in single isomeric form by purification of mixtures of the *cis* and *trans* isomers. Such methods are well-known to those of ordinary skill in the art, and may include separation of isomers by recrystallization or chromatography.

It should be understood that the compounds of the invention may possess tautomeric forms, as well as geometric isomers, and that these also constitute an aspect of the invention.

The present disclosure includes all pharmaceutically acceptable isotopically-labelled compounds of formula (I), (I-a), or (I-b) wherein one or more atoms are replaced by atoms having the same atomic number, but an atomic mass or mass number different from the atomic mass or mass number which predominates in nature. Examples of isotopes suitable for inclusion in the compounds of the disclosure include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S. Certain isotopically-labelled compounds of formula (I), (I-a), or (I-b), for example, those incorporating a radioactive isotope, are useful in drug and/or substrate tissue distribution studies. The radioactive isotopes tritium, *i.e.* ³H, and carbon-14, *i.e.* ¹⁴C, are particularly useful for this purpose in view of their ease of incorporation and ready means of detection. Substitution with heavier isotopes such as deuterium, *i.e.* ²H, may afford certain therapeutic advantages resulting from greater metabolic stability, for example, increased *in vivo* half-life or reduced dosage requirements, and hence may be preferred in some circumstances. Substitution with positron emitting isotopes, such as ¹¹C, ¹⁸F, ¹⁵O and ¹³N, may be useful in Positron Emission Topography (PET) studies for examining substrate receptor occupancy. Isotopically-labeled compounds of formula (I), (I-a), or (I-b) may generally be prepared by conventional techniques known to those skilled in the art or by processes analogous to those described in the accompanying Examples using an appropriate isotopically-labeled reagents in place of the non-labeled reagent previously employed.

Thus, the formula drawings within this specification can represent only one of the possible tautomeric, geometric, or stereoisomeric forms. It is to be understood that the invention encompasses any tautomeric, geometric, or stereoisomeric form, and mixtures thereof, and is not to be limited merely to any one tautomeric, geometric, or stereoisomeric form utilized within the formula drawings.

Exemplary compounds include, but are not limited to:
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]acetamide;
N-[cis-4-({5-[2-(2,6-dimethylphenoxy)-5-(ethanesulfonyl)phenyl]-1-methyl-2-oxo-1,2-dihydropyridin-4-yl}oxy)cyclohexyl]acetamide;
methyl [trans-4-({5-[2-(2,6-dimethylphenoxy)-5-(ethanesulfonyl)phenyl]-1-methyl-2-oxo-1,2-dihydropyridin-4-yl}oxy)cyclohexyl]carbamate;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]methanesulfonamide;
tert-butyl[trans-4-({5-[2-(2,6-dimethylphenoxy)-5-(ethanesulfonyl)phenyl]-1-methyl-2-oxo-1,2-dihydropyridin-4-yl}oxy)cyclohexyl]carbamate;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-2,2,2-trifluoroacetamide;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]propanamide;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-2,2-dimethylpropanamide;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-2-methoxyacetamide;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-1-methylcyclopropane-1-carboxamide;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]cyclopropanecarboxamide;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-3-methyloxetane-3-carboxamide;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-3,3-difluorocyclobutane-1-carboxamide;
N-{trans-4-[(5-{5-[(ethanesulfonyl)amino]-2-[4-(2-hydroxypropan-2-yl)-2,6-dimethylphenoxy]phenyl}-1-methyl-2-oxo-1,2-dihydropyridin-4-yl)oxy]cyclohexyl}acetamide;
methyl {trans-4-[(5-{5-[(ethanesulfonyl)amino]-2-[4-(2-hydroxypropan-2-yl)-2,6-dimethylphenoxy]phenyl}-1-methyl-2-oxo-1,2-dihydropyridin-4-yl)oxy]cyclohexyl}carbamate;
methyl {trans-4-[(5-{5-[(ethanesulfonyl)amino]-2-(4-fluoro-2,6-dimethylphenoxy)phenyl}-1-methyl-2-oxo-1,2-dihydropyridin-4-yl)oxy]cyclohexyl}carbamate;
methyl [trans-4-({5'-[(ethanesulfonyl)amino]-2'-(4-fluoro-2,6-dimethylphenoxy)-1-methyl-6-oxo[1,6-dihydro[3,3'-bipyridine]]-4-yl}oxy)cyclohexyl]carbamate;
methyl {trans-4-[(5-{2-(4-fluoro-2,6-dimethylphenoxy)-5-[(methanesulfonyl)methyl]phenyl}-1-methyl-2-oxo-1,2-dihydropyridin-4-yl)oxy]cyclohexyl}carbamate;
5-[5-(ethanesulfonyl)-2-(4-fluoro-2,6-dimethylphenoxy)phenyl]-1-methyl-4-{[trans-4-(2-oxopyrrolidin-1-yl)cyclohexyl]oxy}pyridin-2(1H)-one; and
N-[trans-4-({5-[5-(ethanesulfonyl)-2-(4-fluoro-2,6-dimethylphenoxy)phenyl]-1-methyl-2-oxo-1,2-dihydropyridin-4-yl}oxy)cyclohexyl]-N-methylacetamide.

Compounds of formula (I), (I-a), or (I-b) may be used in the form of pharmaceutically acceptable salts. The phrase "pharmaceutically acceptable salt" means those salts which are, within the scope of sound medical judgement, suitable for use in contact with the tissues of humans and lower animals without undue toxicity, irritation, allergic response and the like and are commensurate with a reasonable benefit/risk ratio.

Pharmaceutically acceptable salts have been described in S. M. Berge et al. J. Pharmaceutical Sciences, 1977, 66: 1-19.

Compounds of formula (I), (I-a), or (I-b) may contain either a basic or an acidic functionality, or both, and may be converted to a pharmaceutically acceptable salt, when desired, by using a suitable acid or base. The salts may be prepared in situ during the final isolation and purification of the compounds of the invention.

Examples of acid addition salts include, but are not limited to acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, fumarate, hydrochloride, hydrobromide, hydroiodide, 2-hydroxyethansulfonate (isothionate), lactate, malate, maleate, methanesulfonate, nicotinate, 2-naphthalenesulfonate, oxalate, palmitoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups may be quaternized with such agents as lower alkyl halides such as, but not limited to, methyl, ethyl, propyl, and butyl chlorides, bromides and iodides; dialkyl sulfates like dimethyl, diethyl, dibutyl and diamyl sulfates; long chain halides such as, but not limited to, decyl, lauryl, myristyl and stearyl chlorides, bromides and iodides; arylalkyl halides like benzyl and phenethyl bromides and others. Water or oil-soluble or dispersible products are thereby obtained. Examples of acids which may be employed to form pharmaceutically acceptable acid addition salts include such inorganic acids as hydrochloric acid, hydrobromic acid, sulfuric acid, and phosphoric acid and such organic acids as acetic acid, fumaric acid, maleic acid, 4-methylbenzenesulfonic acid, succinic acid and citric acid.

Basic addition salts may be prepared in situ during the final isolation and purification of compounds of this invention by reacting a carboxylic acid-containing moiety with a suitable base such as, but not limited to, the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as, but not limited to, lithium, sodium, potassium, calcium, magnesium and aluminum salts and the like and nontoxic quaternary ammonia and amine cations including ammonium, tetramethylammonium, tetraethylammonium, methylamine, dimethylamine, trimethylamine, triethylamine, diethylamine, ethylamine and the like. Other examples of organic amines useful for the formation of base addition salts include ethylenediamine, ethanolamine, diethanolamine, piperidine, piperazine and the like.

The term "pharmaceutically acceptable prodrug" or "prodrug"as used herein, represents those prodrugs of the compounds of the present invention which are, within the scope of sound medical judgement, suitable for use in contact with the tissues of humans and lower animals without undue toxicity, irritation, allergic response, and the like, commensurate with a reasonable benefit/risk ratio, and effective for their intended use.

The present invention contemplates compounds of formula (I), (I-a), or (I-b) formed by synthetic means or formed by in vivo biotransformation of a prodrug.

Compounds described herein may exist in unsolvated as well as solvated forms, including hydrated forms, such as hemi-hydrates. In general, the solvated forms, with pharmaceutically acceptable solvents such as water and ethanol among others are equivalent to the unsolvated forms for the purposes of the invention.

### c. General Synthesis

The compounds described herein, including compounds of general formula (I), (I-a), or (I-b) and specific examples, may be prepared, for example, through the reaction routes depicted in schemes 1-3. The variables X¹, X², R¹, R², R³, R⁴, R⁵, R⁶, R^{a}, R^{b}, Y, and m used in the following schemes have the meanings as set forth in the summary and detailed description sections unless otherwise noted.

Abbreviations used in the descriptions of the schemes and the specific examples have the following meanings: DMSO for dimethyl sulfoxide, psi for pounds per square inch, HPLC for high performance liquid chromatography, LC/MS for liquid chromatography/mass spectrometry, and MPLC for medium performance liquid chromatography.

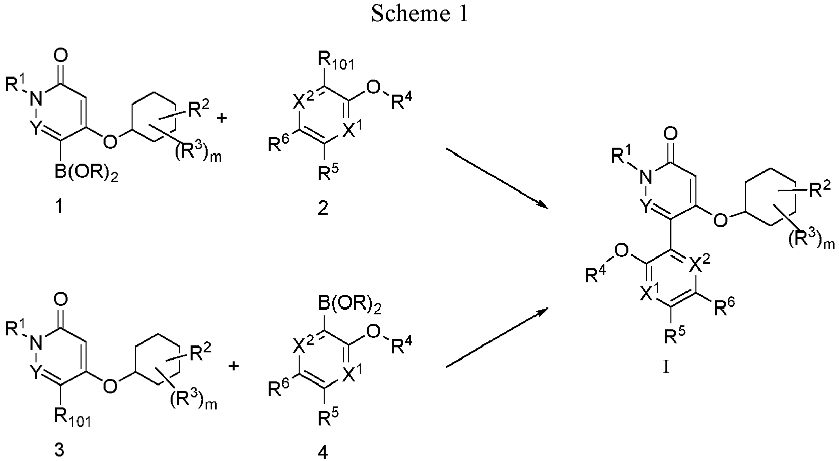

Compounds of general formula (I) may be prepared as shown in Scheme 1 by reaction of boronic acids or a derivative thereof (e.g., a pinacol ester) of formula (1) with compounds of formula (2), wherein R₁₀₁ is Cl, Br, I, or triflate, under Suzuki coupling conditions (N. Miyama and A. Suzuki, Chem. Rev. 1995, 95:2457-2483, J. Organomet. Chem. 1999, 576:147-148). For example, the coupling reaction may be conducted in the presence of a palladium catalyst and a base, and optionally in the presence of a ligand, and in a suitable solvent at elevated temperature (about 60 °C to about 150 °C). The reaction may be facilitated by microwave irradiation. Examples of the palladium catalyst include, but are not limited to, tetrakis(triphenylphosphine)palladium(0), tris(dibenzylideneacetone)dipalladium(0), bis(triphenylphosphine)palladium(II) dichloride, and palladium(II)acetate. Examples of suitable bases that may be employed include, but not limited to, carbonates, acetates, or phosphates of sodium, potassium, and cesium, and cesium fluoride. Examples of suitable ligands include, but are not limited to, 1,3,5,7-tetramethyl-8-phenyl-2,4,6-trioxa-8-phosphaadamante, 2-dicyclohexylphosphino-2',4',6'-triisopropylbiphenyl (X-phos), and 1,1'-bis(diphenylphosphanyl) ferrocene. Non-limiting examples of suitable solvent include methanol, ethanol, dimethoxyethane, N,N-dimethylformamide, dimethylsulfoxide, dioxane, tetrahydrofuran, toluene, and water, or a mixture thereof.

Compounds of general formula (I) may also be prepared as shown in Scheme 1 by reaction of boronic acids or a derivative thereof (e.g., a pinacol ester) of formula (4) with compounds of formula (3), wherein R₁₀₁ is Cl, Br, I, or triflate, under Suzuki coupling conditions as described above.

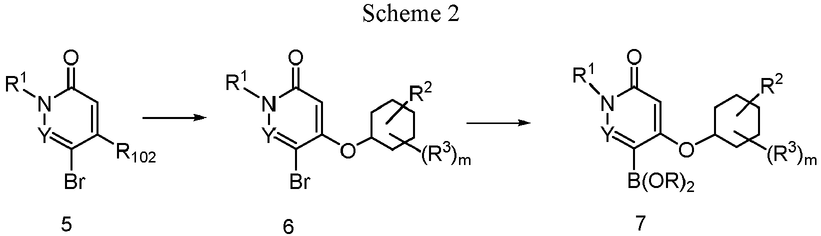

Compounds of formula (1) and formula (3) wherein R₁₀₁ is Br may be prepared using general synthetic route as shown in Scheme 2. Displacement of the halogen of compounds of formula (5), wherein R₁₀₂ is Cl or F, with appropriate alcohols may be accomplished in a solvent such as, but not limited to, dimethylsulfoxide, dimethylformamide, dioxane, or tetrahydrofuran and in the presence of a base such as, but not limited to, potassium *tert-*butoxide, carbonate of cesium, potassium, or sodium, or sodium hydride, and at a temperature from about 40 °C to about 120 °C. Treatment of the compounds of formula (6) with 4,4,4',4',5,5,5',5'-octamethyl-2,2'-bi(1,3,2-dioxaborolane) generally affords compounds of formula (7). In general, the conversion may be facilitated by a palladium catalyst such as, but not limited to, tetrakis(triphenylphosphine)palladium(0), tris(dibenzylideneacetone)dipalladium(0), or palladium(II)acetate, an optional ligand such as, but not limited to, 2-dicyclohexylphosphino-2',4',6'-triisopropylbiphenyl, 2-dicyclohexylphosphino-2',4',6'-triisopropylbiphenyl (X-phos), or 1,1'-bis( diphenylphosphanyl) ferrocene, and a base such as, but not limited to, carbonates, acetates, or phosphates.of sodium, potassium, and cesium; and cesium fluoride. Non-limiting examples of suitable solvents include methanol, dimethoxyethane, N,N-dimethylformamide, dimethylsulfoxide, dioxane, tetrahydrofuran, and water, or a mixture thereof.

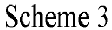

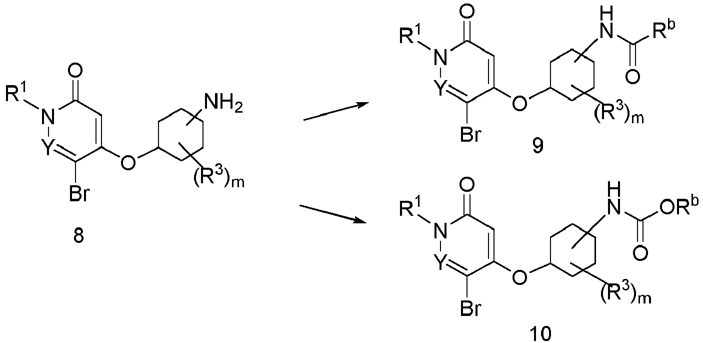

Representative examples of compound of formula (3) wherein R₁₀₁ is bromo, and G¹ is substituted with N(H)C(O)R^{b} or N(H)C(O)OR^{b} may be prepared as shown as illustrated in Scheme 3.

Transformation of (8) to the corresponding amides (9) and carbamates (10) are known to one skilled in the art, for example, by treatment with suitable anhydrides or suitable chloroformates respectively, in the presence of a base such as, but not limited to, trimethylamine.

It can be appreciated that the synthetic schemes and specific examples as illustrated in the synthetic examples section are illustrative and are not to be read as limiting the scope of the invention as it is defined in the appended claims. All alternatives, modifications, and equivalents of the synthetic methods and specific examples are included within the scope of the claims.

Optimum reaction conditions and reaction times for each individual step can vary depending on the particular reactants employed and substituents present in the reactants used. Unless otherwise specified, solvents, temperatures and other reaction conditions may be readily selected by one of ordinary skill in the art. Specific procedures are provided in the Synthetic Examples section. Reactions may be worked up in the conventional manner, e.g. by eliminating the solvent from the residue and further purified according to methodologies generally known in the art such as, but not limited to, crystallization, distillation, extraction, trituration and chromatography. Unless otherwise described, the starting materials and reagents are either commercially available or may be prepared by one skilled in the art from commercially available materials using methods described in the chemical literature.

Routine experimentations, including appropriate manipulation of the reaction conditions, reagents and sequence of the synthetic route, protection of any chemical functionality that can not be compatible with the reaction conditions, and deprotection at a suitable point in the reaction sequence of the method are included in the scope of the invention. Suitable protecting groups and the methods for protecting and deprotecting different substituents using such suitable protecting groups are well known to those skilled in the art; examples of which can be found in T. Greene and P. Wuts, Protecting Groups in Organic Synthesis (3rd ed.), John Wiley & Sons, NY (1999), which is incorporated herein by reference in its entirety. Synthesis of the compounds of the invention can be accomplished by methods analogous to those described in the synthetic schemes described hereinabove and in specific examples.

Starting materials, if not commercially available, may be prepared by procedures selected from standard organic chemical techniques, techniques that are analogous to the synthesis of known, structurally similar compounds, or techniques that are analogous to the above described schemes or the procedures described in the synthetic examples section.

When an optically active form of a compound is required, it may be obtained by carrying out one of the procedures described herein using an optically active starting material (prepared, for example, by asymmetric induction of a suitable reaction step), or by resolution of a mixture of the stereoisomers of the compound or intermediates using a standard procedure (such as chromatographic separation, recrystallization or enzymatic resolution).

Similarly, when a pure geometric isomer of a compound is required, it may be prepared by carrying out one of the above procedures using a pure geometric isomer as a starting material, or by resolution of a mixture of the geometric isomers of the compound or intermediates using a standard procedure such as chromatographic separation.

### d. Pharmaceutical Compositions

When employed as a pharmaceutical, a compound of the invention is typically administered in the form of a pharmaceutical composition. Such composition may be prepared in a manner well known in the pharmaceutical art and comprise a therapeutically effective amount of a compound of formula (I), (I-a), or (I-b), or a pharmaceutically acceptable salt thereof, alone or in combination with at least one additional therapeutic agent, together with a pharmaceutically acceptable carrier. The phrase "pharmaceutical composition" refers to a composition suitable for administration in medical or veterinary use.

The pharmaceutical compositions that comprise a compound of formula (I), (I-a), or (I-b), alone or in combination with at least one additional therapeutic agent, may be administered to the subjects orally, rectally, parenterally, intracisternally, intravaginally, intraperitoneally, topically (as by powders, ointments or drops), bucally or as an oral or nasal spray. The term "parenterally" as used herein, refers to modes of administration which include intravenous, intramuscular, intraperitoneal, intrasternal, subcutaneous, and intraarticular injection and infusion.

The term "pharmaceutically acceptable carrier" as used herein, means a non-toxic, inert solid, semi-solid or liquid filler, diluent, encapsulating material or formulation auxiliary of any type. Some examples of materials which can serve as pharmaceutically acceptable carriers are sugars such as, but not limited to, lactose, glucose and sucrose; starches such as, but not limited to, corn starch and potato starch; cellulose and its derivatives such as, but not limited to, sodium carboxymethyl cellulose, ethyl cellulose and cellulose acetate; powdered tragacanth; malt; gelatin; talc; excipients such as, but not limited to, cocoa butter and suppository waxes; oils such as, but not limited to, peanut oil, cottonseed oil, safflower oil, sesame oil, olive oil, corn oil and soybean oil; glycols; such a propylene glycol; esters such as, but not limited to, ethyl oleate and ethyl laurate

; agar; buffering agents such as, but not limited to, magnesium hydroxide and aluminum hydroxide; alginic acid; pyrogen-free water; isotonic saline; Ringer's solution; ethyl alcohol, and phosphate buffer solutions, as well as other non-toxic compatible lubricants such as, but not limited to, sodium lauryl sulfate and magnesium stearate, as well as coloring agents, releasing agents, coating agents, sweetening, flavoring and perfuming agents, preservatives and antioxidants can also be present in the composition, according to the judgment of the formulator.

Pharmaceutical compositions for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), vegetable oils (such as olive oil), injectable organic esters (such as ethyl oleate) and suitable mixtures thereof. Proper fluidity may be maintained, for example, by the use of coating materials such as lecithin, by the maintenance of the required particle size in the case of dispersions and by the use of surfactants.

These compositions may also contain adjuvants such as preservatives, wetting agents, emulsifying agents and dispersing agents. Prevention of the action of microorganisms ma be ensured by the inclusion of various antibacterial and antifungal agents, for example, paraben, chlorobutanol, phenol sorbic acid and the like. It may also be desirable to include isotonic agents such as sugars, sodium chloride and the like. Prolonged absorption of the injectable pharmaceutical form may be brought about by the inclusion of agents, which delay absorption such as aluminum monostearate and gelatin.

In some cases, in order to prolong the effect of the drug, it may be desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally-administered drug form may be accomplished by dissolving or suspending the drug in an oil vehicle.

Injectable depot forms may be made by forming microencapsule matrices of the drug in biodegradable polymers such as polylactide-polyglycolide. Depending upon the ratio of drug to polymer and the nature of the particular polymer employed, the rate of drug release may be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations may be sterilized, for example, by filtration through a bacterial-retaining filter or by incorporating sterilizing agents in the form of sterile solid compositions which may be dissolved or dispersed in sterile water or other sterile injectable medium just prior to use.

Solid dosage forms for oral administration include capsules, tablets, pills, powders and granules. In certain embodiments, solid dosage forms may contain from 1% to 95% (w/w) of a compound of formula (I), (I-a), or (I-b). In certain embodiments, the compound of formula (I), (I-a), or (I-b) may be present in the solid dosage form in a range of from 5% to 70% (w/w). In such solid dosage forms, the active compound may be mixed with at least one inert, pharmaceutically acceptable excipient or carrier, such as sodium citrate or dicalcium phosphate and/or a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate, solid polyethylene glycols, sodium lauryl sulfate and mixtures thereof. In the case of capsules, tablets and pills, the dosage form may also comprise buffering agents.

The pharmaceutical composition may be a unit dosage form. In such form the preparation is subdivided into unit doses containing appropriate quantities of the active component. The unit dosage form may be a packaged preparation, the package containing discrete quantities of preparation, such as packeted tablets, capsules, and powders in vials or ampules. Also, the unit dosage form may be a capsule, tablet, cachet, or lozenge itself, or it may be the appropriate number of any of these in packaged form. The quantity of active component in a unit dose preparation may be varied or adjusted from 0.1 mg to 1000 mg, from 1 mg to 100 mg, or from 1% to 95% (w/w) of a unit dose, according to the particular application and the potency of the active component. The composition may, if desired, also contain other compatible therapeutic agents.

The dose to be administered to a subject may be determined by the efficacy of the particular compound employed and the condition of the subject, as well as the body weight or surface area of the subject to be treated. The size of the dose also will be determined by the existence, nature, and extent of any adverse side-effects that accompany the administration of a particular compound in a particular subject. In determining the effective amount of the compound to be administered in the treatment or prophylaxis of the disorder being treated, the physician can evaluate factors such as the circulating plasma levels of the compound, compound toxicities, and/or the progression of the disease, etc. In general, the dose equivalent of a compound is from about 1 µg/kg to 100 mg/kg for a typical subject.

For administration, compounds of the formula (I), (I-a), or (I-b) may be administered at a rate determined by factors that can include, but are not limited to, the LD₅₀ of the compound, the pharmacokinetic profile of the compound, contraindicated drugs, and the side-effects of the compound at various concentrations, as applied to the mass and overall health of the subject. Administration may be accomplished via single or divided doses.

The compounds utilized in the pharmaceutical method of the invention may be administered at the initial dosage of about 0.001 mg/kg to about 100 mg/kg daily. In certain embodiments, the daily dose range is from about 0.1 mg/kg to about 10 mg/kg. The dosages, however, may be varied depending upon the requirements of the subject, the severity of the condition being treated, and the compound being employed. Determination of the proper dosage for a particular situation is within the skill of the practitioner. Treatment may be initiated with smaller dosages, which are less than the optimum dose of the compound. Thereafter, the dosage is increased by small increments until the optimum effect under circumstances is reached. For convenience, the total daily dosage may be divided and administered in portions during the day, if desired.

Solid compositions of a similar type may also be employed as fillers in soft and hard-filled gelatin capsules using such carriers as lactose or milk sugar as well as high molecular weight polyethylene glycols and the like.

The solid dosage forms of tablets, dragees, capsules, pills and granules may be prepared with coatings and shells such as enteric coatings and other coatings well-known in the pharmaceutical formulating art. They may optionally contain opacifying agents and may also be of a composition such that they release the active ingredient(s) only, or preferentially, in a certain part of the intestinal tract, optionally, in a delayed manner. Examples of embedding compositions which may be used include polymeric substances and waxes.

The active compounds may also be in micro-encapsulated form, if appropriate, with one or more of the above-mentioned carriers.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof.

Besides inert diluents, the oral compositions may also include adjuvants such as wetting agents, emulsifying and suspending agents, sweetening, flavoring and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar, tragacanth and mixtures thereof.

Compositions for rectal or vaginal administration are preferably suppositories which may be prepared by mixing the compounds of this invention with suitable non-irritating carriers or carriers such as cocoa butter, polyethylene glycol or a suppository wax which are solid at room temperature but liquid at body temperature and therefore melt in the rectum or vaginal cavity and release the active compound.

Compounds of formula (I), (I-a), or (I-b) may also be administered in the form of liposomes. Liposomes generally may be derived from phospholipids or other lipid substances. Liposomes are formed by mono- or multi-lamellar hydrated liquid crystals which are dispersed in an aqueous medium. Any non-toxic, physiologically acceptable and metabolizable lipid capable of forming liposomes may be used. The present compositions in liposome form may contain, in addition to a compound of formula (I), (I-a), or (I-b), stabilizers, preservatives, excipients and the like. Examples of lipids include, but are not limited to, natural and synthetic phospholipids and phosphatidyl cholines (lecithins), used separately or together.

Methods to form liposomes have been described, see example, Prescott, Ed., Methods in Cell Biology, Volume XIV, Academic Press, New York, N.Y. (1976), p. 33 et seq.

Dosage forms for topical administration of a compound described herein include powders, sprays, ointments and inhalants. The active compound may be mixed under sterile conditions with a pharmaceutically acceptable carrier and any needed preservatives, buffers or propellants which may be required. Opthalmic formulations, eye ointments, powders and solutions are also contemplated as being within the scope of this invention.

### e. Methods of Use

The compounds of formula (I), (I-a), or (I-b), or pharmaceutically acceptable salts thereof, and pharmaceutical compositions comprising a compound of formula (I), (I-a), or (I-b), or a pharmaceutically acceptable salt thereof, may be administered to a subject suffering from a bromodomain-mediated disorder or condition. The term "administering" refers to the method of contacting a compound with a subject. Thus, the compounds of formula (I), (I-a), or (I-b) may be administered by injection, that is, intravenously, intramuscularly, intracutaneously, subcutaneously, intraduodenally, parentally, or intraperitoneally. Also, the compounds described herein may be administered by inhalation, for example, intranasally. Additionally, the compounds of formula (I), (I-a), or (I-b) may be administered transdermally, topically, via implantation, and transdermaly. In certain embodiments, the compounds of the formula (I), (I-a), or (I-b) may be delivered orally. The compounds may also be delivered rectally, bucally, intravaginally, ocularly, andially, or by insufflation. Bromodomain-mediated disorders and conditions may be treated prophylactically, acutely, and chronically using compounds of formula (I), (I-a), or (I-b), depending on the nature of the disorder or condition. Typically, the host or subject in each of these methods is human, although other mammals may also benefit from the administration of a compound of formula (I), (I-a), or (I-b).

A "bromodomain-mediated disorder or condition" is characterized by the participation of one or more bromodomains (e.g., BRD4) in the inception, manifestation of one or more symptoms or disease markers, severity, or progression of a disorder or condition. Accordingly, the invention provides a method for treating cancer, including, but not limited to acoustic neuroma, acute leukemia, acute lymphocytic leukemia, acute myelocytic leukemia (monocytic, myeloblastic, adenocarcinoma, angiosarcoma, astrocytoma, myelomonocytic and promyelocytic), acute t-cell leukemia, basal cell carcinoma, bile duct carcinoma, bladder cancer, brain cancer, breast cancer, bronchogenic carcinoma, cervical cancer, chondrosarcoma, chordoma, choriocarcinoma, chronic leukemia, chronic lymphocytic leukemia, chronic myelocytic (granulocytic) leukemia, chronic myelogenous leukemia, colon cancer, colorectal cancer, craniopharyngioma, cystadenocarcinoma, diffuse large B-cell lymphoma, dysproliferative changes (dysplasias and metaplasias), embryonal carcinoma, endometrial cancer, endotheliosarcoma, ependymoma, epithelial carcinoma, erythroleukemia, esophageal cancer, estrogen-receptor positive breast cancer, essential thrombocythemia, Ewing's tumor, fibrosarcoma, follicular lymphoma, germ cell testicular cancer, glioma, glioblastoma, gliosarcoma, heavy chain disease, hemangioblastoma, hepatoma, hepatocellular cancer, hormone insensitive prostate cancer, leiomyosarcoma, leukemia, liposarcoma, lung cancer, lymphagioendotheliosarcoma, lymphangiosarcoma, lymphoblastic leukemia, lymphoma (Hodgkin's and non-Hodgkin's), malignancies and hyperproliferative disorders of the bladder, breast, colon, lung, ovaries, pancreas, prostate, skin and uterus, lymphoid malignancies of T-cell or B-cell origin, leukemia, lymphoma, medullary carcinoma, medulloblastoma, melanoma, meningioma, mesothelioma, multiple myeloma, myelogenous leukemia, myeloma, myxosarcoma, neuroblastoma, NUT midline carcinoma (NMC), non-small cell lung cancer, oligodendroglioma, oral cancer, osteogenic sarcoma, ovarian cancer, pancreatic cancer, papillary adenocarcinomas, papillary carcinoma, pinealoma, polycythemia vera, prostate cancer, rectal cancer, renal cell carcinoma, retinoblastoma, rhabdomyosarcoma, sarcoma, sebaceous gland carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenstrom's macroglobulinemia, testicular tumors, uterine cancer and Wilms' tumor. The method comprises the step of administering to a subject in need thereof a therapeutically effective amount of a compound of formula (I), (I-a), or (I-b) or a preferred embodiment thereof, with or without a pharmaceutically acceptable carrier.

The invention further provides a method for treating inflammatory diseases, inflammatory conditions, and autoimmune diseases, including, but not limited to: Addison's disease, acute gout, ankylosing spondylitis, asthma, atherosclerosis, Behcet's disease, bullous skin diseases, chronic obstructive pulmonary disease (COPD), Crohn's disease, dermatitis, eczema, giant cell arteritis, glomerulonephritis, hepatitis, hypophysitis, inflammatory bowel disease, Kawasaki disease, lupus nephritis, multiple sclerosis, myocarditis,myositis, nephritis, organ transplant rejection, osteoarthritis, pancreatitis, pericarditis, polyarteritis nodosa, pneumonitis, primary biliary cirrhosis, psoriasis, psoriatic arthritis, rheumatoid arthritis, scleritis, sclerosing cholangitis, sepsis, systemic lupus erythematosus, Takayasu's Arteritis, toxic shock, thyroiditis, type I diabetes, ulcerative colitis, uveitis, vitiligo, vasculitis, and Wegener's granulomatosis. The method comprises the step of administering to a subject in need thereof a therapeutically effective amount of a compound of formula (I), (I-a), or (I-b) or a preferred embodiment thereof, with or without a pharmaceutically acceptable carrier.

The invention further provides a method for treating diabetic nephropathy, hypertensive nephropathy, HIV-associated nephropathy, glomerulonephritis, lupus nephritis, IgA nephropathy, focal segmental glomerulosclerosis, membranous glomerulonephritis, minimal change disease, polycystic kidney disease, or tubular interstitial nephritis. The method comprises the step of administering to a subject in need thereof a therapeutically effective amount of a compound of formula (I), (I-a), or (I-b) or a preferred embodiment thereof, with or without a pharmaceutically acceptable carrier.

The invention further provides a method for treating acute kidney injury or disease or condition, wherein said acute kidney injury or disease or condition is selected from the group consisting of: ischemia-reperfusion induced kidney disease, cardiac and major surgery induced kidney disease, percutaneous coronary intervention induced kidney disease, radio-contrast agent induced kidney disease, sepsis induced kidney disease, pneumonia induced kidney disease, and drug toxicity induced kidney disease. The method comprises the step of administering to a subject in need thereof a therapeutically effective amount of a compound of formula (I), (I-a), or (I-b) or a preferred embodiment thereof, with or without a pharmaceutically acceptable carrier.

The invention further provides a method for treating chronic kidney disease or condition, wherein said disease or condition is selected from the group consisting of: diabetic nephropathy, hypertensive nephropathy, HIV-associated nephropathy, glomerulonephritis, lupus nephritis, IgA nephropathy, focal segmental glomerulosclerosis, membranous glomerulonephritis, minimal change disease, polycystic kidney disease, and tubular interstitial nephritis. The method comprises the step of administering to a subject in need thereof a therapeutically effective amount of a compound of formula (I), (I-a), or (I-b), or a preferred embodiment thereof, with or without a pharmaceutically acceptable carrier.

The invention further provides a method for treating AIDS. The method comprises the step of administering to a subject in need thereof a therapeutically effective amount of a compound of formula (I), (I-a), or (I-b), or a preferred embodiment thereof, with or without a pharmaceutically acceptable carrier.

In another embodiment, the present invention provides compounds of the invention, or pharmaceutical compositions comprising a compound of the invention, for use in medicine. In a particular embodiment, the present invention provides compounds of the invention, or pharmaceutical compositions comprising a compound of the invention, for use in the treatment of diseases or disorders as described herein above.

One embodiment is directed to the use of a compound according to formula (I), (I-a), or (I-b), or a pharmaceutically acceptable salt thereof in the preparation of a medicament. The medicament optionally can comprise at least one additional therapeutic agent. In some embodiments the medicament is for use in the treatment of diseases and disorders as described herein above.

This invention is also directed to the use of a compound according to formula (I), (I-a), or (I-b), or a pharmaceutically acceptable salt thereof in the manufacture of a medicament for the treatment of the diseases and disorders as described herein above. The medicament optionally can comprise at least one additional therapeutic agent.

The compounds of formula (I), (I-a), or (I-b) may be administered as the sole active agent or it may be co-administered with other therapeutic agents, including other compounds that demonstrate the same or a similar therapeutic activity and that are determined to be safe and efficacious for such combined administration. The term "co-administered" means the administration of two or more different therapeutic agents or treatments (e.g., radiation treatment) that are administered to a subject in a single pharmaceutical composition or in separate pharmaceutical compositions. Thus co-administration involves administration at the same time of a single pharmaceutical composition comprising two or more different therapeutic agents or administration of two or more different compositions to the same subject at the same or different times.

The compounds of the invention may be co-administered with a therapeutically effective amount of at least one additional therapeutic agent to treat cancer, where examples of the therapeutic agents include, such as radiation, alkylating agents, angiogenesis inhibitors, antibodies, antimetabolites, antimitotics, antiproliferatives, antivirals, aurora kinase inhibitors, apoptosis promoters (for example, Bcl-xL, Bcl-w and Bfl-1) inhibitors, activators of death receptor pathway, Bcr-Abl kinase inhibitors, BiTE (Bi-Specific T cell Engager) antibodies, antibody drug conjugates, biologic response modifiers, cyclin-dependent kinase inhibitors, cell cycle inhibitors, cyclooxygenase-2 inhibitors, DVDs (dual variable domain antibodies), leukemia viral oncogene homolog (ErbB2) receptor inhibitors, growth factor inhibitors, heat shock protein (HSP)-90 inhibitors, histone deacetylase (HDAC) inhibitors, hormonal therapies, immunologicals, inhibitors of inhibitors of apoptosis proteins (IAPs), intercalating antibiotics, kinase inhibitors, kinesin inhibitors, Jak2 inhibitors, mammalian target of rapamycin inhibitors, microRNA's, mitogen-activated extracellular signal-regulated kinase inhibitors, multivalent binding proteins, non-steroidal anti-inflammatory drugs (NSAIDs), poly ADP (adenosine diphosphate)-ribose polymerase (PARP) inhibitors, platinum chemotherapeutics, polo-like kinase (Plk) inhibitors, phosphoinositide-3 kinase (bromodomain) inhibitors, proteosome inhibitors, purine analogs, pyrimidine analogs, receptor tyrosine kinase inhibitors, etinoids/deltoids plant alkaloids, small inhibitory ribonucleic acids (siRNAs), topoisomerase inhibitors, ubiquitin ligase inhibitors, and the like, and in combination with at least one of these agents .

BiTE antibodies are bi-specific antibodies that direct T-cells to attack cancer cells by simultaneously binding the two cells. The T-cell then attacks the target cancer cell. Examples of BiTE antibodies include adecatumumab (Micromet MT201), blinatumomab (Micromet MT103) and the like. Without being limited by theory, one of the mechanisms by which T-cells elicit apoptosis of the target cancer cell is by exocytosis of cytolytic granule components, which include perforin and granzyme B. In this regard, Bcl-2 has been shown to attenuate the induction of apoptosis by both perforin and granzyme B. These data suggest that inhibition of Bcl-2 could enhance the cytotoxic effects elicited by T-cells when targeted to cancer cells (V.R. Sutton, D.L. Vaux and J.A. Trapani, J. of Immunology 1997, 158 (12), 5783).

SiRNAs are molecules having endogenous RNA bases or chemically modified nucleotides. The modifications do not abolish cellular activity, but rather impart increased stability and/or increased cellular potency. Examples of chemical modifications include phosphorothioate groups, 2'-deoxynucleotide, 2'-OCH₃-containing ribonucleotides, 2'-F-ribonucleotides, 2'-methoxyethyl ribonucleotides, combinations thereof and the like. The siRNA can have varying lengths (e.g., 10-200 bps) and structures (e.g., hairpins, single/double strands, bulges, nicks/gaps, mismatches) and are processed in cells to provide active gene silencing. A double-stranded siRNA (dsRNA) can have the same number of nucleotides on each strand (blunt ends) or asymmetric ends (overhangs). The overhang of 1-2 nucleotides may be present on the sense and/or the antisense strand, as well as present on the 5'- and/ or the 3'-ends of a given strand.

Multivalent binding proteins are binding proteins comprising two or more antigen binding sites. Multivalent binding proteins are engineered to have the three or more antigen binding sites and are generally not naturally occurring antibodies. The term "multispecific binding protein" means a binding protein capable of binding two or more related or unrelated targets. Dual variable domain (DVD) binding proteins are tetravalent or multivalent binding proteins binding proteins comprising two or more antigen binding sites. Such DVDs may be monospecific (i.e., capable of binding one antigen) or multispecific (i.e., capable of binding two or more antigens). DVD binding proteins comprising two heavy chain DVD polypeptides and two light chain DVD polypeptides are referred to as DVD Ig's. Each half of a DVD Ig comprises a heavy chain DVD polypeptide, a light chain DVD polypeptide, and two antigen binding sites. Each binding site comprises a heavy chain variable domain and a light chain variable domain with a total of 6 CDRs involved in antigen binding per antigen binding site. Multispecific DVDs include DVD binding proteins that bind DLL4 and VEGF, or C-met and EFGR or ErbB3 and EGFR.

Alkylating agents include altretamine, AMD-473, AP-5280, apaziquone, bendamustine, brostallicin, busulfan, carboquone, carmustine (BCNU), chlorambucil, CLORETAZINE® (laromustine, VNP 40101M), cyclophosphamide, decarbazine, estramustine, fotemustine, glufosfamide, ifosfamide, KW-2170, lomustine (CCNU), mafosfamide, melphalan, mitobronitol, mitolactol, nimustine, nitrogen mustard N-oxide, ranimustine, temozolomide, thiotepa, TREANDA® (bendamustine), treosulfan, rofosfamide and the like.

Angiogenesis inhibitors include endothelial-specific receptor tyrosine kinase (Tie-2) inhibitors, epidermal growth factor receptor (EGFR) inhibitors, insulin growth factor-2 receptor (IGFR-2) inhibitors, matrix metalloproteinase-2 (MMP-2) inhibitors, matrix metalloproteinase-9 (MMP-9) inhibitors, platelet-derived growth factor receptor (PDGFR) inhibitors, thrombospondin analogs, vascular endothelial growth factor receptor tyrosine kinase (VEGFR) inhibitors and the like.

Antimetabolites include ALIMTA® (pemetrexed disodium, LY231514, MTA), 5-azacitidine, XELODA® (capecitabine), carmofur, LEUSTAT® (cladribine), clofarabine, cytarabine, cytarabine ocfosfate, cytosine arabinoside, decitabine, deferoxamine, doxifluridine, eflornithine, EICAR (5-ethynyl-1-β-D-ribofuranosylimidazole-4-carboxamide), enocitabine, ethnylcytidine, fludarabine, 5-fluorouracil alone or in combination with leucovorin, GEMZAR® (gemcitabine), hydroxyurea, ALKERAN®(melphalan), mercaptopurine, 6-mercaptopurine riboside, methotrexate, mycophenolic acid, nelarabine, nolatrexed, ocfosfate, pelitrexol, pentostatin, raltitrexed, Ribavirin, triapine, trimetrexate, S-1, tiazofurin, tegafur, TS-1, vidarabine, UFT and the like.

Antivirals include ritonavir, hydroxychloroquine and the like.

Aurora kinase inhibitors include ABT-348, AZD-1152, MLN-8054, VX-680, Aurora A-specific kinase inhibitors, Aurora B-specific kinase inhibitors and pan-Aurora kinase inhibitors and the like.

Bcl-2 protein inhibitors include AT-101 ((-)gossypol), GENASENSE® (G3139 or oblimersen (Bcl-2-targeting antisense oligonucleotide)), IPI-194, IPI-565, N-(4-(4-((4'-chloro(1,1'-biphenyl)-2-yl)methyl)piperazin-1-yl)benzoyl)-4-(((1R)-3-(dimethylamino)-1-((phenylsulfanyl)methyl)propyl)amino)-3-nitrobenzenesulfonamide) (ABT-737), N-(4-(4-((2-(4-chlorophenyl)-5,5-dimethyl-1-cyclohex-1-en-1-yl)methyl)piperazin-1-yl)benzoyl)-4-(((1R)-3-(morpholin-4-yl)-1-((phenylsulfanyl)methyl)propyl)amino)-3-((trifluoromethyl)sulfonyl)benzenesulfonamide (ABT-263), GX-070 (obatoclax) and the like.

Bcr-Abl kinase inhibitors include DASATINIB® (BMS-3 54825), GLEEVEC® (imatinib) and the like.

CDK inhibitors include AZD-5438, BMI-1040, BMS-032, BMS-387, CVT-2584, flavopyridol, GPC-286199, MCS-5A, PD0332991, PHA-690509, seliciclib (CYC-202, R-roscovitine), ZK-304709 and the like.

COX-2 inhibitors include ABT-963, ARCOXIA® (etoricoxib), BEXTRA® (valdecoxib), BMS347070, CELEBREX® (celecoxib), COX-189 (lumiracoxib), CT-3, DERAMAXX® (deracoxib), JTE-522, 4-methyl-2-(3,4-dimethylphenyl)-1-(4-sulfamoylphenyl-1*H*-pyrrole), MK-663 (etoricoxib), NS-398, parecoxib, RS-57067, SC-58125, SD-8381, SVT-2016, S-2474, T-614, VIOXX® (rofecoxib) and the like.

EGFR inhibitors include EGFR antibodies, ABX-EGF, anti-EGFR immunoliposomes, EGF-vaccine, EMD-7200, ERBITUX® (cetuximab), HR3, IgA antibodies, IRESSA® (gefitinib), TARCEVA® (erlotinib or OSI-774), TP-38, EGFR fusion protein, TYKERB® (lapatinib) and the like.

ErbB2 receptor inhibitors include CP-724-714, CI-1033 (canertinib), HERCEPTIN® (trastuzumab), TYKERB® (lapatinib), OMNITARG® (2C4, petuzumab), TAK-165, GW-572016 (ionafarnib), GW-282974, EKB-569, PI-166, dHER2 (HER2 vaccine), APC-8024 (HER-2 vaccine), anti-HER/2neu bispecific antibody, B7.her2IgG3, AS HER2 trifunctional bispecfic antibodies, mAB AR-209, mAB 2B-1 and the like.

Histone deacetylase inhibitors include depsipeptide, LAQ-824, MS-275, trapoxin, suberoylanilide hydroxamic acid (SAHA), TSA, valproic acid and the like.

HSP-90 inhibitors include 17-AAG-nab, 17-AAG, CNF-101, CNF-1010, CNF-2024, 17-DMAG, geldanamycin, IPI-504, KOS-953, MYCOGRAB® (human recombinant antibody to HSP-90), NCS-683664, PU24FCl, PU-3, radicicol, SNX-2112, STA-9090 VER49009 and the like.

Inhibitors of inhibitors of apoptosis proteins include HGS1029, GDC-0145, GDC-0152, LCL-161, LBW-242 and the like.

Antibody drug conjugates include anti-CD22-MC-MMAF, anti-CD22-MC-MMAE, anti-CD22-MCC-DM1, CR-011-vcMMAE, PSMA-ADC, MEDI-547, SGN-19Am SGN-35, SGN-75 and the like

Activators of death receptor pathway include TRAIL, antibodies or other agents that target TRAIL or death receptors (e.g., DR4 and DR5) such as Apomab, conatumumab, ETR2-ST01, GDC0145, (lexatumumab), HGS-1029, LBY-135, PRO-1762 and trastuzumab.

Kinesin inhibitors include Eg5 inhibitors such as AZD4877, ARRY-520; CENPE inhibitors such as GSK923295A and the like.

JAK-2 inhibitors include CEP-701 (lesaurtinib), XL019 and INCB018424 and the like.

MEK inhibitors include ARRY-142886, ARRY-438162 PD-325901, PD-98059 and the like.

mTOR inhibitors include AP-23573, CCI-779, everolimus, RAD-001, rapamycin, temsirolimus, ATP-competitive TORC1/TORC2 inhibitors, including PI-103, PP242, PP30, Torin 1 and the like.

Non-steroidal anti-inflammatory drugs include AMIGESIC® (salsalate), DOLOBID® (diflunisal), MOTRIN® (ibuprofen), ORUDIS® (ketoprofen), RELAFEN® (nabumetone), FELDENE® (piroxicam), ibuprofen cream, ALEVE® (naproxen) and NAPROSYN® (naproxen), VOLTAREN® (diclofenac), INDOCIN® (indomethacin), CLINORIL® (sulindac), TOLECTIN® (tolmetin), LODINE® (etodolac), TORADOL® (ketorolac), DAYPRO® (oxaprozin) and the like.

PDGFR inhibitors include C-451, CP-673, CP-868596 and the like.

Platinum chemotherapeutics include cisplatin, ELOXATIN® (oxaliplatin) eptaplatin, lobaplatin, nedaplatin, PARAPLATIN® (carboplatin), satraplatin, picoplatin and the like.

Polo-like kinase inhibitors include BI-2536 and the like.

Phosphoinositide-3 kinase (PI3K) inhibitors include wortmannin, LY294002, XL-147, CAL-120, ONC-21, AEZS-127, ETP-45658, PX-866, GDC-0941, BGT226, BEZ235, XL765 and the like.

Thrombospondin analogs include ABT-510, ABT-567, ABT-898, TSP-1 and the like.

VEGFR inhibitors include AVASTIN® (bevacizumab), ABT-869, AEE-788, ANGIOZYME™ (a ribozyme that inhibits angiogenesis (Ribozyme Pharmaceuticals (Boulder, CO.) and Chiron, (Emeryville, CA)), axitinib (AG-13736), AZD-2171, CP-547,632, IM-862, MACUGEN (pegaptamib), NEXAVAR® (sorafenib, BAY43-9006), pazopanib (GW-786034), vatalanib (PTK-787, ZK-222584), SUTENT® (sunitinib, SU-11248), VEGF trap, ZACTIMA™ (vandetanib, ZD-6474), GA101, ofatumumab, ABT-806 (mAb-806), ErbB3 specific antibodies, BSG2 specific antibodies, DLL4 specific antibodies and C-met specific antibodies, and the like.

Antibiotics include intercalating antibiotics aclarubicin, actinomycin D, amrubicin, annamycin, adriamycin, BLENOXANE® (bleomycin), daunorubicin, CAELYX® or MYOCET® (liposomal doxorubicin), elsamitrucin, epirbucin, glarbuicin, ZAVEDOS® (idarubicin), mitomycin C, nemorubicin, neocarzinostatin, peplomycin, pirarubicin, rebeccamycin, stimalamer, streptozocin, VALSTAR® (valrubicin), zinostatin and the like.

Topoisomerase inhibitors include aclarubicin, 9-aminocamptothecin, amonafide, amsacrine, becatecarin, belotecan, BN-80915, CAMPTOSAR® (irinotecan hydrochloride), camptothecin, CARDIOXANE® (dexrazoxine), diflomotecan, edotecarin, ELLENCE® or PHARMORUBICIN® (epirubicin), etoposide, exatecan, 10-hydroxycamptothecin, gimatecan, lurtotecan, mitoxantrone, orathecin, pirarbucin, pixantrone, rubitecan, sobuzoxane, SN-38, tafluposide, topotecan and the like.

Antibodies include AVASTIN® (bevacizumab), CD40-specific antibodies, chTNT-1/B, denosumab, ERBITUX® (cetuximab), HUMAX-CD4® (zanolimumab), IGF1R-specific antibodies, lintuzumab, PANOREX® (edrecolomab), RENCAREX® (WX G250), RITUXAN® (rituximab), ticilimumab, trastuzimab, CD20 antibodies types I and II and the like.

Hormonal therapies include ARIMIDEX® (anastrozole), AROMASIN® (exemestane), arzoxifene, CASODEX® (bicalutamide), CETROTIDE® (cetrorelix), degarelix, deslorelin, DESOPAN® (trilostane), dexamethasone, DROGENIL® (flutamide), EVISTA® (raloxifene), AFEMA™ (fadrozole), FARESTON® (toremifene), FASLODEX® (fulvestrant), FEMARA® (letrozole), formestane, glucocorticoids, HECTOROL® (doxercalciferol), RENAGEL® (sevelamer carbonate), lasofoxifene, leuprolide acetate, MEGACE® (megesterol), MIFEPREX® (mifepristone), NILANDRON™ (nilutamide), NOLVADEX® (tamoxifen citrate), PLENAXIS™ (abarelix), prednisone, PROPECIA® (finasteride), rilostane, SUPREFACT® (buserelin), TRELSTAR® (luteinizing hormone releasing hormone (LHRH)), VANTAS® (Histrelin implant), VETORYL® (trilostane or modrastane), ZOLADEX® (fosrelin, goserelin) and the like.

Deltoids and retinoids include seocalcitol (EB1089, CB1093), lexacalcitrol (KH1060), fenretinide, PANRETIN® (aliretinoin), ATRAGEN® (liposomal tretinoin), TARGRETIN® (bexarotene), LGD-1550 and the like.

PARP inhibitors include ABT-888 (veliparib), olaparib, KU-59436, AZD-2281, AG-014699, BSI-201, BGP-15, INO-1001, ONO-2231 and the like.

Plant alkaloids include, but are not limited to, vincristine, vinblastine, vindesine, vinorelbine and the like.

Proteasome inhibitors include VELCADE® (bortezomib), MG132, NPI-0052, PR-171 and the like.

Examples of immunologicals include interferons and other immune-enhancing agents. Interferons include interferon alpha, interferon alpha-2a, interferon alpha-2b, interferon beta, interferon gamma-la, ACTIMMUNE® (interferon gamma-lb) or interferon gamma-nl, combinations thereof and the like. Other agents include ALFAFERONE®,(IFN-α), BAM-002 (oxidized glutathione), BEROMUN® (tasonermin), BEXXAR® (tositumomab), CAMPATH® (alemtuzumab), CTLA4 (cytotoxic lymphocyte antigen 4), decarbazine, denileukin, epratuzumab, GRANOCYTE® (lenograstim), lentinan, leukocyte alpha interferon, imiquimod, MDX-010 (anti-CTLA-4), melanoma vaccine, mitumomab, molgramostim, MYLOTARG™ (gemtuzumab ozogamicin), NEUPOGEN® (filgrastim), OncoVAC-CL, OVAREX® (oregovomab), pemtumomab (Y-muHMFG1), PROVENGE® (sipuleucel-T), sargaramostim, sizofilan, teceleukin, THERACYS® (Bacillus Calmette-Guerin), ubenimex, VIRULIZIN® (immunotherapeutic, Lorus Pharmaceuticals), Z-100 (Specific Substance of Maruyama (SSM)), WF-10 (Tetrachlorodecaoxide (TCDO)), PROLEUKIN® (aldesleukin), ZADAXIN® (thymalfasin), ZENAPAX® (daclizumab), ZEVALIN® (90Y-Ibritumomab tiuxetan) and the like.

Biological response modifiers are agents that modify defense mechanisms of living organisms or biological responses, such as survival, growth or differentiation of tissue cells to direct them to have anti-tumor activity and include krestin, lentinan, sizofiran, picibanil PF-3512676 (CpG-8954), ubenimex and the like.

Pyrimidine analogs include cytarabine (ara C or Arabinoside C), cytosine arabinoside, doxifluridine, FLUDARA® (fludarabine), 5-FU (5-fluorouracil), floxuridine, GEMZAR® (gemcitabine), TOMUDEX® (ratitrexed), TROXATYL™ (triacetyluridine troxacitabine) and the like.

Purine analogs include LANVIS® (thioguanine) and PURI-NETHOL® (mercaptopurine).

Antimitotic agents include batabulin, epothilone D (KOS-862), N-(2-((4-hydroxyphenyl)amino)pyridin-3-yl)-4-methoxybenzenesulfonamide, ixabepilone (BMS 247550), paclitaxel, TAXOTERE® (docetaxel), PNU100940 (109881), patupilone, XRP-9881 (larotaxel), vinflunine, ZK-EPO (synthetic epothilone) and the like.

Ubiquitin ligase inhibitors include MDM2 inhibitors, such as nutlins, NEDD8 inhibitors such as MLN4924 and the like.

Compounds of this invention may also be used as radiosensitizers that enhance the efficacy of radiotherapy. Examples of radiotherapy include external beam radiotherapy, teletherapy, brachytherapy and sealed, unsealed source radiotherapy and the like.

Additionally, compounds of formula (I), (I-a), or (I-b) may be combined with other chemotherapeutic agents such as ABRAXANE™ (ABI-007), ABT-100 (farnesyl transferase inhibitor), ADVEXIN® (Ad5CMV-p53 vaccine), ALTOCOR® or MEVACOR® (lovastatin), AMPLIGEN® (poly I:poly C12U, a synthetic RNA), APTOSYN® (exisulind), AREDIA® (pamidronic acid), arglabin, L-asparaginase, atamestane (1-methyl-3,17-dione-androsta-1,4-diene), AVAGE® (tazarotene), AVE-8062 (combreastatin derivative) BEC2 (mitumomab), cachectin or cachexin (tumor necrosis factor), canvaxin (vaccine), CEAVAC® (cancer vaccine), CELEUK® (celmoleukin), CEPLENE® (histamine dihydrochloride), CERVARIX® (human papillomavirus vaccine), CHOP® (C: CYTOXAN® (cyclophosphamide); H: ADRIAMYCIN® (hydroxydoxorubicin); O: Vincristine (ONCOVIN®); P: prednisone), CYPAT™ (cyproterone acetate), combrestatin A4P, DAB(389)EGF (catalytic and translocation domains of diphtheria toxin fused via a His-Ala linker to human epidermal growth factor) or TransMID-107R™ (diphtheria toxins), dacarbazine, dactinomycin, 5,6-dimethylxanthenone-4-acetic acid (DMXAA), eniluracil, EVIZON™ (squalamine lactate), DIMERICINE® (T4N5 liposome lotion), discodermolide, DX-8951f (exatecan mesylate), enzastaurin, EPO906 (epithilone B), GARDASIL® (quadrivalent human papillomavirus (Types 6, 11, 16, 18) recombinant vaccine), GASTRIMMUNE®, GENASENSE®, GMK (ganglioside conjugate vaccine), GVAX® (prostate cancer vaccine), halofuginone, histerelin, hydroxycarbamide, ibandronic acid, IGN-101, IL-13-PE38, IL-13-PE38QQR (cintredekin besudotox), IL-13-pseudomonas exotoxin, interferon-a, interferon-y, JUNOVAN™ or MEPACT™ (mifamurtide), lonafarnib, 5,10-methylenetetrahydrofolate, miltefosine (hexadecylphosphocholine), NEOVASTAT®(AE-941), NEUTREXIN® (trimetrexate glucuronate), NIPENT® (pentostatin), ONCONASE® (a ribonuclease enzyme), ONCOPHAGE® (melanoma vaccine treatment), ONCOVAX® (IL-2 Vaccine), ORATHECIN™ (rubitecan), OSIDEM® (antibody-based cell drug), OVAREX® MAb (murine monoclonal antibody), paclitaxel, PANDIMEX™ (aglycone saponins from ginseng comprising 20(S)protopanaxadiol (aPPD) and 20(S)protopanaxatriol (aPPT)), panitumumab, PANVAC®-VF (investigational cancer vaccine), pegaspargase, PEG Interferon A, phenoxodiol, procarbazine, rebimastat, REMOVAB® (catumaxomab), REVLIMID® (lenalidomide), RSR13 (efaproxiral), SOMATULINE® LA (lanreotide), SORIATANE® (acitretin), staurosporine (Streptomyces staurospores), talabostat (PT100), TARGRETIN® (bexarotene), TAXOPREXIN® (DHA-paclitaxel), TELCYTA® (canfosfamide, TLK286), temilifene, TEMODAR® (temozolomide), tesmilifene, thalidomide, THERATOPE® (STn-KLH), thymitaq (2-amino-3,4-dihydro-6-methyl-4-oxo-5-(4-pyridylthio)quinazoline dihydrochloride), TNFERADE™ (adenovector: DNA carrier containing the gene for tumor necrosis factor-a), TRACLEER® or ZAVESCA® (bosentan), tretinoin (Retin-A), tetrandrine, TRISENOX® (arsenic trioxide), VIRULIZIN®, ukrain (derivative of alkaloids from the greater celandine plant), vitaxin (anti-alphavbeta3 antibody), XCYTRIN® (motexafin gadolinium), XINLAY™ (atrasentan), XYOTAX™ (paclitaxel poliglumex), YONDELIS® (trabectedin), ZD-6126, ZINECARD® (dexrazoxane), ZOMETA® (zolendronic acid), zorubicin and the like.

The compounds of the invention may also be co-administered with a therapeutically effective amount of at least one additional therapeutic agents to treat an inflammatory disease or condition, or autoimmune disease, where examples of the agents include, such as methotrexate, 6-mercaptopurine, azathioprine sulphasalazine, mesalazine, olsalazine chloroquinine/ hydroxychloroquine, pencillamine, aurothiomalate (intramuscular and oral), azathioprine, cochicine, corticosteroids (oral, inhaled and local injection), beta-2 adrenoreceptor agonists (salbutamol, terbutaline, salmeteral), xanthines (theophylline, aminophylline), cromoglycate, nedocromil, ketotifen, ipratropium and oxitropium, cyclosporin, FK506, rapamycin, mycophenolate mofetil, leflunomide, NSAIDs, for example, ibuprofen, corticosteroids such as prednisolone, phosphodiesterase inhibitors, adensosine agonists, antithrombotic agents, complement inhibitors, adrenergic agents, agents which interfere with signalling by proinflammatory cytokines such as TNFα or IL-1 (e.g., NIK, IKK, p38 or MAP kinase inhibitors), IL-1β converting enzyme inhibitors, T-cell signalling inhibitors such as kinase inhibitors, metalloproteinase inhibitors, sulfasalazine, 6-mercaptopurines, angiotensin converting enzyme inhibitors, soluble cytokine receptors and derivatives thereof (e.g. soluble p55 or p75 TNF receptors and the derivatives p75TNFRIgG (etanercept) and p55TNFRIgG (Lenercept), sIL-1RI, sIL-1RII, sIL-6R), antiinflammatory cytokines (e.g. IL-4, IL-10, IL-11, IL-13 and TGFβ), celecoxib, folic acid, hydroxychloroquine sulfate, rofecoxib, etanercept, infliximab, naproxen, valdecoxib, sulfasalazine, methylprednisolone, meloxicam, methylprednisolone acetate, gold sodium thiomalate, aspirin, triamcinolone acetonide, propoxyphene napsylate/apap, folate, nabumetone, diclofenac, piroxicam, etodolac, diclofenac sodium, oxaprozin, oxycodone HCl, hydrocodone bitartrate/apap, diclofenac sodium/misoprostol, fentanyl, anakinra, tramadol HCl, salsalate, sulindac, cyanocobalamin/fa/pyridoxine, acetaminophen, alendronate sodium, prednisolone, morphine sulfate, lidocaine hydrochloride, indomethacin, glucosamine sulf/chondroitin, amitriptyline HCl, sulfadiazine, oxycodone HCl/acetaminophen, olopatadine HCl misoprostol, naproxen sodium, omeprazole, cyclophosphamide, rituximab, IL-1 TRAP, MRA, CTLA4-IG, IL-18 BP, anti-IL-12, Anti-IL15, BIRB-796, SCIO-469, VX-702, AMG-548, VX-740, Roflumilast, IC-485, CDC-801, S1P1 agonists (such as FTY720), PKC family inhibitors (such as Ruboxistaurin or AEB-071) and Mesopram. In certain embodiments, combinations include methotrexate or leflunomide and in moderate or severe rheumatoid arthritis cases, cyclosporine and anti-TNF antibodies as noted above.

Non-limiting examples of therapeutic agents for inflammatory bowel disease with which a compound of formula (I), (I-a), or (I-b) may be co-administered include the following: budenoside; epidermal growth factor; corticosteroids; cyclosporin, sulfasalazine; aminosalicylates; 6-mercaptopurine; azathioprine; metronidazole; lipoxygenase inhibitors; mesalamine; olsalazine; balsalazide; antioxidants; thromboxane inhibitors; IL-1 receptor antagonists; anti-IL-1β monoclonal antibodies; anti-IL-6 monoclonal antibodies; growth factors; elastase inhibitors; pyridinyl-imidazole compounds; antibodies to or antagonists of other human cytokines or growth factors, for example, TNF, LT, IL-1, IL-2, IL-6, IL-7, IL-8, IL-12, IL-15, IL-16, IL-23, EMAP-II, GM-CSF, FGF, and PDGF; cell surface molecules such as CD2, CD3, CD4, CD8, CD25, CD28, CD30, CD40, CD45, CD69, CD90 or their ligands; methotrexate; cyclosporine; FK506; rapamycin; mycophenolate mofetil; leflunomide; NSAIDs, for example, ibuprofen; corticosteroids such as prednisolone; phosphodiesterase inhibitors; adenosine agonists; antithrombotic agents; complement inhibitors; adrenergic agents; agents which interfere with signalling by proinflammatory cytokines such as TNFα or IL-1 (e.g. NIK, IKK, or MAP kinase inhibitors); IL-1β converting enzyme inhibitors; TNFα converting enzyme inhibitors; T-cell signalling inhibitors such as kinase inhibitors; metalloproteinase inhibitors; sulfasalazine; azathioprine; 6-mercaptopurines; angiotensin converting enzyme inhibitors; soluble cytokine receptors and derivatives thereof (e.g. soluble p55 or p75 TNF receptors, sIL-1RI, sIL-1RII, sIL-6R) and antiinflammatory cytokines (e.g. IL-4, IL-10, IL-11, IL-13 and TGFβ). Preferred examples of therapeutic agents for Crohn's disease with which a compound of formula (I), (I-a), or (I-b) may be combined include the following: TNF antagonists, for example, anti-TNF antibodies, D2E7 (adalimumab), CA2 (infliximab), CDP 571, TNFR-Ig constructs, (p75TNFRIgG (etanercept) and p55TNFRIgG (LENERCEPT™) inhibitors and PDE4 inhibitors. A compound of formula (I), (I-a), or (I-b) may be combined with corticosteroids, for example, budenoside and dexamethasone; sulfasalazine, 5-aminosalicylic acid; olsalazine; and agents which interfere with synthesis or action of proinflammatory cytokines such as IL-1, for example, IL-1β converting enzyme inhibitors and IL-1ra; T cell signaling inhibitors, for example, tyrosine kinase inhibitors; 6-mercaptopurine; IL-11; mesalamine; prednisone; azathioprine; mercaptopurine; infliximab; methylprednisolone sodium succinate; diphenoxylate/atrop sulfate; loperamide hydrochloride; methotrexate; omeprazole; folate; ciprofloxacin/dextrose-water; hydrocodone bitartrate/apap; tetracycline hydrochloride; fluocinonide; metronidazole; thimerosal/boric acid; cholestyramine/sucrose; ciprofloxacin hydrochloride; hyoscyamine sulfate; meperidine hydrochloride; midazolam hydrochloride; oxycodone HCl/acetaminophen; promethazine hydrochloride; sodium phosphate; sulfamethoxazole/trimethoprim; celecoxib; polycarbophil; propoxyphene napsylate; hydrocortisone; multivitamins; balsalazide disodium; codeine phosphate/apap; colesevelam HCl; cyanocobalamin; folic acid; levofloxacin; methylprednisolone; natalizumab and interferon-gamma.

Non-limiting examples of therapeutic agents for multiple sclerosis with which a compound of formula (I), (I-a), or (I-b) may be co-administered include the following: corticosteroids; prednisolone; methylprednisolone; azathioprine; cyclophosphamide; cyclosporine; methotrexate; 4-aminopyridine; tizanidine; interferon-β1a (AVONEX®; Biogen); interferon-β1b (BETASERON®; Chiron/Berlex); interferon α-n3) (Interferon Sciences/Fujimoto), interferon-α (Alfa Wassermann/J&J), interferon β1A-IF (Serono/Inhale Therapeutics), Peginterferon α 2b (Enzon/Schering-Plough), Copolymer 1 (Cop-1; COPAXONE®; Teva Pharmaceutical Industries, Inc.); hyperbaric oxygen; intravenous immunoglobulin; cladribine; antibodies to or antagonists of other human cytokines or growth factors and their receptors, for example, TNF, LT, IL-1, IL-2, IL-6, IL-7, IL-8, IL-12, IL-23, IL-15, IL-16, EMAP-II, GM-CSF, FGF, and PDGF. A compound of formula (I), (I-a), or (I-b) may be combined with antibodies to cell surface molecules such as CD2, CD3, CD4, CD8, CD19, CD20, CD25, CD28, CD30, CD40, CD45, CD69, CD80, CD86, CD90 or their ligands. A compound of formula (I), (I-a), or (I-b) may also be combined with agents such as methotrexate, cyclosporine, FK506, rapamycin, mycophenolate mofetil, leflunomide, an S1P1 agonist, NSAIDs, for example, ibuprofen, corticosteroids such as prednisolone, phosphodiesterase inhibitors, adensosine agonists, antithrombotic agents, complement inhibitors, adrenergic agents, agents which interfere with signalling by proinflammatory cytokines such as TNFα or IL-1 (e.g., NIK, IKK, p38 or MAP kinase inhibitors), IL-1β converting enzyme inhibitors, TACE inhibitors, T-cell signaling inhibitors such as kinase inhibitors, metalloproteinase inhibitors, sulfasalazine, azathioprine, 6-mercaptopurines, angiotensin converting enzyme inhibitors, soluble cytokine receptors and derivatives thereof (e.g. soluble p55 or p75 TNF receptors, sIL-1RI, sIL-1RII, sIL-6R) and antiinflammatory cytokines (e.g. IL-4, IL-10, IL-13 and TGFβ).

A compound of formula (I), (I-a), or (I-b) may also be co-administered with agents, such as alemtuzumab, dronabinol, daclizumab, mitoxantrone, xaliproden hydrochloride, fampridine, glatiramer acetate, natalizumab, sinnabidol, α-immunokine NNSO3, ABR-215062, AnergiX.MS, chemokine receptor antagonists, BBR-2778, calagualine, CPI-1189, LEM (liposome encapsulated mitoxantrone), THC.CBD (cannabinoid agonist), MBP-8298, mesopram (PDE4 inhibitor), MNA-715, anti-IL-6 receptor antibody, neurovax, pirfenidone allotrap 1258 (RDP-1258), sTNF-R1, talampanel, teriflunomide, TGF-beta2, tiplimotide, VLA-4 antagonists (for example, TR-14035, VLA4 Ultrahaler, Antegran-ELAN/Biogen), interferon gamma antagonists and IL-4 agonists.

Non-limiting examples of therapeutic agents for ankylosing spondylitis with which a compound of formula (I), (I-a), or (I-b) may be co-administered include the following: ibuprofen, diclofenac, misoprostol, naproxen, meloxicam, indomethacin, diclofenac, celecoxib, rofecoxib, sulfasalazine, methotrexate, azathioprine, minocyclin, prednisone, and anti-TNF antibodies, D2E7 (HUMIRA®), CA2 (infliximab), CDP 571, TNFR-Ig constructs, (p75TNFRIgG (ENBREL®) and p55TNFRIgG (LENERCEPT®).

Non-limiting examples of therapeutic agents for asthma with which a compound of formula (I), (I-a), or (I-b) may be co-administered include the following: albuterol, salmeterol/fluticasone, montelukast sodium, fluticasone propionate, budesonide, prednisone, salmeterol xinafoate, levalbuterol HCl, albuterol sulfate/ipratropium, prednisolone sodium phosphate, triamcinolone acetonide, beclomethasone dipropionate, ipratropium bromide, azithromycin, pirbuterol acetate, prednisolone, theophylline anhydrous, methylprednisolone sodium succinate, clarithromycin, zafirlukast, formoterol fumarate, influenza virus vaccine, amoxicillin trihydrate, flunisolide, allergy injection, cromolyn sodium, fexofenadine hydrochloride, flunisolide/menthol, amoxicillin/clavulanate, levofloxacin, inhaler assist device, guaifenesin, dexamethasone sodium phosphate, moxifloxacin HCl, doxycycline hyclate, guaifenesin/d-methorphan, p-ephedrine/cod/chlorphenir, gatifloxacin, cetirizine hydrochloride, mometasone furoate, salmeterol xinafoate, benzonatate, cephalexin, pe/hydrocodone/chlorphenir, cetirizine HCl/pseudoephed, phenylephrine/cod/promethazine, codeine/promethazine, cefprozil, dexamethasone, guaifenesin/pseudoephedrine, chlorpheniramine/hydrocodone, nedocromil sodium, terbutaline sulfate, epinephrine, methylprednisolone, anti-IL-13 antibody, and metaproterenol sulfate.

Non-limiting examples of therapeutic agents for COPD with which a compound of formula (I), (I-a), or (I-b) may be co-administered include the following: albuterol sulfate/ipratropium, ipratropium bromide, salmeterol/fluticasone, albuterol, salmeterol xinafoate, fluticasone propionate, prednisone, theophylline anhydrous, methylprednisolone sodium succinate, montelukast sodium, budesonide, formoterol fumarate, triamcinolone acetonide, levofloxacin, guaifenesin, azithromycin, beclomethasone dipropionate, levalbuterol HCl, flunisolide, ceftriaxone sodium, amoxicillin trihydrate, gatifloxacin, zafirlukast, amoxicillin/clavulanate, flunisolide/menthol, chlorpheniramine/hydrocodone, metaproterenol sulfate, methylprednisolone, mometasone furoate, p-ephedrine/cod/chlorphenir, pirbuterol acetate, p-ephedrine/loratadine, terbutaline sulfate, tiotropium bromide, (R,R)-formoterol, TgAAT, cilomilast and roflumilast.

Non-limiting examples of therapeutic agents for psoriasis with which a compound of formula (I), (I-a), or (I-b) may be co-administered include the following: calcipotriene, clobetasol propionate, triamcinolone acetonide, halobetasol propionate, tazarotene, methotrexate, fluocinonide, betamethasone diprop augmented, fluocinolone acetonide, acitretin, tar shampoo, betamethasone valerate, mometasone furoate, ketoconazole, pramoxine/fluocinolone, hydrocortisone valerate, flurandrenolide, urea, betamethasone, clobetasol propionate/emoll, fluticasone propionate, azithromycin, hydrocortisone, moisturizing formula, folic acid, desonide, pimecrolimus, coal tar, diflorasone diacetate, etanercept folate, lactic acid, methoxsalen, hc/bismuth subgal/znox/resor, methylprednisolone acetate, prednisone, sunscreen, halcinonide, salicylic acid, anthralin, clocortolone pivalate, coal extract, coal tar/salicylic acid, coal tar/salicylic acid/sulfur, desoximetasone, diazepam, emollient, fluocinonide/emollient, mineral oil/castor oil/na lact, mineral oil/peanut oil, petroleum/isopropyl myristate, psoralen, salicylic acid, soap/tribromsalan, thimerosal/boric acid, celecoxib, infliximab, cyclosporine, alefacept, efalizumab, tacrolimus, pimecrolimus, PUVA, UVB, sulfasalazine, ABT-874 and ustekinamab.

Non-limiting examples of therapeutic agents for psoriatic arthritis with which a compound of formula (I), (I-a), or (I-b) may be co-administered include the following: methotrexate, etanercept, rofecoxib, celecoxib, folic acid, sulfasalazine, naproxen, leflunomide, methylprednisolone acetate, indomethacin, hydroxychloroquine sulfate, prednisone, sulindac, betamethasone diprop augmented, infliximab, methotrexate, folate, triamcinolone acetonide, diclofenac, dimethylsulfoxide, piroxicam, diclofenac sodium, ketoprofen, meloxicam, methylprednisolone, nabumetone, tolmetin sodium, calcipotriene, cyclosporine, diclofenac sodium/misoprostol, fluocinonide, glucosamine sulfate, gold sodium thiomalate, hydrocodone bitartrate/apap, ibuprofen, risedronate sodium, sulfadiazine, thioguanine, valdecoxib, alefacept, D2E7 (adalimumab), and efalizumab.

Examples of therapeutic agents for SLE (Lupus) with which a compound of formula (I), (I-a), or (I-b) may be co-administered include the following: NSAIDS, for example, diclofenac, naproxen, ibuprofen, piroxicam, indomethacin; COX2 inhibitors, for example, celecoxib, rofecoxib, valdecoxib; anti-malarials, for example, hydroxychloroquine; steroids, for example, prednisone, prednisolone, budenoside, dexamethasone; cytotoxics, for example, azathioprine, cyclophosphamide, mycophenolate mofetil, methotrexate; inhibitors of PDE4 or purine synthesis inhibitor, for example Cellcept®. A compound of formula (I), (I-a), or (I-b) may also be combined with agents such as sulfasalazine, 5-aminosalicylic acid, olsalazine, Imuran® and agents which interfere with synthesis, production or action of proinflammatory cytokines such as IL-1, for example, caspase inhibitors like IL-1β converting enzyme inhibitors and IL-1ra. A compound of formula (I), (I-a), or (I-b) may also be used with T cell signaling inhibitors, for example, tyrosine kinase inhibitors; or molecules that target T cell activation molecules, for example, CTLA-4-IgG or anti-B7 family antibodies, anti-PD-1 family antibodies. A compound of formula (I), (I-a), or (I-b) may be combined with IL-11 or anti-cytokine antibodies, for example, fonotolizumab (anti-IFNg antibody), or anti-receptor receptor antibodies, for example, anti-IL-6 receptor antibody and antibodies to B-cell surface molecules. A compound of formula (I), (I-a), or (I-b) may also be used with LJP 394 (abetimus), agents that deplete or inactivate B-cells, for example, Rituximab (anti-CD20 antibody), lymphostat-B (anti-BlyS antibody), TNF antagonists, for example, anti-TNF antibodies, D2E7 (adalimumab), CA2 (infliximab), CDP 571, TNFR-Ig constructs, (p75TNFRIgG (etanercept) and p55TNFRIgG (LENERCEPT™).

The compounds of the invention can also be co-administered with a therapeutically effective amount of at least one additional therapeutic agents used in the prevention or treatment of AIDS, where examples of the agents include, HIV reverse transcriptase inhibitors, HIV protease inhibitors, immunomodulators, and other retroviral drugs. Examples of reverse transcriptase inhibitors include, but are not limited to, abacavir, adefovir, didanosine, dipivoxil delavirdine, efavirenz, lamivudine, nevirapine, stavudine zalcitabine, and zidovudine. Examples of protease inhibitors include, but are not limited to, amprenavir, indinavir, lopinavir, nelfinavir, ritonavir, and saquinavir.

The following Examples may be used for illustrative purposes and should not be deemed to narrow the scope of the invention.

### f. Examples

All reagents were of commercial grade and were used as received without further purification, unless otherwise stated. Commercially available anhydrous solvents were used for reactions conducted under inert atmosphere. Reagent grade solvents were used in all other cases, unless otherwise specified. Chemical shifts (δ) for ¹H NMR spectra were reported in parts per million (ppm) relative to tetramethylsilane (δ 0.00) or the appropriate residual solvent peak, i.e. CHCl₃ (δ 7.27), as internal reference. Multiplicities were given as singlet (s), doublet (d), triplet (t), quartet (q), quintuplet (quin), multiplet (m) and broad (br).

### Preparative Reverse Phase HPLC/MS Method TFA6

Samples were purified by reverse phase preparative HPLC on a Phenomenex Luna C8(2) 5 µm 100Å AXIA column (50mm × 21.2mm). A gradient of acetonitrile (A) and 0.1% trifluoroacetic acid in water (B) was used, at a flow rate of 30 mL/min (0-0.5 min 15% A, 0.5-8.0 min linear gradient 15-100% A, 8.0-9.0 min 100% A, 7.0-8.9 min 100% A, 9.0-9.1 min linear gradient 100-15% A, 9.1-10 min 15% A). A custom purification system was used, consisting of the following modules: Gilson 305 and 306 pumps; Gilson 806 Manometric module; Gilson UV/Vis 155 detector; Gilson 506C interface box; Gilson FC204 fraction collector; Agilent G1968D Active Splitter; Thermo MSQ Plus mass spectrometer. The system was controlled through a combination of Thermo Xcalibur 2.0.7 software and a custom application written in-house using Microsoft Visual Basic 6.0.

### Preparative Reverse Phase HPLC/MS Method TFA7

Samples were purified by reverse phase preparative HPLC on a Phenomenex Luna C8(2) 5 µm 100Å AXIA column (50mm × 21.2mm). A gradient of acetonitrile (A) and 0.1% trifluoroacetic acid in water (B) was used, at a flow rate of 30mL/min (0-0.5 min 25% A, 0.5-8.0 min linear gradient 25-100% A, 8.0-9.0 min 100% A, 7.0-8.9 min 100% A, 9.0-9.1 min linear gradient 100-25% A, 9.1-10 min 25% A). A custom purification system was used, consisting of the following modules: Gilson 305 and 306 pumps; Gilson 806 Manometric module; Gilson UV/Vis 155 detector; Gilson 506C interface box; Gilson FC204 fraction collector; Agilent G1968D Active Splitter; Thermo MSQ Plus mass spectrometer. The system was controlled through a combination of Thermo Xcalibur 2.0.7 software and a custom application written in-house using Microsoft Visual Basic 6.0.

### Preparative Reverse Phase HPLC/MS Method TFA8

Samples were purified by reverse phase preparative HPLC on a Phenomenex Luna C8(2) 5 µm 100Å AXIA column (50mm × 21.2mm). A gradient of acetonitrile (A) and 0.1% trifluoroacetic acid in water (B) was used, at a flow rate of 30mL/min (0-0.5 min 35% A, 0.5-8.0 min linear gradient 35-100% A, 8.0-9.0 min 100% A, 7.0-8.9 min 100% A, 9.0-9.1 min linear gradient 100-35% A, 9.1-10 min 35% A). A custom purification system was used, consisting of the following modules: Gilson 305 and 306 pumps; Gilson 806 Manometric module; Gilson UV/Vis 155 detector; Gilson 506C interface box; Gilson FC204 fraction collector; Agilent G1968D Active Splitter; Thermo MSQ Plus mass spectrometer. The system was controlled through a combination of Thermo Xcalibur 2.0.7 software and a custom application written in-house using Microsoft Visual Basic 6.0.

### Preparative Reverse Phase HPLC/MS Method AA1

Samples were purified by reverse phase preparative HPLC on a Phenomenex Luna C8(2) 5 µm 100Å AXIA column (50mm × 21.2mm). A gradient of acetonitrile (A) and 0.1% ammonium acetate in water (B) was used, at a flow rate of 30mL/min (0-0.5 min 5% A, 0.5-8.0 min linear gradient 5-100% A, 8.0-9.0 min 100% A, 7.0-8.9 min 100% A, 9.0-9.1 min linear gradient 100-5% A, 9.1-10 min 5% A). A custom purification system was used, consisting of the following modules: Gilson 305 and 306 pumps; Gilson 806 Manometric module; Gilson UV/Vis 155 detector; Gilson 506C interface box; Gilson FC204 fraction collector; Agilent G1968D Active Splitter; Thermo MSQ Plus mass spectrometer. The system was controlled through a combination of Thermo Xcalibur 2.0.7 software and a custom application written in-house using Microsoft Visual Basic 6.0.

### Embodiments:

The invention provides the following embodiments:
1. A compound of formula (I) or a pharmaceutically acceptable salt thereof, 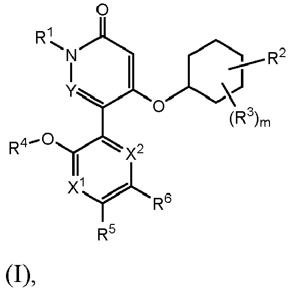 wherein
   R¹ is C₁-C₃ alkyl;
   Y is N or C(R^{Y}) wherein R^{Y} is hydrogen or C₁-C₃ alkyl;
   m is 0, 1, 2, 3, or 4;
   R² is G^{1A}, -N(R^{a})S(O)₂R^{b}, -N(R^{a})C(O)R^{b}, or -N(R^{a})C(O)OR^{b};
   R^{a}, at each occurrence, is independently hydrogen, C₁-C₆ alkyl, C₁-C₆ haloalkyl, or -(C₂-C₆ alkylenyl)-OR^{x} wherein R^{x} is hydrogen, C₁-C₆ alkyl, or C₁-C₆ haloalkyl;
   R^{b}, at each occurrence, is independently C₁-C₆ alkyl, C₂-C₆ alkenyl, C₁-C₆ haloalkyl, G^{1B}, or -(C₁-C₆ alkylenyl)-OR^{j};
   G^{1A} is formula (i) 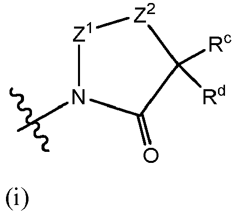 wherein
      Z¹ is C(O) or C(R^{e})(R^{f});
      Z² is a bond, O, -(C(R^{g})(R^{h}))ₚ-C(R^{m})(Rⁿ)- wherein the left end of the moiety is attached to Z¹, or N(Rⁱ) wherein Rⁱ is hydrogen, C₁-C₃ alkyl, or cyclopropyl; wherein the cyclopropyl is optionally substituted with 1, 2, 3, or 4 independently selected R^{s} groups;
      p is 0 or 1;
      R^{c}, R^{d}, R^{e}, R^{f}, R^{g}, R^{h}, R^{m}, and Rⁿ, are each independently hydrogen, C₁-C₆ alkyl, C₁-C₆ haloalkyl, or cyclopropyl; wherein the cyclopropyl, at each occurrence, is optionally substituted with 1, 2, 3, or 4 independently selected R^{s} groups; or
      R^{c} and R^{m} together with the carbon atoms to which they are attached, form a C₃-C₆ monocyclic cycloalkyl or a 4-6 membered monocyclic heterocycle; wherein the C₃-C₆ monocyclic cycloalkyl and the 4-6 membered monocyclic heterocycle are each optionally substituted with 1, 2, 3, or 4 independently selected R^{s} groups; or
      R^{c} and Rⁱ together with the atoms to which they are attached, form a 4-6 membered monocyclic heterocycle; wherein the 4-6 membered monocyclic heterocycle is optionally substituted with 1, 2, 3, or 4 independently selected R^{s} groups; or
      R^{c} and R^{d} together with the carbon atoms to which they are attached, form a C₃-C₆ monocyclic cycloalkyl or a 4-6 membered monocyclic heterocycle; wherein the C₃-C₆ monocyclic cycloalkyl and the 4-6 membered monocyclic heterocycle are each optionally substituted with 1, 2, 3, or 4 independently selected R^{s} groups;
      G^{1B} is oxetanyl, cyclopropyl, cyclobutyl, or cyclopentyl; wherein each G^{1B} is optionally substituted with 1, 2, 3, or 4 R^{t} groups; wherein each R^{t} is independently halogen, C₁-C₆ alkyl, or C₁-C₆ haloalkyl;
      each R³ is independently C₁-C₃ alkyl, C₁-C₃ haloalkyl, halogen, -CN, -OR^{3a}, -N(R^{3a})₂, -C(O)R^{3a}, cyclopropyl, or cyclobutyl; wherein each R^{3a} is independently hydrogen, C₁-C₃ alkyl, or C₁-C₃ haloalkyl;
      R⁴ is phenyl or monocyclic heteroaryl; wherein each R⁴ is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH;
      R⁵ is hydrogen, halogen, -CN, C₁-C₆ haloalkyl, or C₁-C₆ alkyl;
      R⁶ is -(C₁-C₃ alkylenyl)ₙ-S(O)₂-R^{6a} or -N(R^{6b})-SO₂-R^{6c};
      n is 0 or 1;
      R^{6a} and R^{6c} are each independently C₁-C₆ alkyl, C₁-C₆ haloalkyl, or cyclopropyl; wherein the cyclopropyl is optionally substituted with 1, 2, 3, or 4 substituents independently selected from the group consisting of halogen, C₁-C₆ alkyl, and C₁-C₆ haloalkyl;
      R^{6b} is hydrogen, C₁-C₆ alkyl, or C₁-C₆ haloalkyl;
      X¹ and X² are C(R⁷) or
      one of X¹ and X² is N and the other is C(R⁷);
      R⁷, at each occurrence, is independently hydrogen or halogen;
      R^{s}, at each occurrence, is independently C₁-C₆ alkyl, halogen, or C₁-C₆ haloalkyl;
      R^{j}, at each occurrence, is independently hydrogen, C₁-C₆ alkyl, or C₁-C₆ haloalkyl; and
      R^{k}, at each occurrence, is independently C₁-C₆ alkyl or C₁-C₆ haloalkyl.
2. The compound of embodiment 1 or a pharmaceutically acceptable salt thereof, wherein R² is G^{1A}.
3. The compound of embodiment 1 or a pharmaceutically acceptable salt thereof, wherein
   R² is -N(R^{a})S(O)₂R^{b}.
4. The compound of embodiment 1 or a pharmaceutically acceptable salt thereof, wherein
   R² is -N(R^{a})C(O)R^{b}.
5. The compound of embodiment 1 or a pharmaceutically acceptable salt thereof, wherein
   R² is -N(R^{a})C(O)OR^{b}.
6. The compound of embodiment 1 or a pharmaceutically acceptable salt thereof, wherein
   R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.
7. The compound of embodiment 1 or a pharmaceutically acceptable salt thereof, wherein
   R⁴ is monocyclic heteroaryl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.
8. The compound of embodiment 1 of formula (I) or a pharmaceutically acceptable salt thereof, wherein
   R² is G^{1A}; and
   R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.
9. The compound of embodiment 1 of formula (I) or a pharmaceutically acceptable salt thereof, wherein
   R² is -N(R^{a})C(O)R^{b}; and
   R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.
10. The compound of embodiment 1 of formula (I-a) or a pharmaceutically acceptable salt thereof,
   R² is -N(R^{a})C(O)OR^{b}; and
   R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.
11. The compound of embodiment 1 of formula (I-a) or a pharmaceutically acceptable salt thereof, 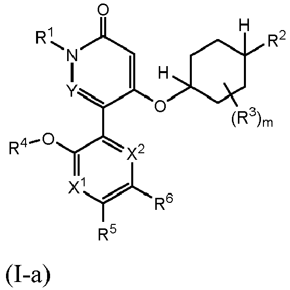 wherein R¹, Y, X¹, X², R², R³, R⁴, R⁵, R⁶, and m are as set forth in embodiment 1.
12. The compound of embodiment 1 of formula (I-b) or a pharmaceutically acceptable salt thereof, 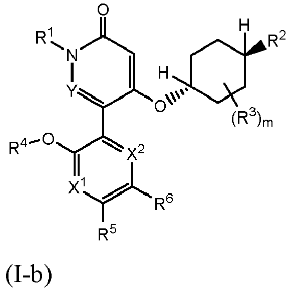 wherein R¹, Y, X¹, X², R², R³, R⁴, R⁵, R⁶, and m are as set forth in embodiment 1.
13. The compound of any one of embodiment 1-12 or a pharmaceutically acceptable salt thereof, wherein
   Y is C(R^{Y});
   X¹ is N or C(R⁷); and
   X² is C(R⁷)
14. The compound of embodiment 13 or a pharmaceutically acceptable salt thereof, wherein
   X¹ and X² are C(R⁷).
15. The compound of embodiment 13 or a pharmaceutically acceptable salt thereof, wherein
   X¹ is N and X² is C(R⁷).
16. The compound of embodiment 1 or a pharmaceutically acceptable salt thereof, wherein the compound is selected from the group consisting of
   N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]acetamide;
   N-[cis-4-({5-[2-(2,6-dimethylphenoxy)-5-(ethanesulfonyl)phenyl]-1-methyl-2-oxo-1,2-dihydropyridin-4-yl}oxy)cyclohexyl]acetamide;
   methyl [trans-4-({5-[2-(2,6-dimethylphenoxy)-5-(ethanesulfonyl)phenyl]-1-methyl-2-oxo-1,2-dihydropyridin-4-yl}oxy)cyclohexyl]carbamate;
   N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]methanesulfonamide;
   tert-butyl [trans-4-({5-[2-(2,6-dimethylphenoxy)-5-(ethanesulfonyl)phenyl]-1-methyl-2-oxo-1,2-dihydropyridin-4-yl}oxy)cyclohexyl]carbamate;
   N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-2,2,2-trifluoroacetamide;
   N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]propanamide;
   N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-2,2-dimethylpropanamide;
   N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-2-methoxyacetamide;
   N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-1-methylcyclopropane-1-carboxamide;
   N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]cyclopropanecarboxamide;
   N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-3-methyloxetane-3-carboxamide;
   N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-3,3-difluorocyclobutane-1-carboxamide;
   N-{trans-4-[(5-{5-[(ethanesulfonyl)amino]-2-[4-(2-hydroxypropan-2-yl)-2,6-dimethylphenoxy]phenyl}-1-methyl-2-oxo-1,2-dihydropyridin-4-yl)oxy]cyclohexyl}acetamide;
   methyl {trans-4-[(5-{5-[(ethanesulfonyl)amino]-2-[4-(2-hydroxypropan-2-yl)-2,6-dimethylphenoxy]phenyl}-1-methyl-2-oxo-1,2-dihydropyridin-4-yl)oxy]cyclohexyl}carbamate;
   methyl {trans-4-[(5-{5-[(ethanesulfonyl)amino]-2-(4-fluoro-2,6-dimethylphenoxy)phenyl}-1-methyl-2-oxo-1,2-dihydropyridin-4-yl)oxy]cyclohexyl}carbamate;
   methyl [trans-4-({5'-[(ethanesulfonyl)amino]-2'-(4-fluoro-2,6-dimethylphenoxy)-1-methyl-6-oxo[1,6-dihydro[3,3'-bipyridine]]-4-yl}oxy)cyclohexyl]carbamate;
   methyl{trans-4-[(5-{2-(4-fluoro-2,6-dimethylphenoxy)-5-[(methanesulfonyl)methyl]phenyl}-1-methyl-2-oxo-1,2-dihydropyridin-4-yl)oxy]cyclohexyl}carbamate;
   5-[5-(ethanesulfonyl)-2-(4-fluoro-2,6-dimethylphenoxy)phenyl]-1-methyl-4-{[trans-4-(2-oxopyrrolidin-1-yl)cyclohexyl]oxy}pyridin-2(1H)-one; and
   N-[trans-4-({5-[5-(ethanesulfonyl)-2-(4-fluoro-2,6-dimethylphenoxy)phenyl]-1-methyl-2-oxo-1,2-dihydropyridin-4-yl}oxy)cyclohexyl]-N-methylacetamide.
17. A pharmaceutical composition comprising a therapeutically effective amount of a compound of formula (I) according to embodiment 1, or a pharmaceutically acceptable salt thereof, in combination with a pharmaceutically acceptable carrier.
18. A method for treating cancer in a subject comprising administering a therapeutically effective amount of a compound of formula (I) according to embodiment 1 or a pharmaceutically acceptable salt thereof, to a subject in need thereof.
19. The method of embodiment 18 wherein the cancer is selected from the group consisting of: acoustic neuroma, acute leukemia, acute lymphocytic leukemia, acute myelocytic leukemia (monocytic, myeloblastic, adenocarcinoma, angiosarcoma, astrocytoma, myelomonocytic and promyelocytic), acute t-cell leukemia, basal cell carcinoma, bile duct carcinoma, bladder cancer, brain cancer, breast cancer, bronchogenic carcinoma, cervical cancer, chondrosarcoma, chordoma, choriocarcinoma, chronic leukemia, chronic lymphocytic leukemia, chronic myelocytic (granulocytic) leukemia, chronic myelogenous leukemia, colon cancer, colorectal cancer, craniopharyngioma, cystadenocarcinoma, diffuse large B-cell lymphoma, dysproliferative changes (dysplasias and metaplasias), embryonal carcinoma, endometrial cancer, endotheliosarcoma, ependymoma, epithelial carcinoma, erythroleukemia, esophageal cancer, estrogen-receptor positive breast cancer, essential thrombocythemia, Ewing's tumor, fibrosarcoma, follicular lymphoma, germ cell testicular cancer, glioma, glioblastoma, gliosarcoma, heavy chain disease, hemangioblastoma, hepatoma, hepatocellular cancer, hormone insensitive prostate cancer, leiomyosarcoma, leukemia, liposarcoma, lung cancer, lymphagioendotheliosarcoma, lymphangiosarcoma, lymphoblastic leukemia, lymphoma (Hodgkin's and non-Hodgkin's), malignancies and hyperproliferative disorders of the bladder, breast, colon, lung, ovaries, pancreas, prostate, skin, and uterus, lymphoid malignancies of T-cell or B-cell origin, leukemia, lymphoma, medullary carcinoma, medulloblastoma, melanoma, meningioma, mesothelioma, multiple myeloma, myelogenous leukemia, myeloma, myxosarcoma, neuroblastoma, NUT midline carcinoma (NMC), non-small cell lung cancer, oligodendroglioma, oral cancer, osteogenic sarcoma, ovarian cancer, pancreatic cancer, papillary adenocarcinomas, papillary carcinoma, pinealoma, polycythemia vera, prostate cancer, rectal cancer, renal cell carcinoma, retinoblastoma, rhabdomyosarcoma, sarcoma, sebaceous gland carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenstrom's macroglobulinemia, testicular tumors, uterine cancer, and Wilms' tumor.
20. A method for treating a disease or condition in a subject comprising administering a therapeutically effective amount of a compound of formula (I) according to embodiment 1 or a pharmaceutically acceptable salt thereof, to a subject in need thereof, wherein said disease or condition is selected from the group consisting of Addison's disease, acute gout, ankylosing spondylitis, asthma, atherosclerosis, Behcet's disease, bullous skin diseases, chronic obstructive pulmonary disease (COPD), Crohn's disease, dermatitis, eczema, giant cell arteritis, glomerulonephritis, hepatitis, hypophysitis, inflammatory bowel disease, Kawasaki disease, lupus nephritis, multiple sclerosis, myocarditis, myositis, nephritis, organ transplant rejection, osteoarthritis, pancreatitis, pericarditis, polyarteritis nodosa, pneumonitis, primary biliary cirrhosis, psoriasis, psoriatic arthritis, rheumatoid arthritis, scleritis, sclerosing cholangitis, sepsis, systemic lupus erythematosus, Takayasu's Arteritis, toxic shock, thyroiditis, type I diabetes, ulcerative colitis, uveitis, vitiligo, vasculitis, and Wegener's granulomatosis.
21. A method for treating an acquired immunodeficiency syndrome (AIDS) in a subject comprising administering a therapeutically effective amount of a compound of formula (I) according to embodiment 1 or a pharmaceutically acceptable salt thereof, to a subject in need thereof.
22. A method for treating a disease or condition in a subject comprising administering a therapeutically effective amount of a compound of formula (I) according to embodiment 1 or a pharmaceutically acceptable salt thereof, to a subject in need thereof, wherein said disease or condition is selected from the group consisting of: obesity, dyslipidemia, hypercholesterolemia, Alzheimer's disease, metabolic syndrome, hepatic steatosis, type II diabetes, insulin resistance, diabetic retinopathy, and diabetic neuropathy.
23. A method of contraception in a male subject comprising administering a therapeutically effective amount of a compound of formula (I) according to embodiment 1 or a pharmaceutically acceptable acceptable salt thereof, to a subject in need thereof.
24. A method for treating an acute kidney disease or condition in a subject comprising administering a therapeutically effective amount of a compound of embodiment 1 or a pharmaceutically acceptable salt thereof, to a subject in need thereof, wherein said acute kidney disease or condition is selected from the group consisting of: ischemia-reperfusion induced kidney disease, cardiac and major surgery induced kidney disease, percutaneous coronary intervention induced kidney disease, radio-contrast agent induced kidney disease, sepsis induced kidney disease, pneumonia induced kidney disease, and drug toxicity induced kidney disease.
25. A method of treating a chronic kidney disease or condition in a subject comprising administering a therapeutically effective amount of a compound of embodiment 1 or a pharmaceutically acceptable salt thereof, to a subject in need thereof, wherein said disease or condition is selected from the group consisting of: diabetic nephropathy, hypertensive nephropathy, HIV-associated nephropathy, glomerulonephritis, lupus nephritis, IgA nephropathy, focal segmental glomerulosclerosis, membranous glomerulonephritis, minimal change disease, polycystic kidney disease and tubular interstitial nephritis.

### Example 1

### N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]acetamide

### Example 1a

### 2-(2-bromo-4-(ethylsulfonyl)phenoxy)-1,3-dimethylbenzene

2,6-Dimethylphenol (2.195 g, 17.97 mmol), 2-bromo-4-(ethylsulfonyl)-1-fluorobenzene (4 g, 14.97 mmol) and cesium carbonate (5.85 g, 17.97 mmol) were combined in dimethylsulfoxide (29.9 mL) under nitrogen and heated at 110 °C overnight, cooled and partitioned between ethyl acetate and water. The mixture was extracted with ethyl acetate (300 mL) three times. The combined organic layers was washed with saturated aqueous saturated aqueous sodium chloride (100 mL) twice, dried over anhydrous sodium sulfate, filtered and concentrated. The residue was purified by chromatography (silica gel, 10-50% ethyl acetate in heptanes) to afford the title compound (5.19 g, 94%).

### Example 1b

### 4-(benzyloxy)-5-bromo-1-methylpyridin-2(1H)-one

Under N₂ to a solution of phenylmethanol (4.86 g, 45.0 mmol) in anhydrous tetrahydrofuran (50 mL) was added 1.0M potassium *tert*-butoxide (45.0 mL, 45.0 mmol) dropwise via a cannula over 20 minutes at -7°C in an ice/methanol. The temperature rose to about -5 °C. After the addition was complete, the reaction mixture was warmed to 0°C and stirred for about 30 minutes. To this solution was added dropwise a solution of 5-bromo-4-chloro-1-methylpyridin-2(1*H*)-one (5 g, 22.48 mmol) in anhydrous tetrahydrofuran (50 mL). Temperature rose to 0°C. The mixture was stirred for 2 hours at 0°C and overnight at ambient temperature. The mixture was quenched with saturated ammonium chloride and water, extracted with ethyl acetate and washed with saturated aqueous sodium chloride, dried with Na₂SO₄, filtered and concentrated under reduced pressure. Petroleum ether/ethyl acetate (10:1) was added to the residue, and the solid formed was collected by filtration to provide the title compound (5.7 g, 19.38 mmol, 86 % yield).

### Example 1c

### 4-(benzyloxy)-1-methyl-5-(4,4,5,5-tetramethyl-1,3,2-dioxaborolan-2-yl)pyridin-2(1H)-one

Example 1b (9.3 g, 31.6 mmol), potassium acetate (7.14 g, 72.7 mmol), and 4,4,4',4',5,5,5',5'-octamethyl-2,2'-bi(1,3,2-dioxaborolane) (12.04 g, 47.4 mmol) were combined in a flask and purged with nitrogen for 10 minutes. Dicyclohexyl(2',4',6'-triisopropyl-[1,1'-biphenyl]-2-yl)phosphine (1.507 g, 3.16 mmol) and tris(dibenzylideneacetone)dipalladium(0) (0.724 g, 0.790 mmol) were added and the mixture was purged for another 10 minutes. In the meantime, the dioxane was degassed for 10 minutes and then added to the reactor. The reaction mixture was stirred for about 7 hours at 80 °C (internal temp). It was then cooled to ambient temperature and silica gel was added and the mixture concentrated down by rotary evaporation. The dried silica loaded with reaction mixture residue was placed over a bed of silica gel and ethyl acetate was passed over until all color came off. The filtrate was concentrated by rotary evaporation and diethyl ether was added. Upon sonication, solids crashed out and the flask was placed in freezer over weekend. The solids were collected by filtration, quickly washed with cold diethyl ether and dried to give the title compound (7 g, 20.52 mmol, 64.9 % yield) as a light tan solid.

### Example 1d

### 4-(benzyloxy)-5-(2-(2,6-dimethylphenoxy)-5-(ethylsulfonyl)phenyl)-1-methylpyridin-2(1H)-one

A 500 mL 3-neck round bottom flask was charged with Example 1c (20 g, 58.6 mmol), Example 1a (23.81 g, 64.5 mmol), 1,3,5,7-tetramethyl-6-phenyl-2,4,8-trioxa-6-phosphaadamantane (1.713 g, 5.86 mmol), sodium carbonate (24.85 g, 234 mmol, dried in oven), and tris(dibenzylideneacetone)dipalladium(0) (1.610 g, 1.758 mmol). A stream of nitrogen was blown over these solids for one hour to degas and deoxygenate the material. In the meantime, in a dried flask were mixed anhydrous tetrahydrofuran (200 mL) and water (50.0 mL). This solution was degassed for one hour by bubbling nitrogen through it. After one hour the solvents were transferred via cannula into the solids. The reaction mixture was heated at 60 °C overnight. Reaction was cooled and diluted with ethyl acetate and water. The two-phase mixture stirred for 30 minutes at ambient temperature. The layers were separated and the aqueous layer back extracted with ethyl acetate. The combined organic layers were washed with saturated aqueous sodium chloride. The orange brown organic layer was stirred for about one hour with about 20 g. SiliaMetS Thiol palladium scavenger (Silicycle). Anhydrous sodium sulfate was added, and mixture stirred for an additional 30 minutes. The mixture was then filtered through diatomaceous earth with ethyl acetate washes, and the solvents were removed under reduced pressure. The residue was purified by flash chromatography on an Analogix MPLC using a Grace Reveleris 330 g silica gel column, eluting with ethyl acetate and then 2% to 5% methanol/ ethyl acetate to give the title compound (24 g, 81% yield) as a white, foamy solid.

### Example 1e

### 5-(2-(2,6-dimethylphenoxy)-5-(ethylsulfonyl)phenyl)-4-hydroxy-1-methylpyridin-2(1H)-one

Example 1d (24 g, 47.7 mmol) and tetrahydrofuran (240 mL) were added to 20% Pd(OH)₂/C, wet (4.8 g, 3.49 mmol) in a 500 mL stainless steel pressure bottle and stirred or shaken for 45 minutes at 30 psi of hydrogen at ambient temperature. The solid was removed by filtration. The filtrate was concentrated to give 17.2 g of title compound.

### Example 1f

### 4-chloro-5-(2-(2,6-dimethylphenoxy)-5-(ethylsulfonyl)phenyl)-1-methylpyridin-2(1H)-one

A mixture of Example 1e (0.8 g, 1.935 mmol) and phosphorus oxychloride (18.03 mL, 193 mmol) was heated at 80 °C for 2 hours. The excess phosphorus oxychloride was removed, and the solid was purified by flash chromatography on silica gel eluting with 100:1 ethyl acetate/methanol to give the title compound (0.31 g, 0.718 mmol, 37.1 % yield).

### Example 1g

### N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]acetamide

Sodium hydride (14.0 mg, 0.58 mmol, 5.0 equivalents) was weighed into a 4 mL vial and a stock solution of *N*-(4-hydroxycyclohexyl)acetamide (53.0 mg 0.35 mmoL, 3.0 equivalents., mixture of cis and trans isomers) in 500 µL of dioxane was added. This mixture was stirred for 10 minutes at ambient temperature. Example 1f (50 mg, 0.12 mmol, 1.0 equivalent) in 500 µL of dioxane was added and the reaction mixture was heated at 100 °C for 24 hours. Additional sodium hydride (14.0 mg, 0.58 mmol, 5.0 equivalents) was added and the reaction mixture was heated at 100 °C for 12 hours. The reaction mixture was cooled to ambient temperature and quenched with water and purified using preparative reverse phase HPLC/MS method AA1. Fractions for the runs were consolidated and dried down to yield the title compound as the first eluting isomer (13.5 mg, 21% yield). ¹H NMR (400 MHz, DMSO-*d₆* :D₂O = 9:1 (v/v)) δ 7.86 (d, *J* = 7.5 Hz, 1H), 7.77 - 7.67 (m, 3H), 7.22 - 7.06 (m, 3H), 6.52 (d, *J* = 8.6 Hz, 1H), 6.02 (s, 1H), 4.45 - 4.36 (m, 1H), 3.42 (s, 4H), 3.24 (q, *J* = 7.4 Hz, 2H), 2.05 - 1.92 (m, 8H), 1.78 (s, 3H), 1.75 - 1.63 (m, 2H), 1.36 - 1.21 (m, 4H), 1.09 (t, *J* = 7.3 Hz, 3H). MS (APCI+) m/z 553.1 (M+H⁺).

### Example 2

### N-[cis-4-({5-[2-(2,6-dimethylphenoxy)-5-(ethanesulfonyl)phenyl]-1-methyl-2-oxo-1,2-dihydropyridin-4-yl}oxy)cyclohexyl]acetamide

Example 2 was isolated as the second eluting isomer from the preparative reverse phase HPLC described in Example 1_{g} (25.2 mg, 39% yield). ¹H NMR (400 MHz, DMSO-*d₆* :D₂O = 9:1 (v/v)) δ 7.81 - 7.70 (m, 3H), 7.22 - 7.09 (m, 3H), 6.55 (d, *J* = 8.6 Hz, 1H), 5.99 (s, 1H), 4.58 (s, 1H), 3.51 (t, *J* = 10.0 Hz, 1H), 3.42 (s, 3H), 3.28 (q, *J* = 7.4 Hz, 2H), 2.02 (s, 6H), 1.82 - 1.72 (m, 5H), 1.68 - 1.46 (m, 4H), 1.23 - 1.08 (m, 5H). MS (APCI+) m/z 553.1 (M+H⁺).

### Example 3

### methyl[trans-4-({5-[2-(2,6-dimethylphenoxy)-5-(ethanesulfonyl)phenyl]-1-methyl-2-oxo-1,2-dihydropyridin-4-yl}oxy)cyclohexyl]carbamate

### Example 3a

### 4-[(trans-4-aminocyclohexyl)oxy]-5-[2-(2,6-dimethylphenoxy)-5-(ethanesulfonyl)phenyl]-1-methylpyridin-2(1H)-one

Sodium hydride (139 mg, 5.79 mmol, 5.0 equivalents) was weighed into a 4 mL vial. Tert-butyl(*trans*-4-hydroxycyclohexyl)carbamate (748 mg, 3.47 mmol, 3.0 equivalents) in dioxane (1 mL) was added and stirred for 10 minutes at ambient temperature. Example 1f (500 mg, 1.158 mmol, 1.0 equivalent) was added and the reaction was heated at 100 °C overnight. After 24 hours, additional sodium hydride (139 mg, 5.79 mmol, 5.0 equivalents) was added and the reaction mixture was heated at 100 °C overnight. After the additional 24 hours, the reaction mixture was dosed with additional sodium hydride (139 mg, 5.79 mmol, 5.0 equivalents) and heated at 100 °C for 5 days. The reaction mixture was quenched with water and the residue was purified using preparative reverse phase HPLC/MS method TFA6 to yield the title compound (345 mg, 58% yield) as a trifluoroacetate salt. ¹H NMR (400 MHz, DMSO-*d*₆) δ 7.86 (s, 2H), 7.78 - 7.67 (m, 3H), 7.22 - 7.08 (m, 3H), 6.49 (d, *J* = 8.5 Hz, 1H), 6.08 (s, 1H), 4.44 - 4.30 (m, 1H), 3.40 (s, 3H), 3.24 (q, *J* = 7.3 Hz, 2H), 3.05 - 2.91 (m, 1H), 2.09 - 1.97 (m, 8H), 1.91 - 1.81 (m, 2H), 1.55 - 1.34 (m, 2H), 1.32 - 1.16 (m, 2H), 1.12 - 1.03 (m, 3H).MS (APCI+) m/z 511.4 (M+H⁺).

### Example 3b

### methyl [trans-4-({5-[2-(2,6-dimethylphenoxy)-5-(ethanesulfonyl)phenyl]-1-methyl-2-oxo-1,2-dihydropyridin-4-yl}oxy)cyclohexyl]carbamate

A mixture of Example 3a (17 mg, 0.033 mmol, 1.0 equivalent), methyl chloroformate (3.09 µL, 0.040 mmol, 1.2 equivalents), and triethylamine (0.014 mL, 0.100 mmol, 3.0 equivalents) in dichloromethane (1 mL) was stirred at room temperature until completion as determined by LC/MS. The mixture was concentrated under a stream of nitrogen. The residue was reconstituted in methanol and purified using preparative reverse phase HPLC/MS method TFA8 to yield the title compound (3.2 mg, 17% yield). ¹H NMR (400 MHz, DMSO-*d*₆) δ 7.76 - 7.67 (m, 3H), 7.20 - 7.07 (m, 4H), 6.49 (d, *J* = 8.4 Hz, 1H), 6.00 (s, 1H), 4.39 - 4.34 (m, 1H), 3.48 (s, 3H), 3.39 (s, 3H), 3.29 - 3.19 (m, 3H), 2.03 - 1.94 (m, 8H), 1.73 - 1.65 (m, 2H), 1.35 - 1.20 (m, 4H), 1.08 (t, *J* = 7.3 Hz, 3H). MS (APCI+) m/z 569.0 (M+H⁺).

### Example 4

### N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]methanesulfonamide

A mixture of Example 3a (17 mg, 0.033 mmol, 1.0 equivalent), methanesulfonyl chloride (5.15 µL, 0.067 mmol, 2.0 equivalents), and triethylamine (0.014 mL, 0.100 mmol, 3.0 equivalents) in dichloromethane (1 mL) was stirred at room temperature for 4 hours. An additional aliquot of methanesulfonyl chloride (5.15 µL, 0.067 mmol, 2.0 equivalents) was added followed by the addition of pyridine (8.08 µL, 0.100 mmol. 3.0 equivalents). The reaction mixture was stirred at ambient temperature for 10 minutes, and concentrated under a stream of nitrogen. The residue was reconstituted in methanol and purified using preparative reverse phase HPLC/MS method TFA6 to yield the title compound (3.1 mg, 16% yield). ¹H NMR (400 MHz, DMSO-*d*₆) δ 7.74 - 7.67 (m, 3H), 7.18 - 7.06 (m, 3H), 7.00 (d, *J* = 6.8 Hz, 1H), 6.47 (d, *J* = 8.3 Hz, 1H), 5.99 (s, 1H), 4.35 (s, 1H), 3.38 (s, 3H), 3.21 (t, *J* = 7.3 Hz, 2H), 3.13 (d, *J* = 5.2 Hz, 1H), 2.84 (s, 3H), 1.99 (s, 6H), 1.97 - 1.90 (m, 2H), 1.81 - 1.71 (m, 2H), 1.44 - 1.16 (m, 4H), 1.07 (q, *J* = 7.1 Hz, 3H). MS (APCI+) m/z 589.0 (M+H⁺).

### Example 5

### tert-butyl [trans-4-({5-[2-(2,6-dimethylphenoxy)-5-(ethanesulfonyl)phenyl]-1-methyl-2-oxo-1,2-dihydropyridin-4-yl}oxy)cyclohexyl]carbamate

A mixture of Example 3a (10 mg, 0.020 mmol, 1.0 equivalent) and di-tert-butyl dicarbonate (7.01 µL, 0.039 mmol, 2.0 equivalents) in acetonitrile (1 mL) was stirred overnight at ambient temperature. The reaction mixture was purified using preparative reverse phase HPLC/MS method TFA8 to yield the title compound (3.4 mg, 28% yield). ¹H NMR (400 MHz, DMSO-*d₆* :D₂O = 9:1 (v/v)) δ 7.74 - 7.60 (m, 3H), 7.16 - 7.03 (m, 3H), 6.46 (d, *J* = 8.7 Hz, 1H), 5.95 (s, 1H), 4.30 (s, 1H), 3.37 (s, 3H), 3.19 (q, *J* = 7.4 Hz, 2H), 3.14-3.06 (m, 1H), 1.96 (s, 8H), 1.71 - 1.55 (m, 2H), 1.33 - 1.11 (m, 13H), 1.04 (t, *J* = 7.3 Hz, 3H). MS (APCI+) m/z 611.2 (M+H⁺).

### Example 6

### N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-2,2,2-trifluoroacetamide

Example 6 was a major byproduct isolated during the preparation and isolation of Example 4 (7.85 mg, 39%). ¹H NMR (501 MHz, DMSO-*d₆* :D₂O = 9:1 (v/v)) δ 9.33 (d, *J* = 7.5 Hz, 1H), 7.79 - 7.70 (m, 3H), 7.21 - 7.10 (m, 3H), 6.52 (d, *J* = 8.5 Hz, 1H), 6.08 (s, 1H), 4.45 - 4.35 (m, 1H), 3.61 - 3.53 (m, 1H), 3.42 (s, 3H), 3.27 (q, *J* = 7.3 Hz, 2H), 2.05 - 2.01 (m, 8H), 1.81 - 1.73 (m, 2H), 1.57 - 1.45 (m, 2H), 1.34 - 1.22 (m, 2H), 1.11 (t, *J* = 7.3 Hz, 3H). MS (APCI+) m/z 607.0 (M+H⁺).

### Example 7

### N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]propanamide

A mixture of Example 3a (0.07 M in dimethylacetamide, 350 µL, 0.024 mmol, 1.0 equivalent), propionic acid (0.4 M in dimethylacetamide, 92 µL, 0.036 mmol, 1.5 equivalents), HATU (1-[bis(dimethylamino)methylene]-1*H*-1,2,3-triazolo[4,5-b]pyridinium 3-oxid hexafluorophosphate, 0.083 M in dimethylacetamide, 350 µL, 0.029 mmol, 1.2 equivalents) and diisopropylethylamine (21 µL, 0.12 mmol, 5.0 equivalents) were combined at room temperature and stirred until complete by LC/MS. The reaction mixture was loaded directly into an injection loop and purified using preparative reverse phase HPLC/MS method TFA7 to provide the title compound (5.38 mg, 39% yield). ¹H NMR (400 MHz, DMSO-*d₆* :D₂O = 9:1 (v/v)) δ 7.77 - 7.67 (m, 3H), 7.21 - 7.08 (m, 3H), 6.52 (d, *J* = 8.6 Hz, 1H), 6.02 (s, 1H), 4.46 - 4.36 (m, 1H), 3.52 - 3.40 (m, 4H), 3.24 (q, *J* = 7.3 Hz, 2H), 2.09 - 1.89 (m, 10H), 1.73 - 1.65 (m, 2H), 1.38 - 1.20 (m, 4H), 1.09 (t, *J* = 7.4 Hz, 3H), 0.96 (t, *J* = 7.6 Hz, 3H). MS (APCI+) m/z 567.4 (M+H⁺).

### Example 8

### N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-2,2-dimethylpropanamide

Example 8 was prepared using the procedure for the preparation of Example 7, substituting 2,2-dimethyl-propionic acid for propionic acid. ¹H NMR (400 MHz, DMSO-*d₆* :D₂O = 9:1 (v/v)) δ 7.77 - 7.67 (m, 3H), 7.21 - 7.08 (m, 3H), 6.51 (d, *J* = 8.6 Hz, 1H), 6.00 (s, 1H), 4.37 - 4.29 (m, 1H), 3.53 - 3.40 (m, 4H), 3.24 (q, *J* = 7.3 Hz, 2H), 2.04 - 1.96 (m, 8H), 1.71 - 1.63 (m, 2H), 1.47 - 1.33 (m, 2H), 1.30 - 1.19 (m, 2H), 1.10 (d, *J* = 7.9 Hz, 3H), 1.05 (s, 9H). MS (APCI+) m/z 595.4 (M+H⁺).

### Example 9

### N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-2-methoxyacetamide

Example 9 was prepared using the procedure for the preparation of Example 7, substituting methoxyacetic acid for propionic acid. ¹H NMR (400 MHz, DMSO-*d₆* :D₂O = 9:1 (v/v)) δ 7.77 - 7.67 (m, 3H), 7.21 - 7.08 (m, 3H), 6.51 (d, *J* = 8.6 Hz, 1H), 6.02 (s, 1H), 4.48 - 4.25 (m, 1H), 3.75 (s, 3H), 3.59 - 3.53 (m, 0H), 3.42 (s, 3H), 3.34 - 3.19 (m, 5H), 2.04 - 1.95 (m, 8H), 1.73 - 1.65 (m, 2H), 1.50 - 1.36 (m, 2H), 1.33 - 1.19 (m, 2H), 1.10 (t, *J* = 7.3 Hz, 3H). MS (APCI+) m/z 583.4 (M+H⁺).

### Example 10

### N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-1-methylcyclopropane-1-carboxamide

Example 10 was prepared using the procedure for the preparation of Example 7, substituting 1-methyl-cyclopropanecarboxylic acid for propionic acid. ¹H NMR (400 MHz, DMSO-*d₆* :D₂O = 9:1 (v/v)) δ 7.76 - 7.67 (m, 3H), 7.21 - 7.08 (m, 3H), 6.51 (d, *J* = 8.7 Hz, 1H), 6.00 (s, 1H), 4.42 - 4.26 (m, 1H), 3.53 - 3.48 (m, 1H), 3.42 (s, 3H), 3.24 (q, *J* = 7.3 Hz, 2H), 2.04 - 1.96 (m, 8H), 1.72 - 1.64 (m, 2H), 1.50 - 1.36 (m, 2H), 1.29 - 1.21 (m, 2H), 1.21 (s, 3H), 1.09 (t, *J* = 7.3 Hz, 3H), 0.94 - 0.87 (m, 2H), 0.52 - 0.45 (m, 2H). MS (APCI+) m/z 593.3 (M+H⁺).

### Example 11

### N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]cyclopropanecarboxamide

Example 11 was prepared using the procedure for the preparation of Example 7, substituting cyclopropanecarboxylic acid for propionic acid. ¹H NMR (400 MHz, DMSO-*d₆* :D₂O = 9:1 (v/v)) δ 7.77 - 7.67 (m, 3H), 7.21 - 7.08 (m, 3H), 6.51 (d, *J* = 8.7 Hz, 1H), 6.03 (s, 1H), 4.43 - 4.38 (m, 1H), 3.53 - 3.40 (m, 4H), 3.23 (q, *J* = 7.3 Hz, 2H), 2.04 - 1.92 (m, 8H), 1.74 - 1.66 (m, 2H), 1.57 - 1.46 (m, 1H), 1.40 - 1.20 (m, 4H), 1.09 (t, *J* = 7.3 Hz, 3H), 0.67 - 0.50 (m, 4H). MS (APCI+) m/z 579.4 (M+H⁺).

### Example 12

### N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-3-methyloxetane-3-carboxamide

Example 12 was prepared using the procedure for the preparation of Example 7, substituting 3-methyloxetane-3-carboxylic acid for propionic acid. ¹H NMR (400 MHz, DMSO-*d₆* :D₂O = 9:1 (v/v)) δ 7.77 - 7.68 (m, 3H), 7.21 - 7.08 (m, 3H), 6.52 (d, *J* = 8.6 Hz, 1H), 6.03 (s, 1H), 4.66 (d, *J* = 6.1 Hz, 2H), 4.42 - 4.35 (m, 1H), 4.23 (d, *J* = 6.0 Hz, 2H), 3.54 - 3.45 (m, 1H), 3.42 (s, 3H), 3.24 (q, *J* = 7.3 Hz, 2H), 2.04 - 1.96 (m, 8H), 1.75 - 1.67 (m, 2H), 1.44 (s, 3H), 1.42 - 1.21 (m, 4H), 1.10 (d, *J* = 7.2 Hz, 3H). MS (APCI+) m/z 609.3 (M+H⁺).

### Example 13

### N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-3,3-difluorocyclobutane-1-carboxamide

Example 13 was prepared using the procedure for the preparation of Example 7, substituting 3,3-difluorocyclobutanecarboxylic acid for propionic acid. ¹H NMR (400 MHz, DMSO-*d₆* :D₂O = 9:1 (v/v)) δ 7.79 - 7.67 (m, 3H), 7.21 - 7.08 (m, 3H), 6.52 (d, *J* = 8.6 Hz, 1H), 6.03 (s, 1H), 4.40 (s, 1H), 3.50 - 3.45 (m, 1H), 3.42 (s, 3H), 3.24 (q, *J* = 7.3 Hz, 2H), 2.88 - 2.76 (m, 1H), 2.71 - 2.57 (m, 4H), 2.04 - 1.93 (m, 8H), 1.74 - 1.66 (m, 2H), 1.39 - 1.21 (m, 4H), 1.09 (t, *J* = 7.3 Hz, 3H). MS (APCI+) m/z 629.4 (M+H⁺).

### Example 14

### N-{trans-4-[(5-{5-[(ethanesulfonyl)amino]-2-[4-(2-hydroxypropan-2-yl)-2,6-dimethylphenoxy]phenyl}-1-methyl-2-oxo-1,2-dihydropyridin-4-yl)oxy]cyclohexyl}acetamide

### Example 14a

### 1-(4-(2-bromo-4-nitrophenoxy)-3,5-dimethylphenyl)ethan-1-one

To a solution of 3,5-dimethyl-4-hydroxyacetophenone (CAS 5325-04-2, 4.66 g, 28.4 mmol) and 2-bromo-1-fluoro-4-nitrobenzene (CAS 701-45-1, 5.67 g, 25.8 mmol) in dimethyl sulfoxide (25.8 mL) was added cesium carbonate (12.60 g, 38.7 mmol). The mixture was stirred at 70 °C under nitrogen for 2 hours, cooled to ambient temperature, diluted with 300 mL of water, and stirred for 10 minutes. The mixture was extracted 4 x 100 mL with ethyl acetate. The organic extracts were combined, washed with saturated aqueous sodium chloride, dried over anhydrous sodium sulfate, filtered, and concentrated to afford the title compound that was used without further purification (9.48 g, 95%).

### Example 14b

### 1-(4-(4-amino-2-bromophenoxy)-3,5-dimethylphenyl)ethan-1-one

Example 14a (5.0 g, 13.73 mmol), iron powder (3.83 g, 68.6 mmol), and ammonia hydrochloride (1.102 g, 20.59 mmol) were combined in the solvent mixture of tetrahydrofuran (29.4 mL), ethanol (29.4 mL) and water (9.81 mL) and heated at 95 °C with vigorous stirring for 2 hours. The mixture was cooled and filtered through a plug of diatomaceous earth to remove solids. The plug was rinsed repeatedly with methanol and tetrahydrofuran. The filtrate was concentrated and the residue partitioned between ethyl acetate and water. The ethyl acetate layer was washed with saturated aqueous sodium chloride, dried over anhydrous sodium sulfate, filtered, and concentrated to afford the title compound as a tan solid (4.89 g, quantitative yield).

### Example 14c

### N-(4-(4-acetyl-2,6-dimethylphenoxy)-3-bromophenyl)ethanesulfonamide

A solution of Example 14b (1.0 g, 3.0 mmol) and triethylamine (1.25 mL, 8.98 mmol) in dichloromethane (7.48 mL) at ambient temperature under nitrogen was treated drop wise with ethanesulfonyl chloride (0.808 g, 6.28 mmol). The mixture was stirred for two hours and concentrated. The residue was dissolved in 1,4-dioxane (7.48 mL), treated with 2.0 M aqueous sodium hydroxide (4 mL, 8.00 mmol) and heated at 90 °C for 2 hours. The reaction mixture was cooled, partitioned between ethyl acetate and water, and the pH was adjusted to pH 8 by careful addition of 1 M hydrochloric acid. The organic layer was washed with saturated aqueous sodium chloride, dried over anhydrous sodium sulfate, filtered, and concentrated to afford the title compound as a yellow solid (1.369 g, 92%).

### Example 14d

### N-(3-bromo-4-(4-(2-hydroxypropan-2-yl)-2,6-dimethylphenoxy)phenyl)ethanesulfonamide

To a solution of Example 14c (1.37 g, 3.21 mmol) in tetrahydrofuran (20 mL) at 20 °C under nitrogen was added dropwise methylmagnesium bromide (4.28 mL, 12.85 mmol, 3M in diethyl ether). The solution was stirred at ambient temperature for 5 hours, quenched with 5% aqueous ammonium chloride and partitioned with ethyl acetate and water. The organic layer was washed with saturated aqueous sodium chloride, dried over anhydrous sodium sulfate, filtered and concentrated. The residue was purified by flash chromatography (silica gel, 5-50% ethyl acetate in heptanes) to afford the title compound as a white powder (0.8 g, 53%).

### Example 14e

### N-(4-(4-(2-hydroxypropan-2-yl)-2,6-dimethylphenoxy)-3-(4,4,5,5-tetramethyl-1,3,2-dioxaborolan-2-yl)phenyl)ethanesulfonamide

A mixture of Example 14d (0.74 g, 1.673 mmol), 4,4,4',4',5,5,5',5'-octamethyl-2,2'-bi(1,3,2-dioxaborolane) (0.850 g, 3.35 mmol), tris(dibenzylideneacetone)dipalladium(0) (0.038 g, 0.042 mmol) and potassium acetate (0.328 g, 3.35 mmol) were combined in a microwave tube, sealed, and sparged with argon for 10 minutes. To this mixture was added argon sparged 2-methyl tetrahydrofuran (6.97 mL) and the mixture was heated at 80 °C for 48 hours, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered and concentrated. The residue was purified by chromatography (silica gel, 5-60% of ethyl acetate in heptanes) to afford the title compound (0.62 g, 72%).

### Example 14f

### 5-bromo-4-fluoropyridin-2-amine

To a solution of 2-amino-4-fluoropyridine (CAS 944401-77-8, 17.56 g, 157 mmol) in acetonitrile (234 mL) at 5 °C under nitrogen in a foil wrapped round bottom flask was added portionwise N-bromosuccinimide (28.4 g, 160 mmol). Upon completion of the addition the mixture was stirred in darkness, warming to ambient temperature over 1 hour. The reaction mixture was concentrated on the rotary evaporator and the residue was washed repeatedly with water (3 x 300 mL) to remove the succinimide. The resulting solid was collected by filtration and dried to constant mass affording the title compound as a light tan powder (23.1 g, 76% yield)

### Example 14g

### 5-bromo-4-fluoropyridin-2-ol

To a solution of sulfuric acid (21.81 mL, 393 mmol) and water (25 mL) at ambient temperature was added portionwise the product from Example 14f (15 g, 79 mmol). The resulting solution was cooled to 5 °C and treated dropwise with a solution of sodium nitrite (17.88 g, 259 mmol) in water (50 mL). The resulting mixture was stirred at 5-10 °C for four hours and treated dropwise with ammonium hydroxide (43.7 mL, 314 mmol) to a constant pH of 9. The mixture was stirred for 20 minutes and the solid was collected by filtration, rinsed with cold water, and dried to a constant mass to afford the title compound as a tan solid (14.5 g, 92% yield).

### Example 14h

### 5-bromo-4-fluoro-1-methylpyridin-2(1H)-one

To a mixture of Example 14g (29 g, 151 mmol) and cesium carbonate (59.1 g, 181 mmol) in dimethylformamide (150 mL) at ambient temperature under nitrogen was added dropwise iodomethane (11.3 mL, 181 mmol), maintaining the temperature below 30 °C. The addition was done over a period of approximately 30 minutes. Upon completion of the addition the mixture was stirred at ambient temperature for 2 hours and then diluted into 800 mL of water. The aqueous mixture was extracted 5 x 150 mL with ethyl acetate. The organics were combined and washed 3 x 50 mL with saturated aqueous sodium chloride, dried over anhydrous sodium sulfate, treated with decolorizing charcoal for 30 minutes at ambient temperature, filtered, and concentrated. The crude solid was triturated with a minimal volume of 9:1 heptane/ethyl acetate and filtered to afford the title compound as an off white powder (17.1 g). The filtrate was concentrated and purified by chromatography (silica gel, 20-50% of 3:1 ethyl acetate/ethanol in heptanes) to afford an additional 8.67 g of title compound. Total yield of the title compound was 25.77 g (81% yield).

### Example 14i

### tert-butyl {trans-4-[(5-bromo-1-methyl-2-oxo-1,2-dihydropyridin-4-yl)oxy]cyclohexyl} carbamate

A solution of Example 14h (9.0 g, 43.7 mmol) and *tert*-butyl(*trans-*4-hydroxycyclohexyl)carbamate (9.88 g, 45.9 mmol) in tetrahydrofuran (300 mL) at 0 °C under nitrogen was treated dropwise with potassium *tert*-butoxide (50.2 mL, 50.2 mmol, 1 M in tetrahydrofuran). The mixture was stirred for 60 minutes under nitrogen at 0-5 °C and then partitioned between ethyl acetate and water. The aqueous layer was extracted once more with ethyl acetate. The organic extracts were combined and washed with saturated aqueous sodium chloride, dried over anhydrous sodium sulfate, filtered, and concentrated to give an off-white solid. Purification of the residue by trituration in a minimal volume of 95:5 heptane/ethyl acetate afforded the title compound as a white powder (17.27 g, 43.0 mmol, 99 % yield).

### Example 14j

### 4-[(trans-4-aminocyclohexyl)oxy]-5-bromo-1-methylpyridin-2(1H)-one hydrochloride

Example 14i (17.27 g, 43.0 mmol) was treated with hydrochloric acid (215 mL, 861 mmol, 4M in dioxane), stirred for three hours at 40 °C, and then at ambient temperature overnight. The reaction mixture was diluted with heptane (200 mL) and the resulting solid was collected by vacuum filtration, washed with additional heptane, and dried overnight in a vacuum oven at 50 °C to provide the title compound (15.54 g, 46.0 mmol, quantitative yield).

### Example 14k

### N-{trans-4-[(5-bromo-1-methyl-2-oxo-1,2-dihydropyridin-4-yl)oxy]cyclohexyl}acetamide

Example 14j (0.359 g, 1.06 mmol) in dichloromethane (10.6 mL) was treated with triethylamine (0.592 mL, 4.25 mmol) and acetic anhydride (0.150 mL, 1.59 mmol). The reaction mixture was stirred at ambient temperature for 1 hour. The reaction mixture was quenched with water and extracted with ethyl acetate. The organic layer was washed with saturated aqueous sodium chloride, dried over anhydrous magnesium sulfate, filtered, and concentrated. The residue was purified by flash chromatography (silica gel, 50% (3:1 ethyl acetate:ethanol):heptanes to 100% (3:1 ethyl acetate:ethanol)) to provide the title compound as a white solid (0.0663g, 0.193 mmol, 18% yield).

### Example 14l

### N-{trans-4-[(5-{5-[(ethanesulfonyl)amino]-2-[4-(2-hydroxypropan-2-yl)-2,6-dimethylphenoxy]phenyl}-1-methyl-2-oxo-1,2-dihydropyridin-4-yl)oxy]cyclohexyl}acetamide

Example 14k (0.035 g, 0.102 mmol), Example 14e (0.060 g, 0.122 mmol), tris(dibenzylideneacetone)dipalladium(0) (2.80 mg, 3.06 µmol), 1,3,5,7-tetramethyl-6-phenyl-2,4,8-trioxa-6-phosphaadamantane (2.98 mg, 10.20 µmol) and sodium carbonate (0.043 g, 0.408 mmol) were combined and sparged with argon for 15 minutes. Meanwhile a solution of 4:1 tetrahydrofuran/water (1.0 mL) was sparged with nitrogen for 15 minutes and transferred by syringe into the reaction vessel under argon. The mixture was stirred for 18 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered and concentrated. Purification by chromatography (silica gel, 2-10% methanol in dichloromethane) afforded the title compound as a white powder (0.060 g, 89%). ¹H NMR (400 MHz, DMSO-*d*₆) δ 9.52 (s, 1H), 7.74 (d, J = 7.5 Hz, 1H), 7.62 (s, 1H), 7.17 (s, 2H), 7.06 (d, J = 2.6 Hz, 1H), 7.02 (dd, J = 8.7, 2.7 Hz, 1H), 5.98 (s, 1H), 4.92 (s, 1H), 4.37 (s, 1H), 3.45 (s, 1H), 3.38 (s, 3H), 3.00 (q, J = 7.4 Hz, 2H), 1.99 (s, 6H), 1.95 (s, 2H), 1.75 (s, 3H), 1.70 (s, 2H), 1.39 (s, 6H), 1.28 (t, J = 8.8 Hz, 4H), 1.20 (t, J = 7.3 Hz, 3H). MS (ESI-) m/z 624.0 (M-H)⁺.

### Example 15

### methyl {trans-4-[(5-{5-[(ethanesulfonyl)amino]-2-[4-(2-hydroxypropan-2-yl)-2,6-dimethylphenoxy]phenyl}-1-methyl-2-oxo-1,2-dihydropyridin-4-yl)oxy]cyclohexyl}carbamate

### Example 15a

### methyl {trans-4-[(5-bromo-1-methyl-2-oxo-1,2-dihydropyridin-4-yl)oxy]cyclohexyl}carbamate

A solution of Example 14j (0.3463 g, 1.026 mmol) in dichloromethane (10.3 mL) was treated with triethylamine (0.572 mL, 4.10 mmol) and methyl chloroformate (0.119 mL, 1.54 mmol). The reaction mixture was stirred at ambient temperature for about 4 hours, quenched with water and extracted with ethyl acetate. The organic layer was washed with saturated aqueous sodium chloride, dried over anhydrous magnesium sulfate, filtered, and concentrated. The residue was purified by flash chromatography (silica gel, 50% (3:1 ethyl acetate:ethanol):heptanes to 100% (3:1 ethyl acetate:ethanol)) to provide the title compound as a white solid (0.0722 g, 0.201 mmol, 20% yield).

### Example 15b

### methyl {trans-4-[(5-{5-[(ethanesulfonyl)amino]-2-[4-(2-hydroxypropan-2-yl)-2,6-dimethylphenoxy]phenyl}-1-methyl-2-oxo-1,2-dihydropyridin-4-yl)oxy]cyclohexyl}carbamate

Example 15a (0.035 g, 0.097 mmol), Example 14e (0.057 g, 0.117 mmol), tris(dibenzylideneacetone)dipalladium(0) (2.68 mg, 2.92 µmol), 1,3,5,7-tetramethyl-6-phenyl-2,4,8-trioxa-6-phosphaadamantane (2.85 mg, 9.74 µmol) and sodium carbonate (0.041 g, 0.390 mmol) were combined and sparged with argon for 15 minutes. Meanwhile a solution of 4:1 tetrahydrofuran/water (1.0 mL) was sparged with nitrogen for 15 minutes and transferred by syringe into the reaction vessel under argon. The mixture was stirred for 18 hours under argon at 60 °C, cooled, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 25-75% of 3:1 ethyl acetate/ethanol in heptanes) afforded the title compound (0.046 g, 69%). ¹H NMR (400 MHz, DMSO-*d*₆) δ 9.49 (m, 1H), 7.62 (s, 1H), 7.17 (s, 2H), 7.15 - 7.08 (m, 1H), 7.06 (d, J = 2.7 Hz, 1H), 7.02 (dd, J = 8.8, 2.7 Hz, 1H), 6.22 (d, J = 8.7 Hz, 1H), 5.95 (s, 1H), 4.92 (s, 1H), 4.33 (m, 1H), 3.48 (s, 3H), 3.37 (s, 3H), 3.23 (s, 1H), 3.00 (q, J = 7.3 Hz, 2H), 1.98 (d, J = 5.9 Hz, 6H), 1.94 (m, 2H), 1.72 (d, J = 8.9 Hz, 2H), 1.39 (s, 6H), 1.35 - 1.22 (m, 4H), 1.18 (t, J = 7.4 Hz, 3H). MS (ESI-) m/z 640.0 (M-H)⁺.

### Example 16

### methyl {trans-4-[(5-{5-[(ethanesulfonyl)amino]-2-(4-fluoro-2,6-dimethylphenoxy)phenyl}-1-methyl-2-oxo-1,2-dihydropyridin-4-yl)oxy]cyclohexyl} carbamate

### Example 16a

### methyl (trans-4-{[1-methyl-2-oxo-5-(4,4,5,5-tetramethyl-1,3,2-dioxaborolan-2-yl)-1,2-dihydropyridin-4-yl]oxy}cyclohexyl)carbamate

Example 15a (0.300 g, 0.835 mmol), 4,4,4',4',5,5,5',5'-octamethyl-2,2'-bi(1,3,2-dioxaborolane) (0.424 g, 1.670 mmol), tris(dibenzylideneacetone) palladium(0) (0.019 g, 0.021 mmol), 1,3,5,7-tetramethyl-6-phenyl-2,4,8-trioxa-6-phosphaadamantane (0.024 g, 0.084 mmol), and potassium acetate (0.164 g, 1.670 mmol) were sparged with nitrogen for about 1 hour. Degassed 2-methyltetrahydrofuran (8.35 mL) was added to give a purple suspension. The reaction mixture was heated to 80 °C (bath temp) for 22 hours. The reaction mixture was cooled to ambient temperature and purified by flash chromatography (silica gel, 50-100% (3:1 ethyl acetate:ethanol):heptanes) to provide the title compound as a white solid (0.174 g, 0.428 mmol, 51% yield).

### Example 16b

### 4-fluoro-2,6-dimethylphenol

A 1L three-necked round-bottomed flask equipped with a magnetic stir bar was charged with di-*tert*-butyl(2',4',6'-triisopropyl-[1,1'-biphenyl]-2-yl)phosphine (8.37 g, 19.70 mmol), tris(dibenzylideneacetone)dipalladium (4.51 g, 4.92 mmol) and potassium hydroxide (41.4 g, 739 mmol). The flask was evacuated and backfilled with nitrogen. Separately, dioxane (150 mL), 2-bromo-5-fluoro-1,3-dimethylbenzene (50 g, 246 mmol) and water (150 mL) were flow purged with nitrogen for about 30 minutes and were transferred to the reaction flask via a cannula. The reaction vessel was heated to about 100 °C and stirred overnight. The reaction mixture was cooled to ambient temperature, acidified to pH 2 by adding 6N HCl, and extracted with dichloromethane (3 x 250 mL). The combined organic layers were stirred with mercaptopropyl silica gel for about 30 minutes, dried over anhydrous magnesium sulfate, filtered, and concentrated to provide the title compound as a white solid. (31.2 g, 223 mmol, 90% yield)

### Example 16c

### 2-(2-bromo-4-nitrophenoxy)-5-fluoro-1,3-dimethylbenzene

To a solution of 3-bromo-4-fluoronitrobenzene (0.7481 g, 3.40 mmol) and Example 16b (0.512 g, 3.66 mmol) in dimethyl sulfoxide (3.40 mL) was added cesium carbonate (1.717 g, 5.27 mmol). The reaction mixture was heated at 80 °C for about 2 hours and cooled to ambient temperature. Water was added, and the reaction mixture was extracted twice with ethyl acetate. The combined organic layers were dried over sodium sulfate, filtered and concentrated. The residue was purified by flash chromatography (silica gel, 10-100% ethyl acetate:heptanes) to provide the title compound as a white solid (0.9495 g, 2.79 mmol, 82% yield).

### Example 16d

### 3-bromo-4-(4-fluoro-2,6-dimethylphenoxy)aniline

To a solution of Example 16c (0.9495 g, 2.79 mmol) in ethanol (12.7 mL) and water (1.27 mL) was added iron (0.779 g, 13.96 mmol) and ammonium chloride (0.747 g, 13.96 mmol). The reaction mixture was heated to 80 °C overnight. The reaction mixture was filtered through a 2g Celite solid-phase extraction (SPE) column and rinsed with ethyl acetate. The filtrate was made basic (pH 10) using saturated sodium bicarbonate. The filtrate was extracted 2x with ethyl acetate. The combined organic layers were washed with saturated aqueous sodium chloride, dried over anhydrous magnesium sulfate, filtered and concentrated to provide the title compound as a yellow solid (0.814 g, 2.62 mmol, 94% yield).

### Example 16e

### N-(3-bromo-4-(4-fluoro-2,6-dimethylphenoxy)phenyl)ethanesulfonamide

To a solution of Example 16d (0.814 g, 2.62 mmol) in dichloromethane (26.2 mL) was added triethylamine (1.463 mL, 10.50 mmol) and ethanesulfonyl chloride (0.746 mL, 7.87 mmol). The reaction mixture was stirred at ambient temperature for about 1 hour. The solvent was removed under reduced pressure, and dioxane (25 mL) and 2N sodium hydroxide (26.2 mL, 52.5 mmol) were added. The reaction mixture was stirred at ambient temperature overnight. The reaction mixture was partitioned between ethyl acetate and water. The aqueous layer was extracted twice with ethyl acetate. The combined organic layers were washed with saturated aqueous sodium chloride, dried over anhydrous magnesium sulfate, filtered and concentrated. The residue was purified by flash chromatography (silica gel, 10-70% (3:1 ethyl acetate:ethanol):heptanes) to provide the title compound as a white solid (0.9877 g, 2.46 mmol, 94% yield).

### Example 16f

### methyl {trans-4-[(5-{5-[(ethanesulfonyl)amino]-2-(4-fluoro-2,6-dimethylphenoxy)phenyl}-1-methyl-2-oxo-1,2-dihydropyridin-4-yl)oxy]cyclohexyl} carbamate

Example 16e (0.040 g, 0.099 mmol), Example 16a (0.0484 g, 0.119 mmol), sodium carbonate (0.037 g, 0.347 mmol), tris(dibenzylideneacetone) palladium(0) (4.55 mg, 4.96 µmol), and 1,3,5,7-tetramethyl-6-phenyl-2,4,8-trioxa-6-phosphaadamantane (4.93 mg, 0.017 mmol) were sparged with nitrogen for about 30 minutes. Degassed tetrahydrofuran (0.794 mL) and water (0.199 mL) were added. The reaction mixture was heated at 50 °C for about 4 hours. The reaction mixture was cooled to ambient temperature and partitioned between ethyl acetate and water. The organic layer was washed with saturated aqueous sodium chloride, dried over anhydrous magnesium sulfate, filtered and concentrated. The residue was purified by flash chromatography (silica gel, 20-80% (3:1 ethyl acetate:ethanol):heptanes) to provide the title compound as an off white solid (0.0363 g, 0.060 mmol, 61% yield). ¹H NMR(501 MHz, DMSO-*d₆*) δ 9.55 (s, 1H), 7.62 (s, 1H), 7.11 (d, J = 7.5 Hz, 1H), 7.06 (d, J = 2.7 Hz, 1H), 7.03 (dd, J = 8.8, 2.8 Hz, 1H), 6.97 (d, J = 9.1 Hz, 2H), 6.26 (d, J = 8.8 Hz, 1H), 5.96 (s, 1H), 3.48 (s, 3H), 3.38 (s, 3H), 3.23 (s, 1H), 3.00 (q, J = 7.4 Hz, 2H), 1.99 (s, 8H), 1.95 (d, J = 10.7 Hz, 2H), 1.71 (d, J = 10.8 Hz, 2H), 1.29 (td, J = 23.1, 10.5 Hz, 4H), 1.18 (t, J = 7.4 Hz, 3H). MS (ESI-) m/z 602.0 (M+H)⁺.

### Example 17

### methyl [trans-4-({5'-[(ethanesulfonyl)amino]-2'-(4-fluoro-2,6-dimethylphenoxy)-1-methyl-6-oxo[1,6-dihydro[3,3'-bipyridine]]-4-yl}oxy)cyclohexyl]carbamate

### Example 17a

### 3-bromo-2-(4-fluoro-2,6-dimethylphenoxy)-5-nitropyridine

Example 17a was prepared according to the procedure used for the preparation of Example 16c, substituting 3-bromo-2-chloro-5-nitropyridine for 3-bromo-4-fluoronitrobenzene, to provide the title compound as a white solid (0.8688 g, 2.55 mmol, 81% yield).

### Example 17b

### 5-bromo-6-(4-fluoro-2,6-dimethylphenoxy)pyridin-3-amine

Example 17b was prepared according to the procedure used for the preparation of Example 16d, substituting Example 17a for Example 16c, to provide the title compound as a yellow solid (0.7928 g, 2.55 mmol, 100% yield).

### Example 17c

### N-(5-bromo-6-(4-fluoro-2,6-dimethylphenoxy)pyridin-3-yl)ethanesulfonamide

Example 17c was prepared according to the procedure used for the preparation of Example 16e, substituting Example 17b for Example 16d, to provide the title compound as a yellow solid (0.8011 g, 1.99 mmol, 78% yield).

### Example 17d

### methyl [trans-4-({5'-[(ethanesulfonyl)amino]-2'-(4-fluoro-2,6-dimethylphenoxy)-1-methyl-6-oxo[1,6-dihydro[3,3'-bipyridine]]-4-yl}oxy)cyclohexyl]carbamate

Example 17d was prepared according to the procedure used for the preparation of Example 16f, substituting Example 17c for Example 16e, to provide the title compound as an off white solid (0.0246 g, 0.041 mmol, 41% yield). ¹H NMR (400 MHz, DMSO-*d₆*) δ 9.74 (s, 1H), 7.80 (d, J = 2.6 Hz, 1H), 7.72 (s, 1H), 7.50 (d, J = 2.7 Hz, 1H), 7.09 (d, J = 7.5 Hz, 1H), 6.89 (d, J = 9.1 Hz, 2H), 5.98 (s, 1H), 4.38 - 4.31 (m, 1H), 3.46 (s, 3H), 3.37 (s, 3H), 3.20 (d, J = 10.4 Hz, 1H), 3.06 (q, J = 7.3 Hz, 2H), 1.95 (s, 8H), 1.70 (d, J = 11.0 Hz, 2H), 1.37 - 1.21 (m, 4H), 1.18 (t, J = 7.3 Hz, 3H). MS (ESI-) m/z 603.0 (M+H)⁺.

### Example 18

### methyl {trans-4-[(5-{2-(4-fluoro-2,6-dimethylphenoxy)-5-[(methanesulfonyl)methyl]phenyl}-1-methyl-2-oxo-1,2-dihydropyridin-4-yl)oxy]cyclohexyl}carbamate

### Example 18a

### methyl 3-bromo-4-(4-fluoro-2,6-dimethylphenoxy)benzoate

Example 18a was prepared according to the procedure used for the preparation of Example 16c, substituting methyl 3-bromo-4-fluorobenzoate for 3-bromo-4-fluoronitrobenzene, to provide the title compound as a white solid (10.5 g, 29.7 mmol, 99% yield).

### Example 18b

### (3-bromo-4-(4-fluoro-2,6-dimethylphenoxy)phenyl)methanol

To a solution of Example 18a (9.45 g, 26.8 mmol) in tetrahydrofuran (80 mL) at 0 °C was added diisobutylaluminum hydride in tetrahydrofuran (1.0 M, 107 mL, 107 mmol) dropwise. The reaction mixture was stirred at room temperature for about 3 hours, quenched with saturated potassium sodium tartrate, and stirred vigorously overnight. The reaction mixture was extracted twice with ethyl acetate. The combined organic layers were washed with saturated aqueous sodium chloride, dried over sodium sulfate, filtered and concentrated. The residue was purified by flash chromatography (silica gel, 20-40% ethyl acetate in heptanes) to provide the title compound as a white solid (8.01 g, 24.6 mmol, 92% yield).

### Example 18c

### 2-(2-bromo-4-(bromomethyl)phenoxy)-5-fluoro-1,3-dimethylbenzene

To a solution of Example 18b (7.00 g, 21.5 mmol) in dichloromethane (50 mL) was added phosphorus tribromide in dichloromethane (1.0 M, 23.7 mL, 23.7 mmol). The reaction mixture was stirred at room temperature for about 2 hours and poured into ice-water. The pH was adjusted to about 8 by addition of saturated aqueous sodium bicarbonate. The reaction mixture was extracted twice with dichloromethane. The combined organic layers were washed with saturated aqueous sodium chloride, dried over anhydrous sodium sulfate, filtered, and concentrated to give the title compound as a white solid (7.10 g, 18.3 mmol, 85% yield).

### Example 18d

### 2-(2-bromo-4-((methylsulfonyl)methyl)phenoxy)-5-fluoro-1,3-dimethylbenzene

To a solution of Example 18c (0.5010 g, 1.291 mmol) in *N*,*N-*dimethylformamide (12.9 mL) was added sodium methanesulfinate (0.198 g, 1.937 mmol). The reaction mixture was stirred at 65 °C for three hours. The reaction mixture was cooled to ambient temperature and diluted with water. The resulting suspension was filtered, and the solids were rinsed with water and dried in a 60 °C vacuum oven overnight to provide the title compound as a white solid (0.4673 g, 1.21 mmol, 93% yield).

### Example 18e

### methyl{trans-4-[(5-{2-(4-fluoro-2,6-dimethylphenoxy)-5-[(methanesulfonyl)methyl]phenyl}-1-methyl-2-oxo-1,2-dihydropyridin-4-yl)oxy]cyclohexyl}carbamate

Example 18e was prepared according to the procedure used for the preparation of Example 16f, substituting Example 18d for Example 16e, to provide the title compound as an off white solid (0.0223 g, 0.038 mmol, 51% yield). ¹H NMR (400 MHz, DMSO-*d₆*) δ 7.62 (s, 1H), 7.26 (d, J = 2.2 Hz, 1H), 7.21 (dd, J = 8.5, 2.3 Hz, 1H), 7.09 (d, J = 7.5 Hz, 1H), 6.99 (d, J = 9.1 Hz, 2H), 6.31 (d, J = 8.4 Hz, 1H), 5.96 (s, 1H), 4.39 (s, 2H), 3.48 (d, J = 5.7 Hz, 4H), 3.38 (s, 3H), 3.20 (s, 1H), 2.86 (s, 3H), 2.00 (s, 6H), 1.94 (d, J = 10.3 Hz, 2H), 1.71 (d, J = 10.4 Hz, 2H), 1.30 (dq, J = 21.8, 12.6, 11.2 Hz, 4H). MS (ESI+) m/z 587.0 (M+H)⁺.

### Example 19

### 5-[5-(ethanesulfonyl)-2-(4-fluoro-2,6-dimethylphenoxy)phenyl]-1-methyl-4-{[trans-4-(2-oxopyrrolidin-1-yl)cyclohexyl]oxy}pyridin-2(1H)-one

### Example 19a

### 5-bromo-1-methyl-4-{[trans-4-(2-oxopyrrolidin-1-yl)cyclohexyl]oxy}pyridin-2(1H)-one

To a solution of Example 14j (0.200 g, 0.592 mmol) and triethylamine (0.248 mL, 1.78 mmol) in dichloromethane (5.9 mL) was added dropwise 4-chlorobutyryl chloride (0.092 g, 0.65 mmol). The mixture was stirred at ambient temperature under nitrogen for about 2 hours and partitioned with water. The organic layer was dried over anhydrous sodium sulfate, filtered and concentrated. The residue was dissolved in tetrahydrofuran (5.9 mL), treated with sodium hydride (0.090 g, 3.6 mmol) and heated at 60 °C for about 18 hours. The mixture was cooled and partitioned between ethyl acetate and water. The aqueous layer was extracted twice with ethyl acetate. The combined organic layers were dried over anhydrous sodium sulfate, filtered and concentrated. The residue was purified by flash chromatography (silica gel, 1-8% methanol in dichloromethane) to provide the title compound as a white solid (0.17 g, 0.45 mmol, 76%).

### Example 19b

### ethyl(4-fluorophenyl)sulfane

Triethylamine (5.44 mL, 39 mmol) was added to a solution of 4-fluorobenzenethiol (5 g, 39 mmol) and iodoethane (3.78 mL, 46.8 mmol) in tetrahydrofuran (50 mL). The resulting mixture was stirred at ambient temperature for 2 hours and then filtered. The filtrate was concentrated. The residue was triturated with hexane, and dried under vacuum to afford the title compound (4.8 g, 76%).

### Example 19c

### 1-(ethylsulfonyl)-4-fluorobenzene

Example 19b (5 g, 32 mmol) in dichloromethane (200 mL) was treated with 3-chloroperoxybenzoic acid (14.3 g, 70.4 mmol) and stirred at ambient temperature for 6 hours. The solid formed during the reaction mixture was removed by filtration and washed with additional dichloromethane. The combined filtrate was washed with 10% aqueous sodium hydroxide solution (50 mL, twice) and saturated aqueous sodium bicarbonate solution, dried over anhydrous sodium sulfate, filtered, and concentrated. The residue was purified by flash chromatography (silica gel, 15% ethyl acetate in petroleum ether) to afford the title compound (4.6 g, 76%).

### Example 19d

### 2-bromo-4-(ethylsulfonyl)-1-fluorobenzene

Example 19c (1 g, 5.31 mmol) in sulfuric acid (6 mL, 113 mmol) was treated with N-bromosuccinimide (1.04 g, 5.84 mmol), stirred at ambient temperature for 6 hours and then at 50 °C for 16 hours. The reaction mixture was then poured into ice water and the resulting solid was collected by filtration, washed with cold water three times, and dried in a vacuum oven for 16 hours. The solid was then purified by flash chromatography (silica gel, 9-20% ethyl acetate in petroleum ether) to afford the title compound (1.1 g, 78%).

### Example 19e

### 2-(5-(ethylsulfonyl)-2-fluorophenyl)-4,4,5,5-tetramethyl-1,3,2-dioxaborolane

4,4,4',4',5,5,5',5'-octamethyl-2,2'-bi(1,3,2-dioxaborolane) (0.665 g, 2.62 mmol), Example 19d (0.5 g, 1.9 mmol), potassium acetate (0.367 g, 3.74 mmol) and [1,1'-bis(diphenylphosphino)ferrocene]dichloropalladium(II) (0.041 g, 0.056 mmol) were combined in an argon-sparged mixture of dioxane (10 mL)/dimethyl sulfoxide (0.3 mL) and heated at 90 °C under argon for 24 hours. The reaction mixture was partitioned between ethyl acetate and water and filtered through a plug of diatomaceous earth to remove elemental palladium. The layers were separated and the organic layer was washed with saturated aqueous sodium chloride, dried over anhydrous sodium sulfate, treated with 3-mercaptopropyl-functionalized silica gel for 15 minutes, filtered, and concentrated. The residue was triturated in a minimal amount of heptane/diethyl ether (20:1) and filtered to give crude product. This material was then dissolved in ethyl acetate, treated again with 3-mercaptopropyl-functionalized silica gel, filtered, and concentrated. The residue was taken into heptane/ethyl acetate (9:1) and the solid formed was collected to afford the title compound (0.3 g, 77%).

### Example 19f

### 5-[5-(ethanesulfonyl)-2-fluorophenyl]-1-methyl-4-{[trans-4-(2-oxopyrrolidin-1-yl)cyclohexyl]oxy}pyridin-2(1H)-one

Example 19a (0.0383, 0.104 mmol), Example 19e (0.039 g, 0.124 mmol), sodium carbonate (0.038 g, 0.363 mmol), tris(dibenzylideneacetone) palladium(0) (4.75 mg, 5.19 µmol), and 1,3,5,7-tetramethyl-6-phenyl-2,4,8-trioxa-6-phosphaadamantane (5.15 mg, 0.018 mmol) were sparged with nitrogen for about 30 minutes. Degassed tetrahydrofuran (0.830 mL) and water (0.207 mL) were added. The reaction mixture was heated at 50 °C for about 4 hours. The reaction mixture was cooled to ambient temperature and partitioned between ethyl acetate and water. The organic layer was washed with saturated aqueous sodium chloride, dried over anhydrous magnesium sulfate, filtered and concentrated. The residue was purified by flash chromatography (silica gel, 50-100% (3:1 ethyl acetate:ethanol):heptanes) to provide the title compound as an off white solid (0.0319 g, 0.067 mmol, 64% yield).

### Example 19g

### 5-[5-(ethanesulfonyl)-2-(4-fluoro-2,6-dimethylphenoxy)phenyl]-1-methyl-4-{[trans-4-(2-oxopyrrolidin-1-yl)cyclohexyl]oxy}pyridin-2(1H)-one

To a solution of Example 19f (0.0319 g, 0.067 mmol) and Example 16b (10.09 mg, 0.072 mmol) in dimethyl sulfoxide (1.0 mL) was added cesium carbonate (0.034 g, 0.104 mmol). The reaction mixture was heated at 80 °C for about 2 hours and cooled to ambient temperature. Water was added, and the reaction mixture was extracted twice with ethyl acetate. The combined organic layers were dried over anhydrous sodium sulfate, filtered, and concentrated. The residue was purified by flash chromatography (30-100% (3:1 ethyl acetate:ethanol):heptanes) to provide the title compound as a white solid (0.0156 g, 0.026 mmol, 39%). ¹H NMR(501 MHz, DMSO-*d*₆) δ 7.75 - 7.67 (m, 3H), 7.04 (d, J = 9.0 Hz, 2H), 6.53 (d, J = 8.6 Hz, 1H), 6.01 (s, 1H), 3.25 (td, J = 7.2, 5.8 Hz, 4H), 2.17 (t, J = 8.1 Hz, 2H), 2.04 (d, J = 4.0 Hz, 1H), 2.02 (s, 6H), 1.92 - 1.82 (m, 2H), 1.69 - 1.58 (m, 2H), 1.53 (d, J = 12.0 Hz, 2H), 1.28 - 1.17 (m, 4H), 1.08 (t, J = 7.3 Hz, 3H). MS (ESI+) m/z 596.9 (M+H)⁺.

### Example 20

### N-[trans-4-({5-[5-(ethanesulfonyl)-2-(4-fluoro-2,6-dimethylphenoxy)phenyl]-1-methyl-2-oxo-1,2-dihydropyridin-4-yl}oxy)cyclohexyl]-N-methylacetamide

### Example 20a

### tert-butyl {trans-4-[(5-bromo-1-methyl-2-oxo-1,2-dihydropyridin-4-yl)oxy]cyclohexyl}methylcarbamate

To a solution of tert-butyl (*trans*-4-hydroxycyclohexyl) (0.935 g, 4.08 mmol) in *N,N-*dimethyl formamide (19.42 mL) was added sodium hydride (0.349 g, 8.74 mmol, 60% in mineral oil). The reaction mixture was stirred for about 30 minutes and then treated with Example 14h (0.6 g, 2.91 mmol). The reaction mixture was stirred for about 2 hours under nitrogen at 50 °C, cooled, and partitioned between ethyl acetate and water. The aqueous layer was extracted 3 x 100 mL with ethyl acetate. The combined organic layers were washed 3 x 25 mL with saturated aqueous sodium chloride, dried over anhydrous sodium sulfate, filtered and concentrated. Purification of the residue by trituration in 95/5 heptane/ethyl acetate afforded the title compound as a white solid (1.034 g, 2.49 mmol, 85% yield).

### Example 20b

### 5-bromo-1-methyl-4-{[trans-4-(methylamino)cyclohexyl]oxy}pyridin-2(1H)-one

Example 20a (1.0 g, 2.408 mmol) was treated with hydrochloric acid (12.04 mL, 48.2 mmol, 4 N in dioxane). The reaction mixture was stirred for about three hours at ambient temperature and concentrated. The residue was azeotroped twice with toluene and dried to constant mass to provide the title compound as the HCl salt (1.14 g, 2.4 mmol, quantitative).

### Example 20c

### N-{trans-4-[(5-bromo-1-methyl-2-oxo-1,2-dihydropyridin-4-yl)oxy]cyclohexyl}-N-methylacetamide

To a solution of Example 20b (0.300 g, 0.853 mmol) and triethylamine (0.357 mL, 2.56 mmol) in dichloromethane (8.5 mL) was added dropwise acetyl chloride (0.079 mL, 1.109 mmol). The mixture was stirred at ambient temperature under nitrogen for about 4 hours, diluted with water, and stirred for 10 minutes. The layers were separated, and the organic layer was dried over anhydrous sodium sulfate, filtered and concentrated. The residue was purified by flash chromatography (20-90% (3:1ethyl acetate:ethanol):heptanes) to provide the title compound as a white foam (0.185 g, 0.487 mmol, 57%).

### Example 20d

### N-[trans-4-({5-[5-(ethanesulfonyl)-2-fluorophenyl]-1-methyl-2-oxo-1,2-dihydropyridin-4-yl}oxy)cyclohexyl]-N-methylacetamide

Example 20d was prepared according to the procedure used for the preparation of Example 19f, substituting Example 20c for Example 19a, to provide the title compound as an off white solid (0.0336 g, 0.072 mmol, 60% yield).

### Example 20e

### N-[trans-4-({5-[5-(ethanesulfonyl)-2-(4-fluoro-2,6-dimethylphenoxy)phenyl]-1-methyl-2-oxo-1,2-dihydropyridin-4-yl}oxy)cyclohexyl]-N-methylacetamide

Example 20e was prepared according to the procedure used for the preparation of Example 19g, substituting Example 20d for Example 19f, to provide the title compound as an off white solid (0.0173 g, 0.030 mmol, 41% yield). ¹H NMR (400 MHz, DMSO-*d₆*) δ 7.74 - 7.64 (m, 3H), 7.02 (d, J = 9.2 Hz, 2H), 6.52 (d, J = 8.6 Hz, 1H), 6.00 (d, J = 9.2 Hz, 1H), 4.35 (d, J = 12.4 Hz, 1H), 3.71 (s, 3H), 3.38 (s, 3H), 3.23 (q, J = 7.4 Hz, 2H), 2.04 - 1.88 (m, 9H), 1.80 - 1.50 (m, 3H), 1.47 - 1.39 (m, 1H), 1.35 - 1.09 (m, 3H), 1.07 (td, J = 7.3, 2.5 Hz, 3H). MS (ESI+) m/z 584.9 (M+H)⁺.

### g. Biological Examples

### Bromodomain domain binding assay

A time-resolved fluorescence resonance energy transfer (TR-FRET) assay was used to determine the affinities of compounds of the Examples listed in Table 1 for each bromodomain of BRD4. His-tagged first (BDI: amino acids K57-E168) and second (BDII: amino acids E352- M457) bromodomains of BRD4 were expressed and purified. An Alexa647-labeled BET-inhibitor was used as the fluorescent probe in the assay.

### Synthesis of Alexa647-labeled bromodomain inhibitor compound 2-((6S,Z)-4-(4-chlorophenyl)-2,3,9-trimethyl-6H-thieno[3,2-f][1,2,4]triazolo[4,3-a][1,4]diazepin-6-yl)acetic acid

Methyl 2-((6S,Z)-4-(4-chlorophenyl)-2,3,9-trimethyl-6H-thieno[3,2-f][1,2,4]triazolo[4,3-a][1,4]diazepin-6-yl)acetate (see e.g., WO 2006129623)(100.95 mg, 0.243 mmol was suspended in 1 mL methanol to which was added a freshly prepared solution of lithium hydroxide monohydrate (0.973 mL, 0.5 M, 0.487 mmol) and shaken at ambient temperature for 3 hours. The methanol was evaporated and the pH adjusted with aqueous hydrochloric acid (1 M, 0.5 mL, 0.5 mmol) and extracted four times with ethyl acetate. The combined ethyl acetate layers were dried over magnesium sulfate and evaporated to afford 2-((6S,Z)-4-(4-chlorophenyl)-2,3,9-trimethyl-6H-thieno[3,2-f][1,2,4]triazolo[4,3-a][1,4]diazepin-6-yl)acetic acid (85.3 mg, 87.0%); ESI-MS m/z = 401.1 [(M+H)⁺] which was used directly in the next reaction.

### N-(2-(2-(2-aminoethoxy)ethoxy)ethyl)-2-((6S,Z)-4-(4-chlorophenyl)-2,3,9-trimethyl-6H-thieno[3,2-f][1,2,4]triazolo[4,3-a][1,4]diazepin-6-yl)acetamide bis(2,2,2-trifluoroacetate)

2-((6S,Z)-4-(4-chlorophenyl)-2,3,9-trimethyl-6H-thieno[3,2-f][1,2,4]triazolo[4,3-a][1,4]diazepin-6-yl)acetic acid)(85.3 mg, 0.213 mmol) was combined with 2,2'-(ethane-1,2-diylbis(oxy))diethanamine (Sigma-Aldrich, 0.315 mg, 2.13 mmol) were combined in 5 mL anhydrous dimethylformamide. (1*H*-benzo[d][1,2,3]triazol-1-yloxy)tripyrrolidin-1-ylphosphonium hexafluorophosphate(V) (PyBOB, CSBio, Menlo Park CA; 332 mg, 0.638 mmol) was added and the reaction shaken at ambient temperature for 16 hours. The reaction was diluted to 6 mL with dimethylsulfoxide:water (9:1, v:v) and purified in two injections with time collection Waters Deltapak C18 200 x 25 mm column eluted with a gradient of 0.1% trifluoroacetic acid (v/v) in water and acetonitrile. The fractions containing the two purified products were lyophilized to afford *N*-(2-(2-(2-aminoethoxy)ethoxy)ethyl)-2-((6S,Z)-4-(4-chlorophenyl)-2,3,9-trimethyl-6*H*-thieno[3,2-f][1,2,4]triazolo[4,3-a][1,4]diazepin-6-yl)acetamide bis(2,2,2-trifluoroacetate) (134.4 mg, 82.3%); ESI-MS m/z = 531.1 [(M+H)⁺]; 529.1 [(M-H)⁻] and (*S,*Z)-*N,N'*-(2,2'-(ethane-1,2-diylbis(oxy))bis(ethane-2,1-diyl))bis(2-((6*S*,Z)-4-(4-chlorophenyl)-2,3,9-trimethyl-6*H*-thieno[3,2-f][1,2,4]triazolo[4,3-a][1,4]diazepin-6-yl)acetamide) bis(2,2,2-trifluoroacetate) (3.0 mg, 1.5%); ESI-MS m/z = 913.2 [(M+H)⁺]; 911.0 [(M-H)⁻].

### N-(2-(2-(2-amido-(Alexa647)-ethoxy)ethoxy)ethyl)-2-((6S,Z)-4-(4-chlorophenyl)-2,3,9-trimethyl-6H-thieno[3,2-f)[1,2,4]triazolo[4,3-a][1,4]diazepin-6-yl)acetamide(2,2,2-trifluoroacetate)

*N*-(2-(2-(2-aminoethoxy)ethoxy)ethyl)-2-((6*S,*Z)-4-(4-chlorophenyl)-2,3,9-trimethyl-6*H*-thieno[3,2-f][1,2,4]triazolo[4,3-a][1,4]diazepin-6-yl)acetamide bis(2,2,2-trifluoroacetate) (5.4 mg, 0.0071 mmol) was combined with Alexa Fluor® 647 carboxylic Acid, succinimidyl ester (Life Technologies, Grand Island, NY; 3 mg, 0.0024 mmol) were combined in 1 mL anhydrous dimethylsulfoxide containing diisopropylethylamine (1% v/v) and shaken at ambient temperature for 16 hours. The reaction was diluted to 3 mL with dimethylsulfoxide:water (9:1, v:v) and purified in one injection with time collection Waters Deltapak C18 200 x 25 mm column eluted with a gradient of 0.1% trifluoroacetic acid (v/v) in water and acetonitrile. The fractions containing the purified product were lyophilized to afford *N*-(2-(2-(2-amido-(Alexa647)-ethoxy)ethoxy)ethyl)-2-((6*S*,Z)-4-(4-chlorophenyl)-2,3,9-trimethyl-6*H*-thieno[3,2-f][1,2,4]triazolo[4,3-a][1,4]diazepin-6-yl)acetamide(2,2,2-trifluoroacetate) (1.8 mg); MALDI-MS m/z = 1371.1, 1373.1 [(M+H)⁺] as a dark blue powder.

### Assay

Compound dilution series were prepared in DMSO via an approximately 3-fold serial dilution. Compound dilutions were added directly into white, low-volume assay plates (Perkin Elmer Proxiplate 384 Plus# 6008280) using a Labcyte Echo in conjunction with Labcyte Access and Thermo Multidrop CombinL robotics. Compounds were then suspended in eight microliters (µL) of assay buffer (20 mM Sodium Phosphate, pH 6.0, 50 mM NaCl, 1 mM Ethylenediaminetetraacetic acid disodium salt dihydrate, 0.01% Triton X-100, 1 mM DL-Dithiothreitol) containing His-tagged bromodomain, Europium-conjugated anti-His antibody (Invitrogen PV5596) and Alexa-647-conjugated probe.

The final concentration of IX assay mixture contained 2% DMSO, 12 nM His tagged BRD4 (BDI_K57-E168) and 100 nM probe or 4 nM His tagged BRD4 (BDII E352-M457) and 30 nM probe, and 1 nM Europium-conjugated anti-His-tag antibody, and compound concentrations in the range of: 49.02 µM-0.61 nM or 0.98 µM - 0.15 nM.

After a one-hour equilibration at room temperature, TR-FRET ratios were determined using an Envision multilabel plate reader (Ex 340, Em 495/520).

TR-FRET data were normalized to the means of 24 no-compound controls ("high") and 8 controls containing 1 µM un-labeled probe ("low"). Percent inhibition was plotted as a function of compound concentration and the data were fit with the 4 parameter logistic equation to obtain IC50s. Inhibition constants (Kᵢ) were calculated from the IC₅₀s, probe K_{d} and probe concentration.

The mean Kᵢ values are reported in Table 1.

**Table 1**

| Example # | TR-FRET Binding Ki: BRD4 (BDI_ K57-E168) (µM) | TR-FRET Binding Ki: BRD4 (BDII_ E352-M457) (µM) |
|---|---|---|
| 1 | 1.16 | 0.00255 |
| 2 | 2.8 | 0.0227 |
| 3 | 1.05 | 0.00313 |
| 4 | 1.05 | 0.0104 |
| 5 | 1.28 | 0.0135 |
| 6 | 0.823 | 0.00153 |
| 7 | 0.489 | 0.00967 |
| 8 | 0.627 | 0.0257 |
| 9 | 0.474 | 0.00508 |
| 10 | 0.524 | 0.0418 |
| 11 | 0.773 | 0.0125 |
| 12 | 0.301 | 0.00226 |
| 13 | 0.462 | 0.00306 |
| 14 | 0.0953 | 0.000504 |
| 15 | 0.102 | 0.000508 |
| 16 | 0.313 | 0.00137 |
| 17 | 0.419 | 0.00175 |
| 18 | 0.903 | 0.0151 |
| 19 | 0.615 | 0.00291 |
| 20 | 0.461 | 0.0033 |

All tested compounds were found to have selectivity for BRD4 BDII over BRD4 BDI in the TR-FRET assay described above, and are at least 10 fold selective for BRD4 BDII over BRD4 BDI. In one embodiment, the present compounds are about 50 to about 100 fold selective for BRD4 BDII over BRD4 BDI. In one embodiment, the present compounds are about 100 to about 200 fold selective for BRD4 BDII over BRD4 BDI. In one embodiment, the present compounds are at least about 200 fold selective for BRD4 BDII over BRD4 BDI.

Compound dilution series for Compounds A-E were prepared in DMSO via an approximately 3-fold serial dilution from 0.47mM to 7.8nM.

Compound dilutions were added directly into white, low-volume assay plates (Perkin Elmer Proxiplate 384 Plus# 6008280) using a Labcyte Echo in conjunction with Labcyte Access and Thermo Multidrop CombinL robotics. Compounds were then suspended in eight microliters (µL) of assay buffer (20 mM Sodium Phosphate, pH 6.0, 50 mM NaCl, 1 mM Ethylenediaminetetraacetic acid disodium salt dihydrate, 0.01% Triton X-100, 1 mM DL-Dithiothreitol) containing His-tagged bromodomain, Europium-conjugated anti-His antibody (Invitrogen PV5596) and Alexa-647-conjugated probe.

The final concentration of IX assay mixture contained 2% DMSO, 8 nM His-tagged bromodomain, 1 nM Europium-conjugated anti-His-tag antibody and 100 nM or 30 nM probe (for BDI or BDII, respectively) and compound concentration in the range of 9.19 µM - 150 pM.

After a one-hour equilibration at room temperature, TR-FRET ratios were determined using an Envision multilabel plate reader (Ex 340, Em 495/520).

TR-FRET data were normalized to the means of 24 no-compound controls ("high") and 8 controls containing 1 µM un-labeled probe ("low"). Percent inhibition was plotted as a function of compound concentration and the data were fit with the 4 parameter logistic equation to obtain IC₅₀s. Inhibition constants (Kᵢ) were calculated from the IC₅₀s, probe K_{d} and probe concentration. Typical Z' values were between 0.65 and 0.75. The minimum significant ratio was determined to evaluate assay reproducibility (Eastwood et al., (2006) J Biomol Screen, 11: 253-261). The MSR was determined to be 2.03 for BDI and 1.93 for BDII, and a moving MSR (last six run MSR overtime) for both BDI and BDII was typically < 3. The Kᵢ values are reported in Table 2.

**Table 2**

| Compounds | TR-FRET Binding Ki: BRD4 (BDI_ K57-E168) (µM) | TR-FRET Binding Ki: BRD4 (BDII_ E352-M457) (µM) |
|---|---|---|
| A | 0.316 | 0.104 |
| B | 0.05 | 0.00915 |
| C | 0.437 | 0.0909 |
| D | 0.045 | 0.0181 |
| E | 0.0306 | 0.0214 |

Compound A is *tert*-butyl 4-[(5-{2-(2,4-difluorophenoxy)-5-[(ethylsulfonyl)amino]phenyl}-1-methyl-2-oxo-1,2-dihydropyridin-4-yl)oxy]piperidine-1-carboxylate
Compound B is *N*-[4-(2,4-difluorophenoxy)-3-(4-{*[trans*-4-(dimethylamino)cyclohexyl]oxy}-1-methyl-6-oxo-1,6-dihydropyridin-3-yl)phenyl]ethanesulfonamide
Compound C is *N*-{4-(2,4-difluorophenoxy)-3-[1-methyl-6-oxo-4-(piperidin-4-yloxy)-1,6-dihydropyridin-3-yl]phenyl}ethanesulfonamide
Compound D is *tert*-butyl 4-{[(5-{2-(2,4-difluorophenoxy)-5-[(ethylsulfonyl)amino]phenyl}-1-methyl-2-oxo-1,2-dihydropyridin-4-yl)oxy]methyl}piperidine-1-carboxylate
Compound E is *tert*-butyl 6-[(5-{2-(2,4-difluorophenoxy)-5-[(ethylsulfonyl)amino]phenyl}-1-methyl-2-oxo-1,2-dihydropyridin-4-yl)oxy]-2-azaspiro [3.3]heptane-2-carboxylate

It is understood that the foregoing detailed description and accompanying examples are merely illustrative and are not to be taken as limitations upon the scope of the invention, which is defined solely by the appended claims and their equivalents. Various changes and modifications to the disclosed embodiments will be apparent to those skilled in the art. Such changes and modifications, including without limitation those relating to the chemical structures, substituents, derivatives, intermediates, syntheses, formulations and/or methods of use of the invention, may be made without departing from the spirit and scope thereof. All publications, patents, and patent applications cited herein are hereby incorporated by reference in their entirety for all purposes.

## Claims

1. A compound of formula (I) or a pharmaceutically acceptable salt thereof, 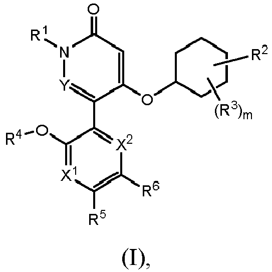 wherein
R¹ is C₁-C₃ alkyl;
Y is N or C(R^{Y}) wherein R^{Y} is hydrogen or C₁-C₃ alkyl;
m is 0, 1, 2, 3, or 4;
R² is G^{1A}, -N(R^{a})S(O)2R^{b}, -N(R^{a})C(O)R^{b}, or -N(R^{a})C(O)OR^{b};
R^{a}, at each occurrence, is independently hydrogen, C₁-C₆ alkyl, C₁-C₆ haloalkyl, or -(C₂-C₆ alkylenyl)-OR^{x} wherein R^{x} is hydrogen, C₁-C₆ alkyl, or C₁-C₆ haloalkyl;
R^{b}, at each occurrence, is independently C₁-C₆ alkyl, C₂-C₆ alkenyl, C₁-C₆ haloalkyl, G^{1B}, or -(C₁-C₆ alkylenyl)-OR^{j};
G^{1A}. is formula (i) 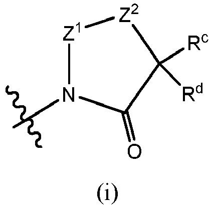 wherein
Z¹ is C(O) or C(R^{e})(R^{f});
Z² is a bond, O, -(C(R^{g})(R^{h}))ₚ-C(R^{m})(Rⁿ)- wherein the left end of the moiety is attached to Z¹, or N(Rⁱ) wherein Rⁱ is hydrogen, C₁-C₃ alkyl, or cyclopropyl; wherein the cyclopropyl is optionally substituted with 1, 2, 3, or 4 independently selected R^{s} groups;
p is 0 or 1;
R^{c}, R^{d}, R^{e}, R^{f}, R^{g}, R^{h}, R^{m}, and Rⁿ, are each independently hydrogen, C₁-C₆ alkyl, C₁-C₆ haloalkyl, or cyclopropyl; wherein the cyclopropyl, at each occurrence, is optionally substituted with 1, 2, 3, or 4 independently selected R^{s} groups; or
R^{c} and R^{m} together with the carbon atoms to which they are attached, form a C₃-C₆ monocyclic cycloalkyl or a 4-6 membered monocyclic heterocycle; wherein the C₃-C₆ monocyclic cycloalkyl and the 4-6 membered monocyclic heterocycle are each optionally substituted with 1, 2, 3, or 4 independently selected R^{s} groups; or
R^{c} and Rⁱ together with the atoms to which they are attached, form a 4-6 membered monocyclic heterocycle; wherein the 4-6 membered monocyclic heterocycle is optionally substituted with 1, 2, 3, or 4 independently selected R^{s} groups; or
R^{c} and R^{d} together with the carbon atoms to which they are attached, form a C₃-C₆ monocyclic cycloalkyl or a 4-6 membered monocyclic heterocycle; wherein the C₃-C₆ monocyclic cycloalkyl and the 4-6 membered monocyclic heterocycle are each optionally substituted with 1, 2, 3, or 4 independently selected R^{s} groups;
G^{1B} is oxetanyl, cyclopropyl, cyclobutyl, or cyclopentyl; wherein each G^{1B} is optionally substituted with 1, 2, 3, or 4 R^{t} groups; wherein each R^{t} is independently halogen, C₁-C₆ alkyl, or C₁-C₆ haloalkyl;
each R³ is independently C₁-C₃ alkyl, C₁-C₃ haloalkyl, halogen, -CN, -OR^{3a}, -N(R^{3a})₂, -C(O)R^{3a}, cyclopropyl, or cyclobutyl; wherein each R^{3a} is independently hydrogen, C₁-C₃ alkyl, or C₁-C₃ haloalkyl;
R⁴ is phenyl or monocyclic heteroaryl; wherein each R⁴ is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH;
R⁵ is hydrogen, halogen, -CN, C₁-C₆ haloalkyl, or C₁-C₆ alkyl;
R⁶ is -(C₁-C₃ alkylenyl)ₙ-S(O)2-R^{6a} or -N(R^{6b})-SO2-R^{6c};
n is 0 or 1;
R^{6a} and R^{6c} are each independently C₁-C₆ alkyl, C₁-C₆ haloalkyl, or cyclopropyl; wherein the cyclopropyl is optionally substituted with 1, 2, 3, or 4 substituents independently selected from the group consisting of halogen, C₁-C₆ alkyl, and C₁-C₆ haloalkyl;
R^{6b} is hydrogen, C₁-C₆ alkyl, or C₁-C₆ haloalkyl;
X¹ and X² are C(R⁷) or
one of X¹ and X² is N and the other is C(R⁷);
R⁷, at each occurrence, is independently hydrogen or halogen;
R^{s}, at each occurrence, is independently C₁-C₆ alkyl, halogen, or C₁-C₆ haloalkyl;
R^{j}, at each occurrence, is independently hydrogen, C₁-C₆ alkyl, or C₁-C₆ haloalkyl; and
R^{k}, at each occurrence, is independently C₁-C₆ alkyl or C₁-C₆ haloalkyl.

2. The compound of claim 1 or a pharmaceutically acceptable salt thereof, wherein R² is G^{1A}.

3. The compound of claim 1 or a pharmaceutically acceptable salt thereof, wherein R² is -N(R^{a})S(O)₂R^{b}.

4. The compound of claim 1 or a pharmaceutically acceptable salt thereof, wherein R² is -N(R^{a})C(O)R^{b}.

5. The compound of claim 1 or a pharmaceutically acceptable salt thereof, wherein R² is -N(R^{a})C(O)OR^{b}.

6. The compound of claim 1 or a pharmaceutically acceptable salt thereof, wherein
R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.

7. The compound of claim 1 or a pharmaceutically acceptable salt thereof, wherein
R⁴ is monocyclic heteroaryl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.

8. The compound of claim 1 of formula (I) or a pharmaceutically acceptable salt thereof, wherein
R² is G^{1A}; and
R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.

9. The compound of claim 1 of formula (I) or a pharmaceutically acceptable salt thereof, wherein
R² is -N(R^{a})C(O)R^{b}; and
R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.

10. The compound of claim 1 of formula (I-a) or a pharmaceutically acceptable salt thereof,
R² is -N(R^{a})C(O)OR^{b}; and
R⁴ is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₁-C₆ alkylenyl)-OH.

11. The compound of claim 1 of formula (I-a) or a pharmaceutically acceptable salt thereof, 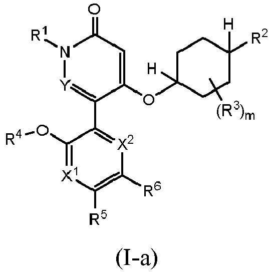 wherein R¹, Y, X¹, X², R², R³, R⁴, R⁵, R⁶, and m are as set forth in claim 1.

12. The compound of claim 1 of formula (I-b) or a pharmaceutically acceptable salt thereof, 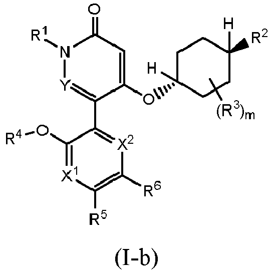 wherein R¹, Y, X¹, X², R², R³, R⁴, R⁵, R⁶, and m are as set forth in claim 1.

13. The compound of any one of claim 1-12 or a pharmaceutically acceptable salt thereof, wherein
Y is C(R^{Y});
X¹ is N or C(R⁷); and
X² is C(R⁷)

14. The compound of claim 13 or a pharmaceutically acceptable salt thereof, wherein
X¹ and X² are C(R⁷).

15. The compound of claim 13 or a pharmaceutically acceptable salt thereof, wherein
X¹ is N and X² is C(R⁷).

16. The compound of claim 1 or a pharmaceutically acceptable salt thereof, wherein the compound is selected from the group consisting of
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]acetamide;
N-[cis-4-({5-[2-(2,6-dimethylphenoxy)-5-(ethanesulfonyl)phenyl]-1-methyl-2-oxo-1,2-dihydropyridin-4-yl}oxy)cyclohexyl]acetamide;
methyl [trans-4-({5-[2-(2,6-dimethylphenoxy)-5-(ethanesulfonyl)phenyl]-1-methyl-2-oxo-1,2-dihydropyridin-4-yl}oxy)cyclohexyl]carbamate;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]methanesulfonamide;
tert-butyl [trans-4-({5-[2-(2,6-dimethylphenoxy)-5-(ethanesulfonyl)phenyl]-1-methyl-2-oxo-1,2-dihydropyridin-4-yl} oxy)cyclohexyl] carbamate;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-2,2,2-trifluoroacetamide;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]propanamide;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-2,2-dimethylpropanamide;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-2-methoxyacetamide;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-1-methylcyclopropane-1-carboxamide;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl] cyclopropanecarboxamide;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-3-methyloxetane-3-carboxamide;
N-[trans-4-({5-[2-(2,6-dimethylphenoxy)-5-(ethanesulfonyl)phenyl]-1-methyl-2-oxo-1,2-dihydropyridin-4-yl}oxy)cyclohexyl]-3,3-difluorocyclobutane-1-carboxamide;
N-{trans-4-[(5-{5-[(ethanesulfonyl)amino]-2-[4-(2-hydroxypropan-2-yl)-2,6-dimethylphenoxy]phenyl}-1-methyl-2-oxo-1,2-dihydropyridin-4-yl)oxy]cyclohexyl}acetamide;
methyl{trans-4-[(5-{5-[(ethanesulfonyl)amino]-2-[4-(2-hydroxypropan-2-yl)-2,6-dimethylphenoxy]phenyl}-1-methyl-2-oxo-1,2-dihydropyridin-4-yl)oxy]cyclohexyl} carbamate;
methyl {trans-4-[(5-{5-[(ethanesulfonyl)amino]-2-(4-fluoro-2,6-dimethylphenoxy)phenyl}-1-methyl-2-oxo-1,2-dihydropyridin-4-yl)oxy]cyclohexyl} carbamate;
methyl [trans-4-({5'-[(ethanesulfonyl)amino]-2'-(4-fluoro-2,6-dimethylphenoxy)-1-methyl-6-oxo[1,6-dihydro[3,3'-bipyridine]]-4-yl}oxy)cyclohexyl]carbamate;
methyl{trans-4-[(5-{2-(4-fluoro-2,6-dimethylphenoxy)-5-[(methanesulfonyl)methyl]phenyl}-1-methyl-2-oxo-1,2-dihydropyridin-4-yl)oxy]cyclohexyl} carbamate;
5-[5-(ethanesulfonyl)-2-(4-fluoro-2,6-dimethylphenoxy)phenyl]-1-methyl-4-{[trans-4-(2-oxopyrrolidin-1-yl)cyclohexyl]oxy}pyridin-2(1H)-one; and
N-[trans-4-({5-[5-(ethanesulfonyl)-2-(4-fluoro-2,6-dimethylphenoxy)phenyl]-1-methyl-2-oxo-1,2-dihydropyridin-4-yl}oxy)cyclohexyl]-N-methylacetamide.

17. A pharmaceutical composition comprising a therapeutically effective amount of a compound of formula (I) according to claim 1, or a pharmaceutically acceptable salt thereof, in combination with a pharmaceutically acceptable carrier.

18. A method for treating cancer in a subject comprising administering a therapeutically effective amount of a compound of formula (I) according to claim 1 or a pharmaceutically acceptable salt thereof, to a subject in need thereof.

19. The method of claim 18 wherein the cancer is selected from the group consisting of: acoustic neuroma, acute leukemia, acute lymphocytic leukemia, acute myelocytic leukemia (monocytic, myeloblastic, adenocarcinoma, angiosarcoma, astrocytoma, myelomonocytic and promyelocytic), acute t-cell leukemia, basal cell carcinoma, bile duct carcinoma, bladder cancer, brain cancer, breast cancer, bronchogenic carcinoma, cervical cancer, chondrosarcoma, chordoma, choriocarcinoma, chronic leukemia, chronic lymphocytic leukemia, chronic myelocytic (granulocytic) leukemia, chronic myelogenous leukemia, colon cancer, colorectal cancer, craniopharyngioma, cystadenocarcinoma, diffuse large B-cell lymphoma, dysproliferative changes (dysplasias and metaplasias), embryonal carcinoma, endometrial cancer, endotheliosarcoma, ependymoma, epithelial carcinoma, erythroleukemia, esophageal cancer, estrogen-receptor positive breast cancer, essential thrombocythemia, Ewing's tumor, fibrosarcoma, follicular lymphoma, germ cell testicular cancer, glioma, glioblastoma, gliosarcoma, heavy chain disease, hemangioblastoma, hepatoma, hepatocellular cancer, hormone insensitive prostate cancer, leiomyosarcoma, leukemia, liposarcoma, lung cancer, lymphagioendotheliosarcoma, lymphangiosarcoma, lymphoblastic leukemia, lymphoma (Hodgkin's and non-Hodgkin's), malignancies and hyperproliferative disorders of the bladder, breast, colon, lung, ovaries, pancreas, prostate, skin, and uterus, lymphoid malignancies of T-cell or B-cell origin, leukemia, lymphoma, medullary carcinoma, medulloblastoma, melanoma, meningioma, mesothelioma, multiple myeloma, myelogenous leukemia, myeloma, myxosarcoma, neuroblastoma, NUT midline carcinoma (NMC), non-small cell lung cancer, oligodendroglioma, oral cancer, osteogenic sarcoma, ovarian cancer, pancreatic cancer, papillary adenocarcinomas, papillary carcinoma, pinealoma, polycythemia vera, prostate cancer, rectal cancer, renal cell carcinoma, retinoblastoma, rhabdomyosarcoma, sarcoma, sebaceous gland carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenström's macroglobulinemia, testicular tumors, uterine cancer, and Wilms' tumor.

20. A method for treating a disease or condition in a subject comprising administering a therapeutically effective amount of a compound of formula (I) according to claim 1 or a pharmaceutically acceptable salt thereof, to a subject in need thereof, wherein said disease or condition is selected from the group consisting of Addison's disease, acute gout, ankylosing spondylitis, asthma, atherosclerosis, Behcet's disease, bullous skin diseases, chronic obstructive pulmonary disease (COPD), Crohn's disease, dermatitis, eczema, giant cell arteritis, glomerulonephritis, hepatitis, hypophysitis, inflammatory bowel disease, Kawasaki disease, lupus nephritis, multiple sclerosis, myocarditis, myositis, nephritis, organ transplant rejection, osteoarthritis, pancreatitis, pericarditis, polyarteritis nodosa, pneumonitis, primary biliary cirrhosis, psoriasis, psoriatic arthritis, rheumatoid arthritis, scleritis, sclerosing cholangitis, sepsis, systemic lupus erythematosus, Takayasu's Arteritis, toxic shock, thyroiditis, type I diabetes, ulcerative colitis, uveitis, vitiligo, vasculitis, and Wegener's granulomatosis.

21. A method for treating an acquired immunodeficiency syndrome (AIDS) in a subject comprising administering a therapeutically effective amount of a compound of formula (I) according to claim 1 or a pharmaceutically acceptable salt thereof, to a subject in need thereof.

22. A method for treating a disease or condition in a subject comprising administering a therapeutically effective amount of a compound of formula (I) according to claim 1 or a pharmaceutically acceptable salt thereof, to a subject in need thereof, wherein said disease or condition is selected from the group consisting of: obesity, dyslipidemia, hypercholesterolemia, Alzheimer's disease, metabolic syndrome, hepatic steatosis, type II diabetes, insulin resistance, diabetic retinopathy, and diabetic neuropathy.

23. A method of contraception in a male subject comprising administering a therapeutically effective amount of a compound of formula (I) according to claim 1 or a pharmaceutically acceptable acceptable salt thereof, to a subject in need thereof.

24. A method for treating an acute kidney disease or condition in a subject comprising administering a therapeutically effective amount of a compound of claim 1 or a pharmaceutically acceptable salt thereof, to a subject in need thereof, wherein said acute kidney disease or condition is selected from the group consisting of: ischemia-reperfusion induced kidney disease, cardiac and major surgery induced kidney disease, percutaneous coronary intervention induced kidney disease, radio-contrast agent induced kidney disease, sepsis induced kidney disease, pneumonia induced kidney disease, and drug toxicity induced kidney disease.

25. A method of treating a chronic kidney disease or condition in a subject comprising administering a therapeutically effective amount of a compound of claim 1 or a pharmaceutically acceptable salt thereof, to a subject in need thereof, wherein said disease or condition is selected from the group consisting of: diabetic nephropathy, hypertensive nephropathy, HIV-associated nephropathy, glomerulonephritis, lupus nephritis, IgA nephropathy, focal segmental glomerulosclerosis, membranous glomerulonephritis, minimal change disease, polycystic kidney disease and tubular interstitial nephritis.
